# EUROPEAN PATENT SPECIFICATION

(11) **EP 4 179 306 B1**
(45) Date of publication and mention of the grant of the patent: **28.10.2026**
(21) Application number: 21838366.9
(22) Date of filing: 08.07.2021
(51) Int. Cl.: G01N 27/74, G01R 33/12, G01N 33/543, G01N 15/10, G01N 15/1031, H10N 52/00, G01R 33/00, G01R 33/07, G01R 33/09

(54) **SINGLE-MOLECULE, REAL-TIME, LABEL-FREE DYNAMIC BIOSENSING WITH NANOSCALE MAGNETIC FIELD SENSORS**
EINZELMOLEKÜL-, ECHTZEIT-, MARKIERUNGSFREIES DYNAMISCHES BIOSENSING MIT NANOSKALIGEN MAGNETFELDSENSOREN
BIO-DÉTECTION DYNAMIQUE SANS MARQUEUR EN TEMPS RÉEL DE MOLÉCULE UNIQUE AVEC DES CAPTEURS DE CHAMP MAGNÉTIQUE À L'ÉCHELLE NANOMÉTRIQUE

(30) Priority: 08.07.2020 US 202062705639 P
(43) Date of publication of application: 17.05.2023
(73) Proprietor: Western Digital Technologies, Inc., San Jose, CA 95119 (US); Roche Diagnostics GmbH, 68305 Mannheim (DE); F. Hoffmann-La Roche AG, 4070 Basel (CH)
(72) Inventor: TOPOLANCIK, Juraj, Pleasanton, California 94588 (US); BRAGANCA, Patrick, San Jose, California 95119 (US); ASTIER, Yann, Pleasanton, California 94588 (US); SHIN, Seong-Ho, Pleasanton, California 94588 (US); MAJZIK, Zsolt, Pleasanton, California 94588 (US)
(74) Representative: Mewburn Ellis LLP
(86) International application number: PCT/US2021/040767
(87) International publication number: WO 2022/011067

(56) References cited:
- CN-A- 102 928 596
- US-A1- 2005 035 757
- US-A1- 2007 207 553
- US-A1- 2009 219 012
- US-A1- 2010 194 386
- US-A1- 2010 259 254
- US-A1- 2011 223 612
- US-A1- 2011 223 612
- US-A1- 2014 292 318
- US-A1- 2018 128 822
- US-A9- 2017 097 392

## Description

### BACKGROUND

The ability to quantify interactions between biomolecules is of interest for a variety of applications, such as diagnosis, screening, disease staging, forensic analysis, pregnancy testing, drug development and testing, and scientific and medical research. Examples of measurable characteristics of a biomolecular interaction include the affinity (e.g., how strongly the molecules bind/interact) and the kinetics (e.g., rates at which the association and dissociation of molecules occur) of the interaction.

Traditional enzyme-linked immunosorbent assay (ELISA) systems are analog systems that require large volumes that ultimately dilute reaction product, requiring millions of enzyme labels to generate signals that are detectable using conventional plate readers. Thus, traditional ELISA sensitivity is limited to the picomolar (pg/mL) range and above.

In contrast to ELISA systems, single-molecule systems are digital in nature, because each molecule provides a respective signal that can be detected and counted. Single-molecule systems have the advantage that it is easier to determine the presence or absence of a signal than it is to detect the absolute amount or amplitude of a signal. In other words, it is easier to count than it is to integrate.

Interest in the detection of single molecules has increased in recent years. For example, the COVID-19 pandemic has put patients with cancer at higher risk than usual, because they may be more susceptible to viral infections after chemotherapy, stem cell transplants, or surgeries. As another example, there is a need for ultrasensitive virus and pathogen detection, such as to detect COVID-19 or human SARS-CoV-2 antibodies. Another example of an application that can benefit from single-molecule detection is single-molecule immunoassays to provide simple and highly sensitive protein biomarker detection.

Detection of single molecules has become possible for some applications. For example, the use of tethered particle motion (TPM) techniques has made it possible to detect the binding of a single biomolecule to a receptor anchored to the surface of a sensing device. In TPM, one end of a biopolymer (e.g., DNA, RNA, etc.) is immobilized onto a solid support, thereby creating a "tethered biopolymer," and a small particle (e.g., micrometer-sized or nanometer-sized) is attached to the other end. In solution, the tethered biopolymer and the attached particle move due to constrained Brownian motion (random motion of particles suspended in a medium). The volume occupied by the tethered biopolymer (and the attached particle) is limited and depends on the size and shape of the tethered biopolymer. Enzymes that interact directly with a biopolymer can change the biopolymer's structure at any given time. For example, for DNA and RNA, the volume occupied by the attached particle varies depending on the deformation of the DNA (e.g., DNA looping or DNA extension). By observing and interpreting changes in the position of the particle as a function of time, the kinetics and biochemical dynamics of, for example, interactions between a biopolymer and enzymes in a solution can be described.

The tethered biopolymer may be a nucleotide sequence such as a DNA fragment. Binding events typically alter the molecular dynamics of the receptor. Before a complementary nucleotide is incorporated, the DNA fragment may adopt a coiled-up or U-shaped (looped) conformation (e.g., due to the presence of a (partial) palindrome in the nucleotide sequence), and then adopt a more linear or stretched conformation when a complementary nucleotide is incorporated. This change in conformation affects the volume of Brownian motion that the tethered biopolymer inhabits. In TPM, the change in volume can be detected by attaching a particle (sometimes referred to as a label) to the receptor and using optical techniques to observe the particle's motion.

Data acquisition in TPM systems typically employs high-resolution, high-speed video microscopy to track and record nanoscale variations of the particle average velocity and range of motion caused by local changes in the microenvironment. This single-molecule analysis technique has been implemented, for example, for dynamic in-vitro monitoring of DNA-protein interactions and detection of biochemically-induced conformational changes in proteins, DNA, and RNA.

Because TPM relies on the ability to resolve small variations in stochastic motion patterns, the image contrast must be sufficient and the frame acquisition rate high enough to enable tracking of the particle and the subsequent analysis. State-of-the-art TPM systems can optically track nanoscale particles attached to short (e.g., about 50 nm) tethers with 1-2 nm localization accuracy. Although the high-resolution is impressive, the number of particles that can be followed and analyzed simultaneously within a small field of view is limited to a few hundred. Therefore, the throughput of such systems is limited. Increasing the field of view to allow monitoring of 10,000 nanoparticles degrades the localization accuracy to greater than about 100 nm. This limitation, together with the technological complexity of high-throughput real-time motion tracking at the nanoscale, has so far confined the use of TPM to within the realm of academic scientific curiosity and has prevented widespread use in commercial applications such as diagnostics and drug discovery.

The particle size plays a significant role in TPM measurements. Large particles are easier to observe and track than smaller particles, but their stochastic motion is only weakly affected by single-molecule processes due to the large size disparity between the particle and the receptor. Furthermore, the proximity of large tethered particles to a solid surface (e.g., to which the receptors are attached) gives rise to a stretching force on the biopolymers that changes their biophysical properties and can possibly cause significant variations in binding equilibria when the molecules are participating in biomarker binding reactions. Therefore, to reproduce in vivo processes accurately, it is desirable to make the tethered particles as small as possible. Stochastic motion patterns of smaller particles are also more sensitive to perturbations caused by binding of individual biomolecules. The problem with small particles, however, is that they are more difficult to observe using optical systems. Strongly-scattering 10 nm gold nanoparticles confined within 2-dimensional biological membranes have been observed and tracked optically. Larger sizes (typically larger than 40 nm in diameter) are preferred for reliable tracking when the particles are tethered to the surface with biopolymers and are allowed to move in and out of the focal-plane. But these dimensions make the particles considerably larger than the sizes of molecules involved in many biomedically-relevant processes. Because the amount of light scattering at these length scales is proportional to the sixth power of the diameter, further reduction of the particle size to match the molecular dimensions would make them untrackable with even the most advanced optical systems available today.

Thus, there is a need for improved single-molecule devices, systems, and methods to monitor and/or quantify interactions between biomolecules.

US 2011/223612 A1 discloses a method for quantitatively determining a binding kinetic parameter of a molecular binding interaction, the method comprising: producing a magnetic sensor device including a magnetic sensor in contact with an assay mixture including a magnetically labeled molecule to produce a detectable molecular binding interaction; obtaining a real-time signal from the magnetic sensor; and quantitatively determining a binding kinetics parameter of the molecular binding interaction from the real-time signal.

US 2010/259254 A1 discloses a sensor device and a method for the determination of the amount of target particles at a contact surface adjacent to a sample chamber. Target particles in the sample chamber are detected by a sensor element and at least one corresponding sensor-signal is provided. An evaluation unit then determines the amount of target particles in a first zone immediately at the contact surface and a second zone a distance away from the contact surface based on this sensor-signal. In a magnetic measurement approach, different magnetic excitation fields may be used to excite magnetic target particles differently in the first and second zone. Moreover, the temporal course of a sensor-signal can be evaluated, particularly with respect to stochastic movements of the target particles.

### SUMMARY

Aspects of the invention are set out in the appended claims.

Disclosed herein are devices, systems, and methods for monitoring single-molecule processes using magnetic sensors. In some embodiments, a magnetic particle (e.g., a magnetic nanoparticle), referred to herein as a MNP, is attached to a biopolymer (e.g., a nucleic acid, protein, etc.), also referred to as a tether, to detect motion of the MNP. For example, the binding of individual molecules, antibody/antigen reactions, and/or changes of conformation of a protein or nucleic acid can be detected by using a magnetic sensor to observe, follow, or track the position and/or the motion of the MNP. The MNP is small (e.g., its size is comparable to the size of the molecule being monitored) and is tethered to a biopolymer, and the volume of Brownian motion of the MNP in a solution changes due to the MNP being bombarded by molecules of the solution, thereby changing the position of the MNP and allowing the movement of the MNP and, by inference, the tethered biopolymer to be observed and/or monitored. Changes in the position and/or motion of the MNP can be inferred from changes in signals obtained from the magnetic sensor. For example, analysis of an autocorrelation function or power spectral density of a signal obtained from the magnetic sensor can reveal the presence, position, and/or movement of the MNP.

A magnetic sensor (e.g., nanoscale or having a size on the order of the sizes of the MNP and/or the biopolymer) can be used to detect even small changes in the position of the MNP within the sensing region of the magnetic sensor. A baseline response of the magnetic sensor (e.g., a signal) can be determined in the absence of any MNP, and then after the MNP has been attached to a biopolymer within the magnetic sensor's sensing region, the signal provided by the magnetic sensor is a superposition of the MNP's Brownian motion and the baseline sensor response. Thus, the effect of the MNP, which moves according to a random process, is to add noise to the baseline sensor response. By detecting and/or analyzing the noise contribution from the MNP in the sensor signal in either or both of the time domain and frequency domain (e.g., by detecting fluctuations around a mean, inspecting/processing/analyzing an autocorrelation function or a power spectral density, etc.), conclusions can be drawn about the presence, position, and/or movement of the MNP. In this way, the MNP can be a reporter of biopolymer activity (e.g., conformational changes).

Because the disclosed devices, systems, and methods do not rely on imaging, the MNPs can be substantially smaller than those used in TPM systems, thereby providing higher resolution and allowing for higher throughput from a device of a selected size. Moreover, magnetic sensors and MNPs can be used to reliably detect nanoscopic motion with high accuracy (e.g., movement on the order of a few nanometers). The disclosed devices, systems, and methods can be used in a variety of single-molecule applications, including but not limited to diagnosis, screening, disease staging, forensic analysis, pregnancy testing, drug development and testing, immunoassays, nucleic acid sequencing, and scientific and medical research. They offer potentially high throughput and higher sensitivity and accuracy than conventional TPM or traditional ELISA approaches that rely on optics.

### BRIEF DESCRIPTION OF THE DRAWINGS

Objects, features, and advantages of the disclosure will be readily apparent from the following description of certain embodiments taken in conjunction with the accompanying drawings in which:
FIG. 1A is a schematic representation of nanoscale monitoring of the motion of a MNP attached to a biopolymer in accordance with some embodiments.
FIG. 1B illustrates an example of a recorded sensor signal in accordance with some embodiments.
FIGS. 2A, 2B, 2C, and 2D illustrate examples of four reversible biomolecular single-molecule processes that affect MNP velocity and range-of-motion patterns in accordance with some embodiments.
FIG. 3 illustrates a portion of a magnetic sensor in accordance with some embodiments.
FIGS. 4A and 4B illustrate the resistance of magneto-resistive (MR) sensors, which may be used in accordance with some embodiments.
FIG. 5A illustrates a spin-torque oscillator (STO) sensor, which may be used in accordance with some embodiments.
FIG. 5B shows the experimental response of a STO under example conditions.
FIGS. 5C and 5D illustrate short nanosecond field pulses of STOs that may be used in accordance with some embodiments.
FIG. 6 is a diagram of a portion of an exemplary read head that includes a magnetic sensor used in a perpendicular magnetic recording (PMR) application.
FIG. 7A illustrates a magnetic sensor without any MNP in its vicinity in accordance with some embodiments.
FIG. 7B illustrates the magnetic sensor with a MNP situated directly above it in accordance with some embodiments.
FIG. 7C illustrates the magnetic sensor with a MNP laterally offset from it in accordance with some embodiments.
FIG. 8 illustrates the results of nanomagnetic simulations of an exemplary magnetic sensor in the presence of a MNP at various positions relative to the magnetic sensor in accordance with some embodiments.
FIG. 9A is a plane view scanning electron microscopy (SEM) image of an exemplary magnetic sensor with a MNP within its sensing region in accordance with some embodiments.
FIGS. 9B and 9C illustrate the behavior of the exemplary magnetic sensor of FIG. 9A in accordance with some embodiments.
FIG. 10A presents an example model to analyze the motion of a MNP in accordance with some embodiments.
FIG. 10B is a pictorial representation of a single particle diffusing in a harmonic potential applied by a DNA strand.
FIGS. 11A and 11B illustrate a thought experiment.
FIG. 12A illustrates an exemplary magnetic sensor in accordance with some embodiments.
FIG. 12B plots the expected noise power spectral density (PSD) of an example magnetic sensor and the Lorentzian function characterizing the PSD of the confined Brownian motion of the MNP.
FIG. 13 is a pictorial illustration of experiments conducted by the inventors.
FIG. 14 illustrates the measured PSDs of three tested magnetic sensors.
FIGS. 15A, 15B, 15C, 15D, and 15E illustrate test results investigating the impact of the magnetic sensor bias voltage.
FIG. 16 illustrates a one-dimensional model that includes a force component due to the magnetic sensor.
FIGS. 17A, 17B, and 17C illustrate three states of a system in accordance with some embodiments.
FIGS. 18A, 18B, and 18C illustrate exemplary recorded current fluctuations of two exemplary magnetic sensors and the corresponding autocorrelation functions in accordance with some embodiments.
FIG. 19A is a block diagram showing components of an exemplary monitoring system in accordance with some embodiments.
FIGS. 19B, 19C, and 19D illustrate portions of an exemplary monitoring system in accordance with some embodiments.
FIG. 19E illustrates a pattern of magnetic sensors of a sensor array in accordance with some embodiments.
FIG. 20 is a flow diagram of an exemplary method of sensing motion of tethered MNPs in accordance with some embodiments.
FIG. 21 illustrates several components involved in multiplexed magnetic digital homogeneous, non-enzymatic (HoNon) ELISA in accordance with some embodiments.
FIGS. 22A and 22B illustrate a portion of an exemplary procedure for multiplexed magnetic digital HoNon ELISA in accordance with some embodiments.
FIG. 23 illustrates additional steps of the exemplary procedure for multiplexed magnetic digital HoNon ELISA in accordance with some embodiments.
FIG. 24A illustrates the addition of a complex biological solution containing multiple biomarkers in accordance with some embodiments.
FIG. 24B is a depiction of how a sensor array might look following the addition of a complex biological solution containing multiple biomarkers in accordance with some embodiments.
FIG. 25 illustrates how the binding of a biomarker can be detected from the detected noise PSD of a particular magnetic sensor in accordance with some embodiments.
FIG. 26 is a flow diagram illustrating a method of using a magnetic sensor array in accordance with some embodiments.

To facilitate understanding, identical reference numerals have been used, where possible, to designate identical elements that are common to the figures. It is contemplated that elements disclosed in one embodiment may be beneficially utilized in other embodiments without specific recitation. Moreover, the description of an element in the context of one drawing is applicable to other drawings illustrating that element.

### DETAILED DESCRIPTION

Stochastic motion of freely-diffusing or tethered particles embedded in biological systems reveals a considerable wealth of information. Statistical analysis of the particle motion may facilitate understanding of important in vivo processes through their in vitro consequences. Although tracking of freely diffusing strongly-scattering particles as small as 10 nm is a powerful tool for studying biological membranes, tracking of tethered particles reveals a much broader range of single-molecule behavior. TPM experiments use biopolymers (e.g., DNA, RNA, proteins) with one end anchored to a solid surface and the other end attached to a particle to monitor various biophysical and biochemical processes, but the throughput and accuracy of traditional TPM systems are limited due to the reliance on optical techniques to track particles.

Disclosed herein are devices, systems, and methods for dynamic sensing of biochemically-induced changes in tethered nanoparticle motion patterns that do not involve imaging. Instead, embodiments disclosed herein use magnetic sensors and monitor the responses of those magnetic sensors to detect the confined diffusion of tethered magnetic particles as they move stochastically within, or in and out of, the respective detection regions of the magnetic sensors. The magnetic sensors may be, for example, nanoscale magnetic field sensors (MFS). The detected response or characteristic of a magnetic sensor may be, for example, a detected tunneling current, voltage, or resistance in the time or frequency domain, or any other characteristic of the magnetic sensor that is detectable. The detection region of a magnetic sensor may have a volume, for example, of between about 10⁵ nm³ and 5 × 10⁵ nm³.

The magnetic particles may be or comprise, for example, a magnetic nanoparticle (MNP), such as, for example, a molecule, a superparamagnetic nanoparticle, or a ferromagnetic particle. As will be appreciated by those having ordinary skill in the art, a magnetic nanoparticle is generally considered to be a particle of matter between 1 and 100 nanometers (nm) in diameter. The magnetic particles may be nanoparticles with high magnetic anisotropy. Examples of magnetic particles with high magnetic anisotropy include, but are not limited to, Fe₃O₄, FePt, FePd, and CoPt. In some applications involving nucleotides, the magnetic particles may be synthesized and coated with, for example, SiO₂. *See, e.g.,* M. Aslam, L. Fu, S. Li, and V.P. Dravid, "Silica encapsulation and magnetic properties of FePt nanoparticles," Journal of Colloid and Interface Science, Volume 290, Issue 2, 15 October 2005, pp. 444-449.

The magnetic particles may be or comprise, for example, organometallic compounds. As will be appreciated, an organometallic compound is any member of a class of substances containing at least one metal-to-carbon bond in which the carbon is part of an organic group. Examples of organometallic compounds include Gilman reagents (which contain lithium and copper), Grinard reagents (which contain magnesium), tetracarbonyl nickel and ferrocene (which contain transition metals), organolithium compounds (e.g., n-butyllithium (n-BuLi)), organozinc compounds (e.g., diethylzinc (Et₂Zn)), organotin compounds (e.g., tributyltin hydride(Bu₃SnH)), organoborane compounds (e.g., triethylborane (Et₃B)), and organoaluminium compounds (e.g., trimethylaluminium (Me₃Al)).

The magnetic particles may be or comprise, for example, charged molecules, or any other functional molecular group that can be detected by nanoscale magnetic sensors. Stated another way, if the magnetic sensors can detect the presence of a candidate magnetic particle, and the candidate magnetic particle can be attached to the biopolymer of interest, that candidate magnetic particle is suitable for use in the devices, systems, and methods described herein.

Although it is expected that the magnetic particles used in many applications will likely be nanoparticles so that they are of comparable size to the biopolymers being observed, the systems, devices, and methods described herein apply generally to magnetic particles. Thus, it is to be understood that the abbreviation "MNP" is used herein for convenience, and that "MNP" can refer to magnetic particles generally. Accordingly, unless indicated otherwise by context, disclosures herein referring to or illustrating MNPs are not necessarily limited solely to nanoparticles. Similarly, although it is expected that the MNPs may be superparamagnetic, the disclosures are not limited to use with superparamagnetic MNPs.

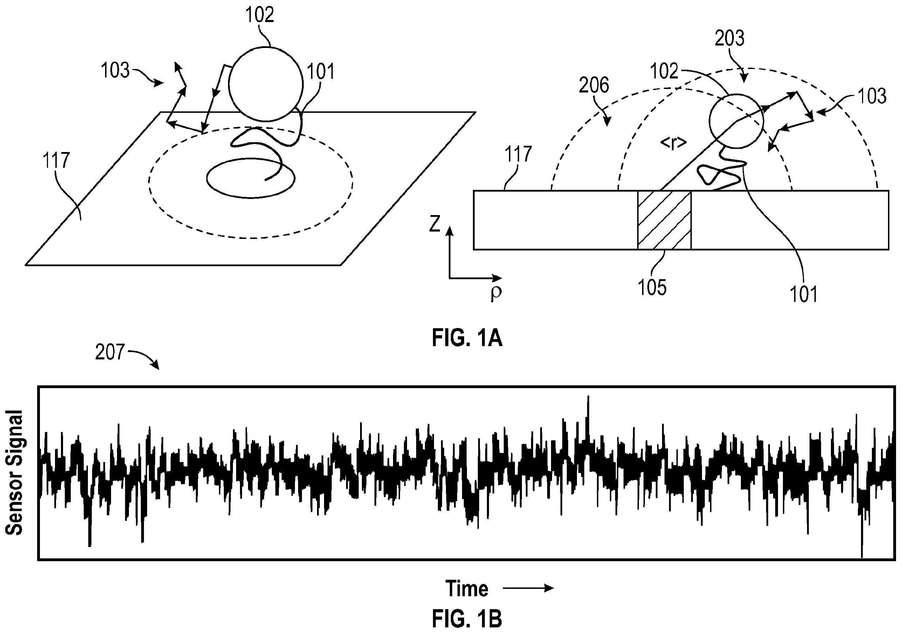

FIGS. 1A and 1B illustrate principles of nanoscale monitoring of the motion of MNPs using magnetic sensors in accordance with some embodiments. As shown in FIG. 1A, a MNP 102 is tethered to the solid surface 117 of a monitoring device by a biopolymer 101 (e.g., ssDNA, dsDNA, RNA, protein, etc.). The biopolymer 101 may also be referred to as a "tether." Because of interactions with molecules of the surrounding fluid, the MNP 102 undergoes stochastic (random) motion, represented by the arrows 103 in FIG. 1A, within a constrained motion region 203, which is a volume around some average distance <r> from a magnetic sensor 105. The MNP 102 moves within, or in and out of, a sensing region 206 of the magnetic sensor 105. For some biosensing applications, the sensing region 206 may have a volume, for example, of between about 10⁵ nm³ and about 5 × 10⁵ nm³. Of course, the volume of the sensing region 206 can be selected to suit a particular application and may be larger or smaller than these values. Depending on the design of the magnetic sensor 105 (e.g., its sensitivity), the bias voltage applied to the magnetic sensor 105, the characteristics of the MNP 102 (e.g., its size), the characteristics of the biopolymer 101 (e.g., its length), and the location at which the biopolymer 101 is tethered to the surface 117 relative to the magnetic sensor 105, the constrained motion region 203 and sensing region 206 may substantially overlap, or they may be offset as shown in the example of FIG. 1A. Similarly, the volumes of the constrained motion region 203 and sensing region 206 may be the same or different. In the example illustrated in FIG. 1A, the constrained motion region 203 is larger than the sensing region 206, and it is offset from it in the lateral direction *ρ*.

FIG. 1B illustrates an example of a recorded sensor signal 207 in accordance with some embodiments. In the example, the sensor signal 207 is recorded as statistically stationary fluctuations in some detectable characteristic of the magnetic sensor 105, which may be, for example, the measured electrical current, voltage, resistance, oscillation frequency, phase noise, frequency noise, or any other characteristic of the magnetic sensor 105 that indicates a detected change in the magnetic environment of the magnetic sensor 105 (e.g., within the sensing region 206, attributable to presence, absence, and/or movement of a MNP 102), as described further below. One benefit of using a magnetic sensor 105 is that the MNP 102 can be considerably smaller than the particles used in TPM systems that rely on optical tracking. In some embodiments, for example, the MNP 102 has biomolecular dimensions (e.g., its size can be around or less than 5 nm).

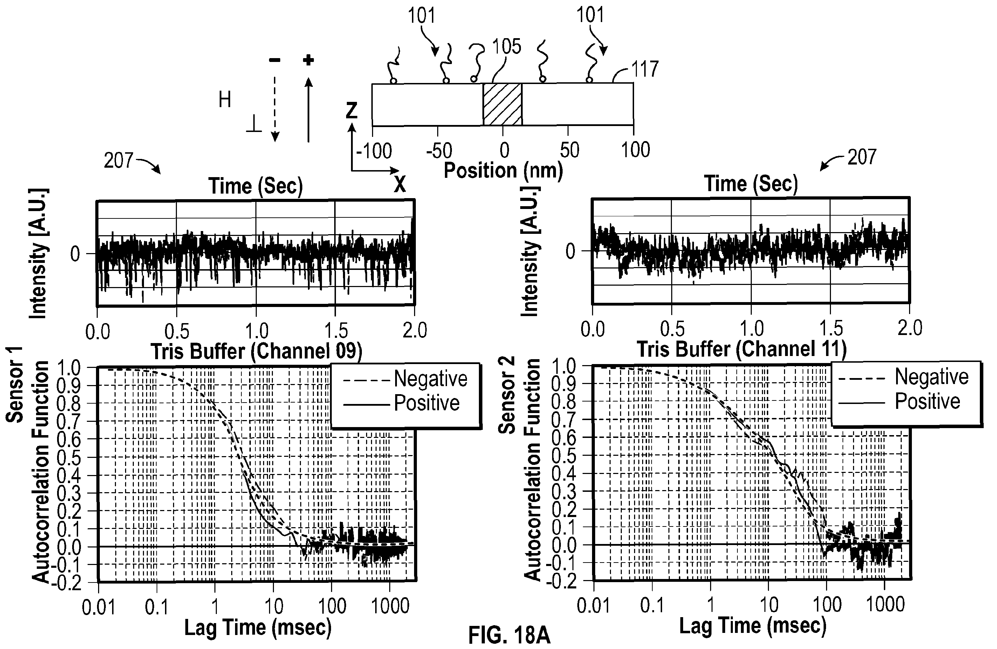

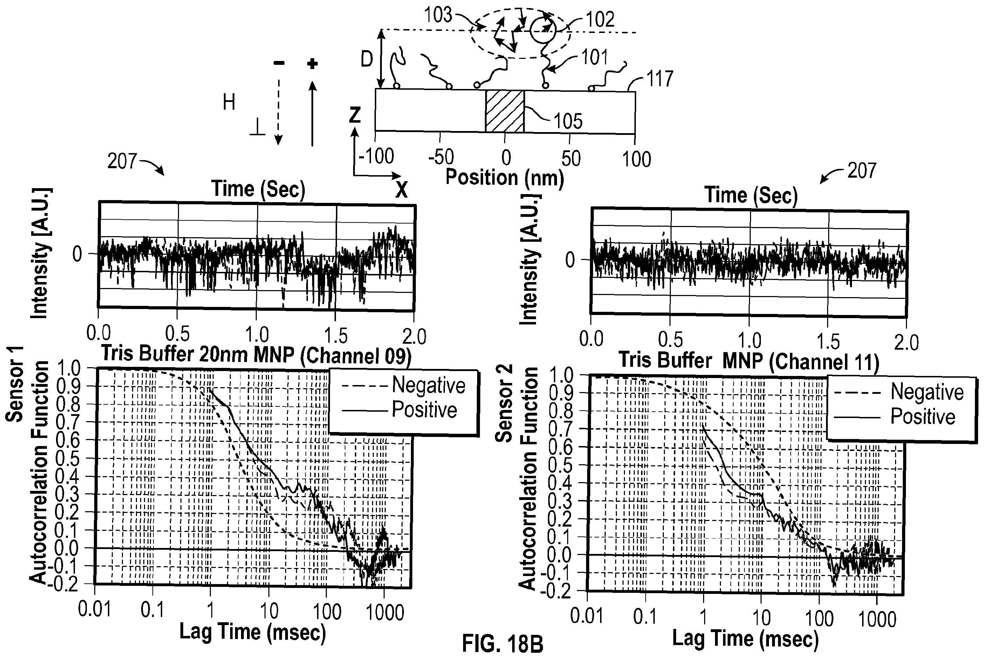

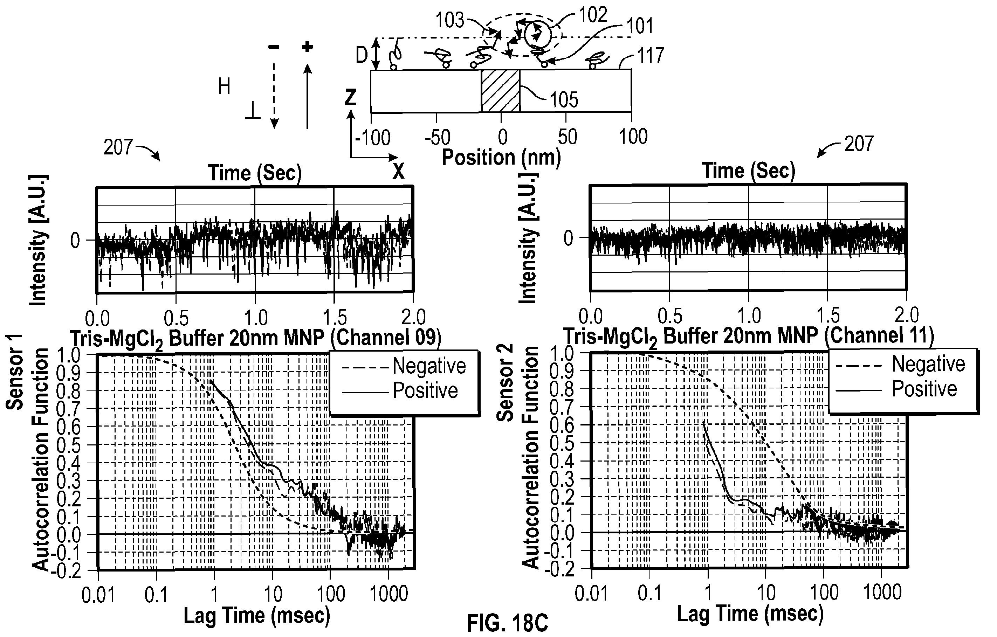

To allow detection of the MNP 102, the response of the magnetic sensor 105, as represented by the sensor signal 207, should change due to the mobility of the MNP 102 being affected by interactions with individual single molecules (e.g., of a surrounding solution). Accordingly, it is desirable for the MNP 102 to be small enough that its mobility is affected by other molecules. The sensor signal 207 (e.g., the noise component of the sensor signal 207 due to the motion of the MNP 102) should change, for example, when a biomolecule of a comparable size binds to molecule attached to the MNP 102, or when the attached molecule (biopolymer 101) changes its conformation, as described below in the discussion of, for example, FIGS. 18A, 18B, and 18C. In both cases, the effective hydrodynamic radius of the tethered MNP 102 changes, and its statistical velocity and range of motion also change. Thus, both the sensor signal 207 amplitude and noise should change both when the tethered MNP 102 is immobilized on or near the magnetic sensor 105 surface by specific target binding, and when the conformational state of the tether/biopolymer 101 (e.g., dsDNA, ssDNA, RNA, protein) changes.

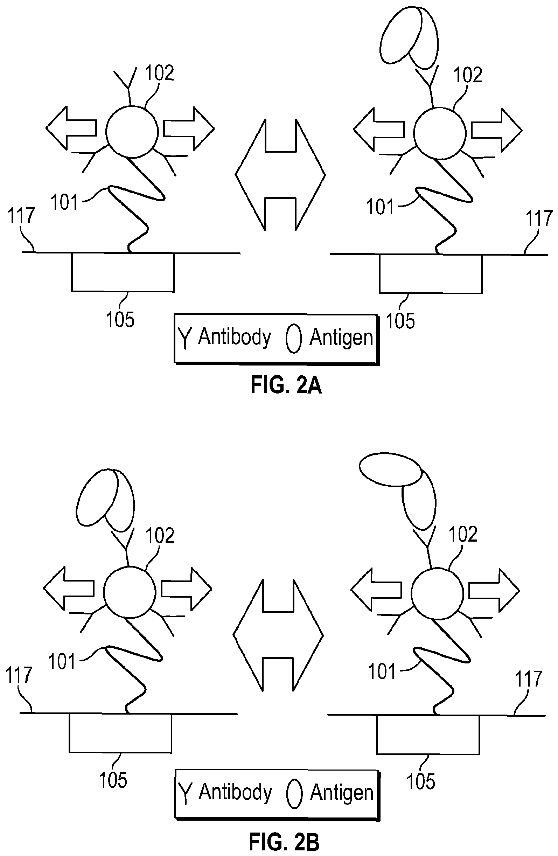

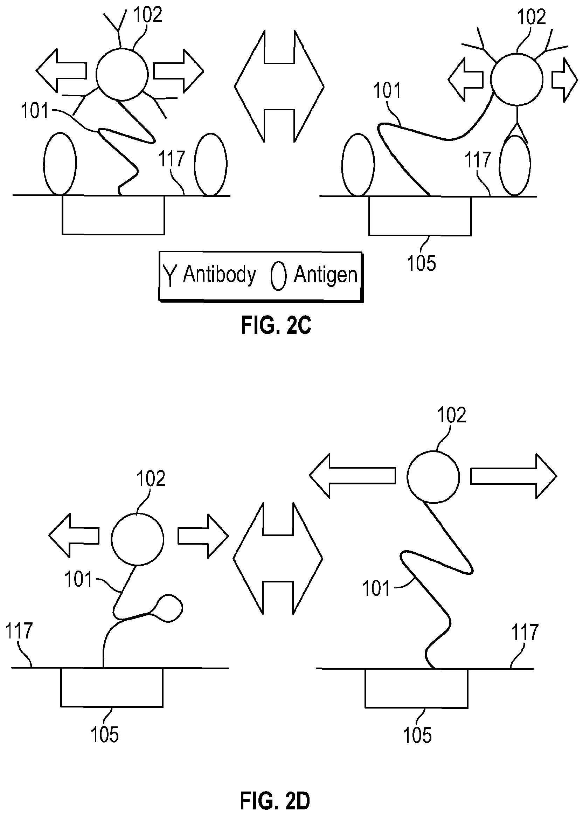

The systems, devices, and methods disclosed herein can be used to detect and/or monitor a variety of changes in biomolecular processes, such as, for example, the conformational kinetics of looping (connecting and disconnecting), folding and unfolding of proteins, antibody/antigen interactions and their strengths, etc. FIGS. 2A, 2B, 2C, and 2D illustrate examples of four reversible biomolecular single-molecule processes that affect MNP 102 velocity and range-of-motion patterns in accordance with some embodiments. Each of FIGS. 2A, 2B, 2C, and 2D illustrates a magnetic sensor 105 along with a biopolymer 101, one end of which is bound to a surface 117 of a monitoring device in the vicinity of the magnetic sensor 105 (e.g., at a binding site 116, discussed below), and the other end of which is attached to a MNP 102. FIGS. 2A and 2C illustrate exemplary antibody-antigen reactions, and FIGS. 2B and 2D illustrate exemplary conformation changes. FIG. 2A illustrates that binding a large biomolecule, such as, for example, a protein, DNA, or RNA, to a MNP 102 increases the mass of the MNP 102 as well as its effective hydrodynamic radius, causing changes to the confined diffusion that can be detected. (As described in further detail below, the binding of a molecule of a size comparable to the MNP 102 can be detected by detecting a change in the corner frequency of a Lorentzian function characterizing the noise PSD of the confined Brownian motion of the MNP 102.) FIG. 2B illustrates that a significant conformational change, such as, for example, a protein or nucleic acid folding and unfolding, also changes the effective hydrodynamic radius of the MNP 102, which can also be detected. FIG. 2C, similar to FIG. 2A, illustrates that the MNP 102 can bind to a molecule (illustrated as an antigen in the example of FIG. 2C) that is immobilized on the surface 117 of the monitoring device. The strength of the interaction can be studied in accordance with some embodiments. FIG. 2D illustrates that a conformational change of the tether (biopolymer 101), such as, for example, DNA or RNA hairpin formation, also restricts the motion of the MNP 102. How the nucleic acid behaves (e.g., wraps and unwraps) as a function of, for example, temperature may be of interest. The devices, systems, and methods disclosed herein can be used to detect and/or monitor changes including, but not limited to, those illustrated in FIGS. 2A, 2B, 2C, and 2D.

### Magnetic Sensors

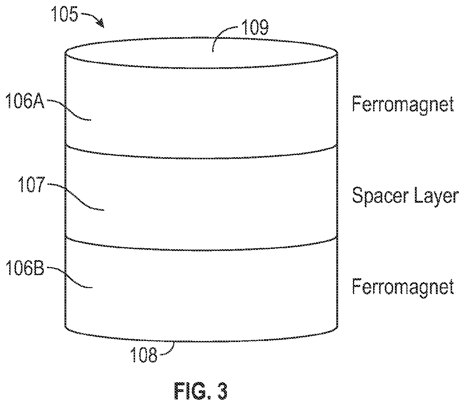

Embodiments disclosed herein use at least one magnetic sensor 105 (e.g., a magnetoresistive nanoscale sensor or any other type of magnetic sensor) to detect the presence of one or more MNPs 102 (e.g., magnetic nanoparticles, organometallic complexes, charged molecules, etc.) coupled to a biopolymer 101. FIG. 3 illustrates a portion of an exemplary magnetic sensor 105 in accordance with some embodiments. The exemplary magnetic sensor 105 of FIG. 3 has a bottom surface 108 and a top surface 109, and it comprises three layers: a first ferromagnetic layer 106A, a second ferromagnetic layer 106B, and a nonmagnetic spacer layer 107 between the first ferromagnetic layer 106A and second ferromagnetic layer 106B. Suitable materials for use in the first ferromagnetic layer 106A and second ferromagnetic layer 106B include, for example, alloys of Co, Ni, and Fe (sometimes mixed with other elements). In some embodiments, the magnetic sensor 105 is implemented using thin-film technology, and the first ferromagnetic layer 106A and second ferromagnetic layer 106B are engineered to have their magnetic moments oriented either in the plane of the film or perpendicular to the plane of the film. The nonmagnetic spacer layer 107 may be, for example, a metallic material such as, for example, copper or silver, in which case the structure is called a spin valve (SV), or it may be an insulator such as, for example, alumina or magnesium oxide, in which case the structure is referred to as a magnetic tunnel junction (MTJ).

Additional materials may be deposited both below and above the first ferromagnetic layer 106A, second ferromagnetic layer 106B, and nonmagnetic spacer layer 107 shown in FIG. 3 to serve purposes such as interface smoothing, texturing, and/or protection from processing used to pattern the device into which the magnetic sensor 105 is incorporated. Moreover, as described further below, the magnetic sensor 105 may be encased in or covered by a material to protect it from fluids used in single-molecule analysis. Nevertheless, the active region of the magnetic sensor 105 lies in the trilayer structure illustrated in FIG. 3. Thus, a component (e.g., read circuitry) that is in contact with a magnetic sensor 105 may be in contact with one of the first ferromagnetic layer 106A, second ferromagnetic layer 106B, or nonmagnetic spacer layer 107, or it may be in contact with another part of the magnetic sensor 105.

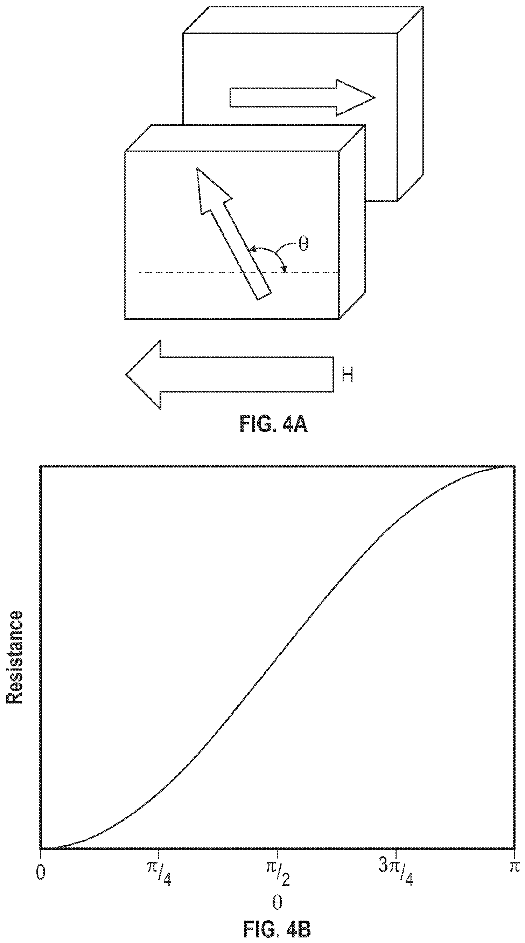

As shown in FIGS. 4A and 4B, the resistance of a magnetoresistive sensor (e.g., one possible type of magnetic sensor 105) is proportional to 1 - cos(*θ*), where *θ* is the angle between the moments of the first ferromagnetic layer 106A and second ferromagnetic layer 106B shown in FIG. 3. To maximize the signal generated by a magnetic field and provide a linear response of the magnetic sensor 105 to an applied magnetic field, the magnetic sensor 105 may be designed such that the moments of the first ferromagnetic layer 106A and second ferromagnetic layer 106B are oriented *π*/2 radians or 90 degrees with respect to one another in the absence of a magnetic field. This orientation can be achieved by any number of methods that are known in the art. For example, one solution is to use an antiferromagnet to "pin" the magnetization direction of one of the ferromagnetic layers (either the first ferromagnetic layer 106A or second ferromagnetic layer 106B, designated as "FM1") through an effect called exchange biasing and then coat the magnetic sensor 105 with a bilayer that has an insulating layer and permanent magnet. The insulating layer avoids electrical shorting of the magnetic sensor 105, and the permanent magnet supplies a "hard bias" magnetic field perpendicular to the pinned direction of FM1 that will then rotate the second ferromagnet (either the second ferromagnetic layer 106B or first ferromagnetic layer 106A, designated as "FM2") and produce the desired configuration. Magnetic fields parallel to FM1 then rotate FM2 about this 90 degree configuration, and the change in the resistance of the magnetic sensor 105 results in a voltage (or current) signal (e.g., sensor signal 207) that can be calibrated to measure the field acting upon the magnetic sensor 105. In this manner, the magnetic sensor 105 acts as a magnetic-field-to-voltage transducer.

For biosensing applications, the magnetic sensor 105 should be designed so that FM1 and FM2 are weakly coupled, and perturbations to the position of FM2 caused by the presence of a MNP 102 can be detected in the sensor signal 207. If the coupling between FM1 and FM2 is too strong, the presence of a MNP 102 will not create enough of a perturbation in the sensor signal 207 to be detected. If, on the other hand, the coupling between FM1 and FM2 is too weak, the magnetic sensor 105 may be thermally unstable such that thermal fluctuations dominate and degrade the signal-to-noise ratio (SNR). As will be explained further below, certain magnetic sensors 105 designed for use in magnetic recording have characteristics that allow them to be used for certain biosensing applications.

Note that although the example discussed immediately above describes the use of ferromagnets that have their moments oriented in the plane of the film at 90 degrees with respect to one another, a perpendicular configuration can alternatively be achieved by orienting the moment of one of the ferromagnetic layers (the first ferromagnetic layer 106A or second ferromagnetic layer 106B) out of the plane of the film, which may be accomplished using what is referred to as perpendicular magnetic anisotropy (PMA).

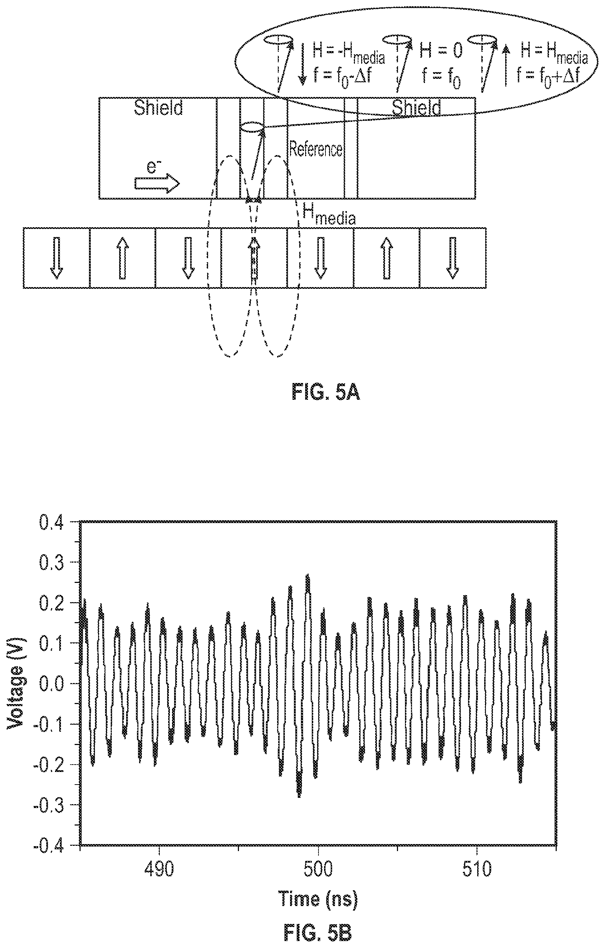

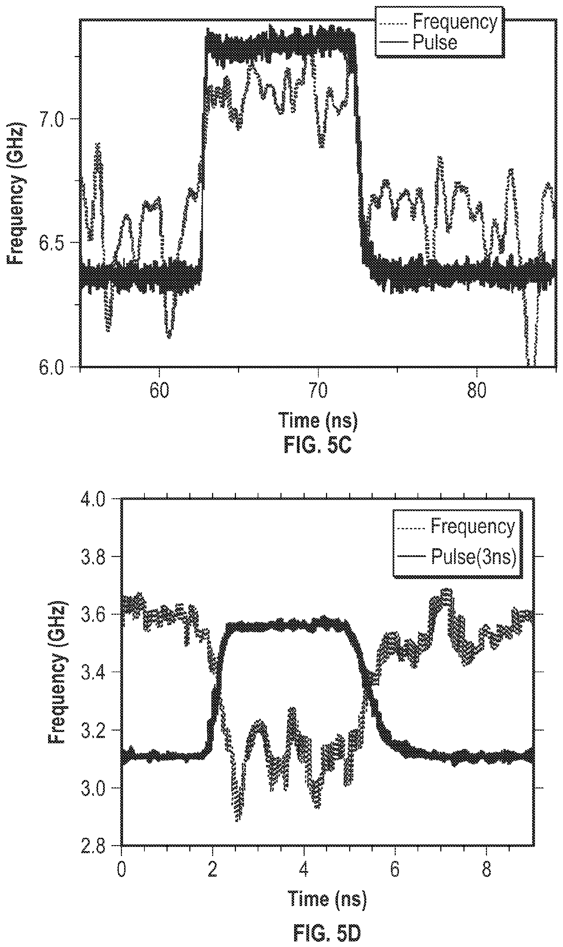

In some embodiments, the magnetic sensors 105 use a quantum mechanical effect known as spin transfer torque. In such magnetic sensors 105, the electrical current passing through the first ferromagnetic layer 106A (or, alternatively, the second ferromagnetic layer 106B) in a SV or a MTJ preferentially allows electrons with spin parallel to the layer's moment to transmit through, while electrons with spin antiparallel are more likely to be reflected. In this manner, the electrical current becomes spin polarized, with more electrons of one spin type than the other. This spin-polarized current then interacts with the second ferromagnetic layer 106B (or the first ferromagnetic layer 106A), exerting a torque on that layer's moment. This torque can in different circumstances either cause the moment of the second ferromagnetic layer 106B (or first ferromagnetic layer 106A) to precess around the effective magnetic field acting upon the ferromagnet, or it can cause the moment to reversibly switch between two orientations defined by a uniaxial anisotropy induced in the system. The resulting spin torque oscillators (STOs) are frequency-tunable by changing the magnetic field acting upon them. Thus, they have the capability to act as magnetic-field-to-frequency (or phase) transducers (thereby producing an AC signal having a frequency), as shown in FIG. 5A, which illustrates the concept of using a STO sensor in magnetic recording. FIG. 5B shows the experimental response of a STO through a delay detection circuit when an AC magnetic field with a frequency of 1 GHz and a peak-to-peak amplitude of 5 mT is applied across the STO. This result and those shown in FIGS. 5C and 5D for short nanosecond field pulses illustrate how these oscillators may be used as nanoscale magnetic field detectors. Further details may be found in T. Nagasawa, H. Suto, K. Kudo, T. Yang, K. Mizushima, and R. Sato, "Delay detection of frequency modulation signal from a spin-torque oscillator under a nanosecond-pulsed magnetic field," Journal of Applied Physics, Vol. 111, 07C908 (2012).

In some embodiments, the magnetic sensor 105 comprises a STO to sense magnetic fields caused by MNPs 102 coupled to biopolymers 101. The magnetic sensor 105 is configured to detect changes in, or a presence or absence of, a precessional oscillation frequency of a magnetization of a magnetic layer of the magnetic sensor 105 to sense the magnetic field of a MNP 102. The magnetic sensor 105 can include a magnetic free layer (e.g., first ferromagnetic layer 106A or second ferromagnetic layer 106B), a magnetic pinned layer (e.g., second ferromagnetic layer 106B or first ferromagnetic layer 106A), and a non-magnetic layer (e.g., nonmagnetic spacer layer 107) between the free and pinned layers as described above in the discussion of FIG. 3. In some embodiments, in operation, detection circuitry coupled to the magnetic sensor 105 induces an electrical (DC) current through the layers of the magnetic sensor 105. Spin polarization of electrons traveling through the magnetic sensor 105 causes a spin-torque-induced precession of the magnetization of one or more of the layers. The frequency of this oscillation changes in response to a magnetic field generated by a MNP 102 in the vicinity of the magnetic sensor 105. In some embodiments, changes in the frequency of oscillations of the sensor or noise in the oscillation frequency (referred to as phase noise or frequency noise) can be used to detect the presence of, absence of, or changes in the magnetic field and, therefore, the MNP 102.

In some embodiments, the magnetic sensor 105 comprises a MTJ, and changes in the resistance, current through, or voltage across of the magnetic sensor 105 are used to detect the presence, absence, or movement of a MNP 102 within the sensing region 206 of the magnetic sensor 105. For example, a MTJ similar to those used in hard disk drives is an example of a magnetic sensor 105 that is suitable for use in the devices, systems, and methods described herein. Such a magnetic sensor 105 can be used to monitor nanoscale changes in the motion patterns of any suitable MNP 102, such as, for example, 20 nm superparamagnetic iron oxide nanoparticles, as described further below. It is to be understood that other MNPs 102, e.g., of Fe₃O₄ and FePt, may also be used, but the experimental results below are for iron oxide nanoparticles because other particles (e.g., Fe₃O₄ and FePt) may be more challenging to functionalize for tethering and difficult or impossible to image using scanning electron microscopy to confirm the presence of a MNP 102 in the sensing region 206. Similarly, MNPs 102 that are larger or smaller than 20 nm can be used.

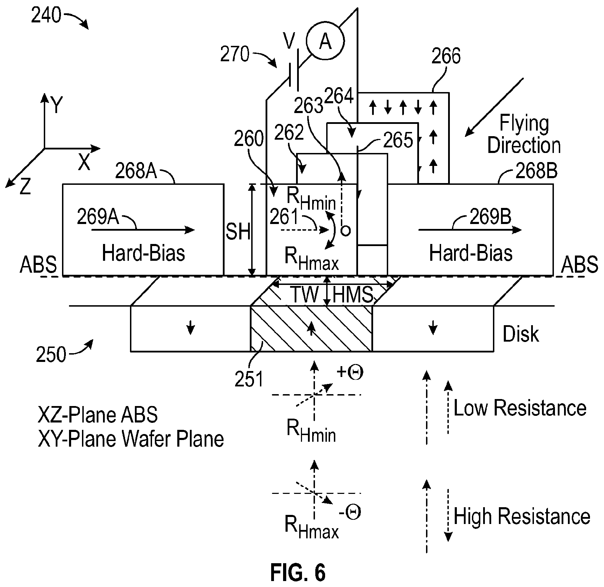

To explain certain concepts applicable to the magnetic sensor 105 used in the devices, systems, and methods described herein, FIG. 6 illustrates the operation of a magnetic sensor that can read data previously recorded on a magnetic recording medium. Specifically, FIG. 6 is a diagram of a portion of an exemplary read head 240 that includes a magnetic sensor used in a perpendicular magnetic recording (PMR) application. The surface of the recording medium 250 is in the x-z plane, as is the air-bearing surface (ABS) of the exemplary read head 240 that reads the information stored on the recording medium 250. The recording medium 250 may have multiple concentric tracks onto which information can be recorded, including track 251, which is the track being read in FIG. 6. The exemplary read head 240 includes multiple layers in the wafer plane, which is the x-y plane in using the coordinates shown in FIG. 6. The multiple layers include the free layer 260, the reference layer 262, and the pinned layer 264. The free layer 260, the reference layer 262, and the pinned layer 264 may correspond, respectively, to the first ferromagnetic layer 106A, nonmagnetic spacer layer 107, and second ferromagnetic layer 106B (or, equivalently, to the second ferromagnetic layer 106B, nonmagnetic spacer layer 107, and first ferromagnetic layer 106A) described above. The magnetic moment 263 of the reference layer 262 is in a particular direction, shown as being in the positive-y direction in FIG. 6. The magnetic moment 265 of the pinned layer 264 may be pinned (fixed in a particular direction) by an antiferromagnet 266, as described above. In FIG. 6, the magnetic moment 265 of the pinned layer 264 is pinned in the negative-y direction. The magnetic moment 261 of the free layer 260 is free to rotate in response to applied or induced magnetic fields. Hard bias regions 268A and 268B may be situated laterally (in what are referred to as the side track directions) from the free layer 260, reference layer 262, and/or pinned layer 264 to supply a magnetic field perpendicular to the direction of the magnetic moment 265 of the pinned layer 264. In FIG. 6, the moments 269A, 269B of the hard bias regions 268A, 268B are oriented to the right of the page in the positive x direction. Circuitry 270 coupled to the layers provides a bias voltage (or, equivalently, bias current) to read the information stored on the recording medium 250.

As shown in FIG. 6, the magnetic moment 261 of the free layer 260 is oriented in some default or equilibrium direction, which, in FIG. 6, is to the right of the page, along the x axis, perpendicular to the magnetic moment 263 of the reference layer 262 and perpendicular to the magnetic moment 265 of the pinned layer 264. As shown in FIG. 6, when the "bit" on the recording medium 250 causes a magnetic field that points upward, toward the exemplary read head 240, the magnetic moment 261 of the free layer 260 rotates upward, constructively adding a component to the magnetic field generated by the bias applied to the exemplary read head 240 by the circuitry 270. As a consequence, the resistance of the exemplary read head 240 decreases. Conversely, when the "bit" on the recording medium 250 causes a magnetic field that points downward, away from the exemplary read head 240, the magnetic moment 261 of the free layer 260 rotates downward, in the opposite direction, thereby adding a destructive component to the magnetic field generated by the bias applied by the circuitry 270. As a result, the resistance of the exemplary read head 240 increases. The changes in resistance therefore indicate which of the two possible "bits" (up or down, which may be interpreted as 0 or 1 (or vice versa)) on the recording medium 250 has been detected.

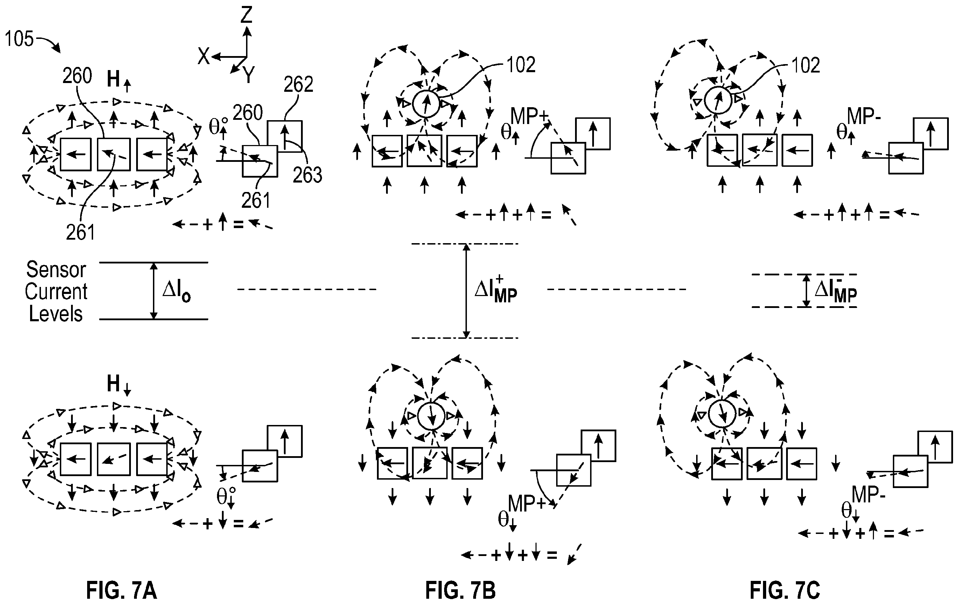

FIGS. 7A, 7B, and 7C illustrate how these same principles can be applied in single-molecule sensing devices, systems, and methods in accordance with some embodiments disclosed herein. FIG. 7A illustrates portions of a magnetic sensor 105 without any MNP 102 in its vicinity. In the presence of an applied magnetic field H oriented in the positive z direction (e.g., caused by a bias voltage), the magnetic moment 261 of the free layer 260 is oriented in the direction shown in the upper panel of FIG. 7A, at an angle ${θ}_{↑}^{O}$ from the x axis. If the applied magnetic field *H* is oriented in the negative z direction, the magnetic moment 261 of the free layer 260 is oriented in the direction shown in the lower panel of FIG. 7A, at an angle ${θ}_{↓}^{O}$ from the x axis. Thus, the peak-to-peak current (e.g., the difference in amplitude under these conditions when the direction of the applied magnetic field is reversed) sensed by the magnetic sensor 105 under the illustrated conditions is given by Δ*I_{O}.* Thus, Δ*I_{O}* provides baseline peak positive and negative current amplitudes for the magnetic sensor 105 in the absence of any MNP 102.

FIG. 7B illustrates the magnetic sensor 105 with a MNP 102 situated directly above the free layer 260 of the magnetic sensor 105 (in the z direction). As shown in the upper panel, the applied magnetic field H in the positive z direction causes the magnetic moment of the MNP 102 to become oriented substantially in the same direction as the applied magnetic field H. As a result, at the location of the free layer 260, the magnetic field caused by the MNP 102 adds constructively to the applied magnetic field *H,* and the magnetic moment 261 of the free layer 260 rotates closer to the direction of the applied magnetic field *H,* now at an angle ${θ}_{↑}^{MP +}$ from the x axis. If the applied magnetic field *H* is oriented in the negative z direction, the magnetic moment 261 of the free layer 260 rotates to the direction shown in the lower panel of FIG. 7B, at an angle ${θ}_{↓}^{MP +}$ from the x axis, because the magnetic field caused by the MNP 102 adds constructively to the applied magnetic field *H*. The peak-to-peak amplitude of the current sensed by the magnetic sensor 105 under these conditions is given by $Δ {I}_{MP}^{+}$ (where "MP" stands for "magnetic particle"). Because the magnetic moment 261 of the free layer 260 is more closely aligned with the applied magnetic field *H* than in the case illustrated in FIG. 7A, the resistance of the magnetic sensor 105 is reduced relative to its value in FIG. 7A, and $Δ {I}_{MP}^{+} > Δ {I}_{O}$*.*

FIG. 7C illustrates the magnetic sensor 105 with a MNP 102 laterally offset from the free layer 260 of the magnetic sensor 105 (specifically, offset in the x direction). As shown in the upper panel of FIG. 7C, the applied magnetic field *H* in the positive z direction causes the magnetic moment of the MNP 102 to become oriented substantially in the same direction as the applied magnetic field *H*. Now, however, because the MNP 102 is laterally offset from the free layer 260, the magnetic field caused by the MNP 102 is in the direction opposite to the applied magnetic field *H* at the location of the free layer 260. Thus, the magnetic field caused by the MNP 102 reduces the effect of the applied magnetic field *H* on the free layer 260, and the magnetic moment 261 of the free layer 260 rotates away from its direction in FIG. 7B. Now the magnetic moment 261 of the free layer 260 is at an angle ${θ}_{↑}^{MP -}$ from the x axis. Similarly, when the applied magnetic field *H* is oriented in the negative z direction, the magnetic moment 261 of the free layer 260 rotates as shown in the lower panel of FIG. 7B, at an angle ${θ}_{↓}^{MP -}$ from the x axis, due to the magnetic field of the MNP 102 detracting from the applied magnetic field *H* at the location of the free layer 260. In this case, the peak-to-peak current amplitude sensed by the magnetic sensor 105 decreases to $Δ {I}_{MP}^{-}$*,* where $Δ {I}_{MP}^{-} < Δ {I}_{O} < {I}_{MP}^{+}$*.*

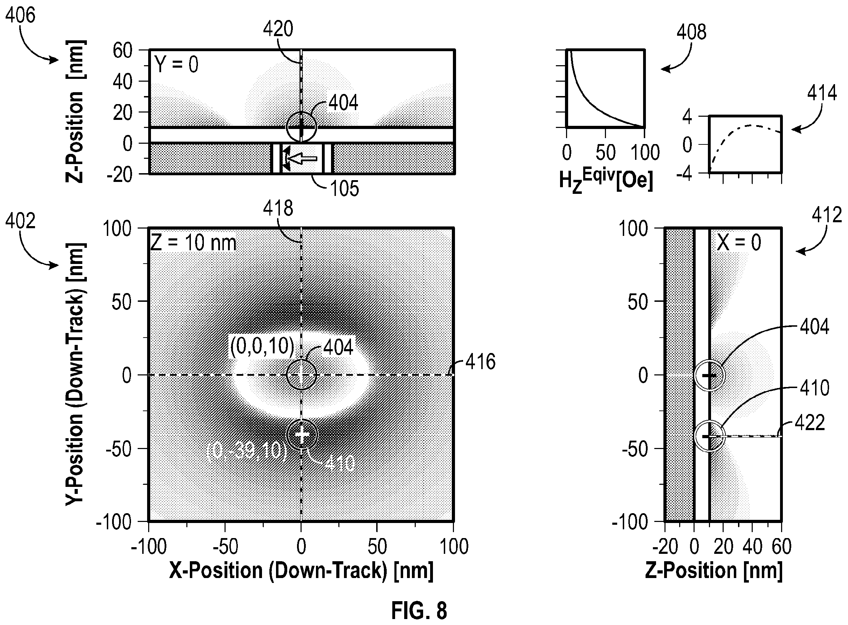

Thus, by monitoring the current through the magnetic sensor 105 (or any proxy for current, such as resistance or voltage; or, in the case of a different type of magnetic sensor 105, some other characteristic that represents the magnetic environment sensed by the magnetic sensor 105), the presence and position of the MNP 102 relative to the free layer 260 (and, therefore, the magnetic sensor 105) can be detected and monitored, as described further below. FIG. 8 illustrates the results of nanomagnetic simulations of an exemplary magnetic sensor 105 in the presence of a MNP 102 at various positions relative to the magnetic sensor 105 in accordance with some embodiments. The contour plot 402 illustrates the magnetic field acting on the magnetic sensor 105 for various lateral positions of the MNP 102 in the x-y plane of FIGS. 7A, 7B, and 7C when the MNP 102 is 10 nm above the x-y plane (at a z value of 10 nm). As indicated by the cross section 406, the magnetic sensor 105 is centered at coordinates (0, 0) in the x-y plane, indicated as position 404. The cross section 406 shows the magnetic field magnitude as a function of the lateral position of the MNP 102 along the x-axis at a position of y = 0 (indicated by the dashed line 416 in the contour plot 402) and at various positions along the z-axis, ranging from 10 nm to 60 nm away from the surface of the magnetic sensor 105. The plot 408 shows the magnetic field magnitude along the dashed line 420 in the cross section 406. As shown, when the MNP 102 is 10 nm directly above the magnetic sensor 105, the magnetic field amplitude is approximately 10 mT (100 Oersted) and when the MNP 102 is 60 nm above the magnetic sensor 105, the magnetic field amplitude is near 0.

The cross section 412 shows the magnetic field magnitude as a function of the lateral position of the MNP 102 along the y-axis at a position of x = 0 (indicated by the dashed line 418 of the contour plot 402) and at various positions along the z-axis, ranging from 10 nm to 60 nm away from the surface of the magnetic sensor 105. The plot 414 shows the magnetic field magnitude along the dashed line 422 in the cross section 412, at the position 410 shown in contour plot 402, which is at a lateral offset of 39 nm along the y-axis. As shown, when the MNP 102 is 10 nm above the surface of the magnetic sensor 105 and laterally offset by 39 nm, the magnetic field amplitude is approximately -0.4 mT (-4 Oersted), and when the MNP 102 is 60 nm above the magnetic sensor 105 and laterally offset by 39 nm, the magnetic field amplitude is near 0. Thus, FIG. 8 illustrates that the magnitude of the magnetic field changes substantially as the MNP 102 changes position in three-dimensional space. Even slight changes in position cause significant changes in the detected magnetic field. Both its amplitude and direction change, and these changes can be detected by a free layer 260 of the magnetic sensor 105. Therefore, the position of the MNP 102 can be inferred by interpreting signals from the magnetic sensor 105 rather than observed directly using an imaging system.

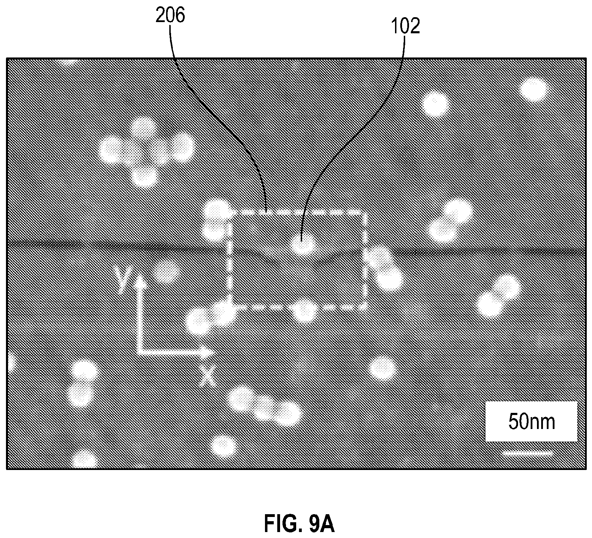

FIG. 9A is a plane view scanning electron microscopy (SEM) image of an exemplary magnetic sensor 105 that is an MTJ with a surface area in the x-y plane of approximately 30 × 40 nm² with a MNP 102 bound within the sensing region 206 (the dashed-line marks the estimated or approximate boundary of the sensing region 206 in the x-y plane). In the example embodiment shown, the junction area is parallel to the x-z plane (out of the page), and the tunneling current flows in the y-axis direction. FIG. 9A shows a single 20 nm MNP 102 within the sensing region 206. The effective sensing region 206 of the exemplary magnetic sensor 105, originally developed for magnetic recording applications, is designed to be extremely small (e.g., between about 10⁵ nm³ and about 5 × 10⁵ nm³) to detect magnetization orientation of small magnetic domains in recording media and to maximize the density of magnetic recording. Thus, it is well suited for the detection of stochastic motion of MNPs 102 as described herein. It is to be understood that the volume of the sensing region 206 can be any suitable value, and the range given above is merely an example.

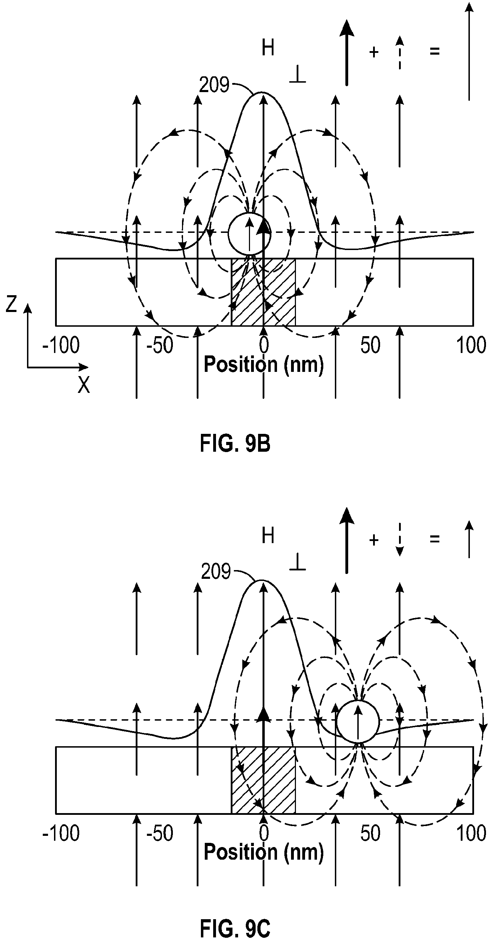

FIGS. 9B and 9C illustrate cross-section views of a magnetic sensor 105 showing an external magnetic field *H* applied perpendicular to the surface of the magnetic sensor 105. In FIG. 9B, the MNP 102 (which is depicted as a circle but is unlabeled to avoid obscuring the drawing) is immobilized above the magnetic sensor 105 (which is also unlabeled but is shown with a diagonal-line fill), and, in the vicinity of the magnetic sensor 105, the magnetic field-lines are aligned with the external field, which is shown as a thick arrow in the sensor area. As described above, the effective field measured by the magnetic sensor 105 increases when the MNP 102 is present because the magnetic fields add constructively.

In FIG. 9C, the MNP 102 (which is depicted as a circle but is unlabeled to avoid obscuring the drawing) is placed a lateral distance away from the magnetic sensor 105 (which, again, is also unlabeled but is shown with a diagonal fill), and the magnetic field-lines affecting the free layer 260 point in the direction opposite to the external field. In this case, as described above, the effective magnetic field measured by the magnetic sensor 105 decreases. The perturbation to the sensor signal 207 due to the presence of the MNP 102 thus changes quickly from positive to negative as the MNP 102 moves laterally away from the magnetic sensor 105. As shown by FIGS. 9B and 9C, the magnetic field perturbations are extremely sensitive to the position of the MNP 102 relative to the magnetic sensor 105. Magnetic field lines of the MNP 102 align with the external magnetic field when the MNP 102 is above the magnetic sensor 105 as shown in FIG. 9B, but they point in the opposite direction when the MNP 102 is displaced laterally as shown in FIG. 9C.

The effect of the movement of the MNP 102 on the sensor signal 207 is illustrated schematically by the curve 209 in FIGS. 9B and 9C. A MNP 102 tethered in the proximity of the magnetic sensor 105 induces dynamic stochastic perturbations in the sensor signal 207 as the MNP 102 moves around while an external magnetic field is applied to fix the magnetic moment of the MNP 102 in a particular direction. The response of the magnetic sensor 105 is affected by both the in-plane (within x-y plane) and out-of-plane (along the z-axis) motion of the MNP 102. The presence of a MNP 102 with sufficiently-high magnetic moments can be detected by the magnetic sensor 105 even when no external field is applied. In other words, the disclosed embodiments can be used with, for example, superparamagnetic and ferromagnetic MNPs.

In video imaging systems used in conventional TPM systems, the consequences of time averaging (exposure time) and the frequency of the observations (frame rate) are well understood. Although exposure time and frame rate do not limit tracking of freely diffusing Brownian particles, they do severely affect observation of particles undergoing anomalous (or confined) diffusion, such as a tethered nanoparticle in a biological system. The time averaging in the imaging of such a particle can have serious consequences on the apparent characteristics of the reported motion because the observed velocity depends on the duration of observation. In the extreme case when the exposure time is too long, the particle will be blurred and will appear stationary in some equilibrium position. These drawbacks can be mitigated or overcome by the systems, devices, and methods using magnetic sensors 105 described herein.

The ability of a magnetic sensor 105 to detect changes in the sensor signal 207 depends on the responsiveness of detection circuitry (e.g., detecting amplifier circuitry, other detection electronics, as described below). For example, if the response of the magnetic sensor 105 is too slow (e.g., due to limitations of detection circuitry, such as, for example, sampling rate), a monitoring device or system may be able to detect when the MNP 102 moves to a different equilibrium position during the processes illustrated in FIGS. 2C and 2D, but it might not be able to detect processes that do not affect the equilibrium position but change the statistical velocity of the MNP 102, such as, for example, the molecular binding and conformational changes shown in FIGS. 2A and 2B.

Unlike a video imaging system that generates a series of particle images to track a particle's position in both space and time, a magnetic sensor 105 generates a time response to a random series of similar (but not identical) shocks or pulses caused by molecules of a solution bombarding the MNP 102. A freely diffusing MNP 102 can be considered to estimate the response time and sampling rate of a magnetic sensor 105 that can detect MNP 102 motion. A freely diffusing MNP 102 is a good first approximation for the case of a MNP 102 tethered to the surface of the magnetic sensor 105 by a long, flexible polymer (e.g., a biopolymer 101). It is assumed that the polymer length is considerably longer than the dimension of the sensing region 206. This constraint increases the probability of detection by preventing the MNP 102 from diffusing too far away from the magnetic sensor 105 (e.g., out of the sensing region 206 for an extended period of time) but does not otherwise constrain its motion, which can still be regarded as simple Brownian motion.

The random movement of a particle in a fluid due to collisions with the molecules of the fluid can be described mathematically by solving the Langevin equation. It is the equation of motion with a velocity damping term that accounts for viscosity or friction. The particle mean-square displacement (MSD) at short-time scales is given by: $\left〈x{\left(t\right)}^{2}\right〉 = \frac{3 {k}_{B} T}{m} {t}^{2}$*,* where *k_{B}* is the Boltzmann constant, *T* is temperature, m is the particle mass and *t* is the time of observation. This essentially describes free particle motion under thermodynamic equilibrium with the average velocity of about $\sqrt{\frac{3 {k}_{B} T}{m}}$. The value of *k_{B}T* at room temperature (RT) (298K) is 4.11 × 10⁻²¹ J, and the example MNP 102 of iron oxide has density of about 5 g/cm³. This puts the mass of a 20 nm spherical particle approximately at 2 × 10⁻²⁰ kg, giving an average particle velocity of about 0.8 m/s. This is considerably greater than visually observed velocities of colloidal nanoparticles of this size. Such velocity could only be measured using an instrument with sub-nanometer spatial resolution and limiting response time below the relaxation time (*τ_{B}*) of a particle experiencing average drag force imparted by the surrounding liquid. The particle initial velocity would decrease as $v \left(t\right) = {v}_{o} {e}^{\frac{- t}{{τ}_{B}}}$ and the relaxation time is related to the viscosity of the fluid (*η*) by: ${τ}_{B} = \frac{m}{6 πηa}$ , where *a* is the particle radius. Substituting in the viscosity of water ( $η = 8.9 \times {10}^{- 4} \frac{N}{s \times {m}^{2}}$ at room temperature) yields the relaxation time of approximately 0.1 ns, which is below the response time of video imaging systems but is within the reach of some magnetic sensors 105. At longer timescales (t ≫ *τ_{B}*) the particle MSD grows linearly in time as: $\left〈x{\left(t\right)}^{2}\right〉 = \frac{2 {k}_{B} T}{6 πηa} t = 2 Dt$*.* This describes the random diffusion due to collisions with water molecules. D is the microscopic diffusion coefficient from the Stokes-Einstein equation. The Brownian motion of a 20 nm iron oxide MNP 102, *D* ≅ 2.5 × 10⁷ nm²/s, is fairly fast (approximately 0.25 mm/s), and it would take the particle on average about 0.2 ms to diffuse over an approximate 100 × 130 nm effective sensing region. This falls well within the range of properly designed commercial magnetic sensors 105 that can operate in the gigahertz regime, e.g., with the response time in nanoseconds.

The response of a magnetic sensor 105 to the motion of a tightly confined nanoparticle (e.g., tether length ≈ magnetic sensor 105 sensing region 206 size ≈ MNP 102 size) is considerably more difficult to interpret. The MNP 102 is diffusing only locally within the sensing region 206, and its apparent diffusion coefficient (free-diffusion equivalent) is significantly affected by time averaging. The arriving signal pulses (e.g., of the sensor signal 207) due to motion of the MNP 102 are neither discrete nor well defined. The MNP 102 motion generates another source of random noise that is added to the intrinsic magnetic sensor 105 noise and changes the noise characteristics of the detected sensor signal 207. To detect changes of MNP 102 motion, the difference between the signal spectrum and noise spectrum over the sensing bandwidth can be exploited as described further below. Various advanced sensing schemes such as energy detection or autocorrelation are developed and implemented as described below to improve detection in low signal-to-noise ratio (SNR) conditions.

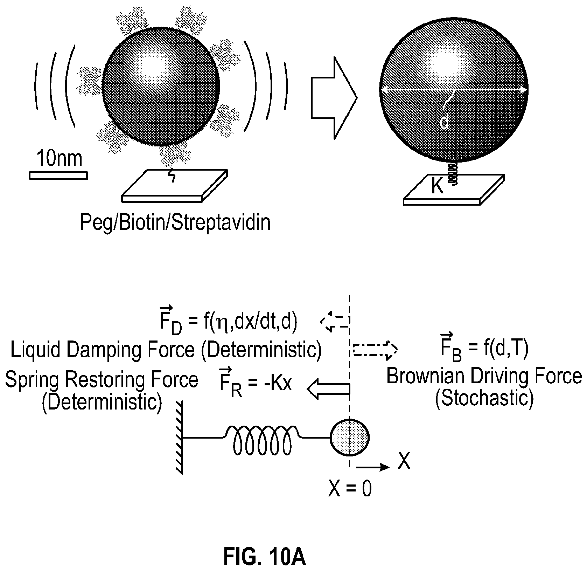

A physics problem can be defined to assist in understanding how the presence and position of a MNP 102 affects a magnetic sensor 105. FIG. 10A presents an example model. A MNP 102 is attached to a surface of a magnetic sensor 105 by a tether. (It is to be appreciated, and is explained further elsewhere herein, that the surface of the magnetic sensor 105 itself may in fact be physically separated from the tether (e.g., biopolymer 101), the MNP 102, and any fluids acting on the MNP 102 by some kind of protective barrier, such as an insulator. It is to be understood that when this document refers to "the surface of a magnetic sensor 105," it is for simplicity, and that the surface of the magnetic sensor 105 might not be exposed but is physically nearby.) For example, the tether (biopolymer 101) might comprise peg/biotin/streptavidin as shown in FIG. 10A. As molecules of the surrounding solution collide with the MNP 102, the MNP 102 moves via stochastic Brownian perturbation. The motion can be approximated as a one-dimensional harmonic potential. Specifically, as shown in FIG. 10A, the MNP 102 can be considered as a mass on a spring (e.g., biopolymer 101). Ignoring gravity, the driving force is Brownian and stochastic, caused by the molecules of the surrounding solution colliding with the MNP 102. The Brownian driving force is a function of the diameter of the MNP 102 and the temperature in degrees Kelvin, and it can be represented as *F̅_{B} = f*(*d*, *T*). The spring restoring force and the liquid damping force, both of which are deterministic, oppose the driving force. The spring restoring force can be represented as *F̅_{R} = -Kx,* where *K* is the spring constant of the molecular tether (e.g., biopolymer 101), and x is the position of the MNP 102. The deterministic liquid damping force can be represented as ${\vec{F}}_{D} = f \left(η, \frac{dx}{dt}, d\right)$ *,* where *η* is the dynamic viscosity of the surrounding liquid (for water at room temperature, it is approximately $8.9 \times {10}^{- 4} \frac{N}{s \times {m}^{2}}$), and d is the diameter of the MNP 102.

The one-dimensional time evolution of the distribution probability P of a diffusing spherical particle at the position *x* and at time *t* given an initial position *x*₀ at time *t*₀ in a harmonic potential field is given by the equation of motion: $\frac{\partial }{\partial t} P \left(x,\left.t\right|{x}_{0},{t}_{0}\right) = \frac{1}{3 πηd} \left({k}_{B}T\frac{{\partial }^{2}}{\partial {x}^{2}}+K\frac{\partial }{\partial x}x\right) P \left(x,\left.t\right|{x}_{0},{t}_{0}\right)$ which has the solution $P \left(x,t\left|{x}_{0}, {t}_{0}\right.\right) = \sqrt{\frac{K}{2 {πk}_{b} TS \left(t, {t}_{0}\right)}} exp \left(-\frac{{\left(x-{x}_{0}{e}^{- 2 \left(t-{t}_{0}\right) / τ}\right)}^{2}}{2 {k}_{b} TS \left(t, {t}_{0}\right) / K}\right)$ where $S \left(t, {t}_{0}\right) = 1 - exp \left[-\frac{4 \left(t-{t}_{0}\right)}{τ}\right]$, and the relaxation time *τ* is $τ = 6 πη \left(\frac{d}{K}\right)$*.* The relaxation time *τ* is related to what is referred to herein as the corner frequency, *f_{c},* in the power spectral density (PSD), where *f_{c}* = 1/*πτ.* Therefore, the corner frequency can be approximated as ${f}_{c} ≅ \frac{1}{6 {π}^{2} η} \left(\frac{K}{d}\right)$

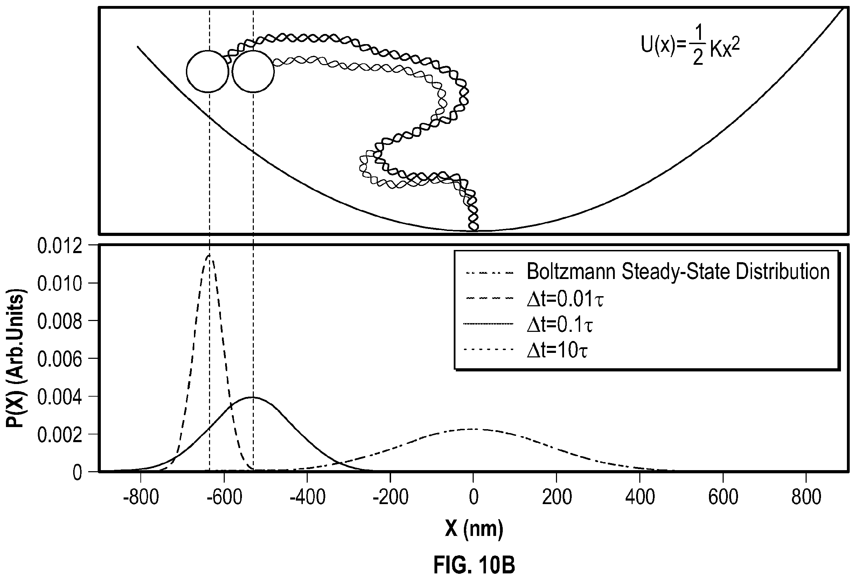

FIG. 10B is a reproduction of figure 1 of the paper by M. Lindner *et al.* entitled "Dynamic analysis of a diffusing particle in a trapping potential." (See M. Lindner et al., "Dynamic analysis of a diffusing particle in a trapping potential," Physical Review E 87, 022716 (2013).) FIG. 10B is a pictorial representation of a single particle diffusing in a harmonic potential applied by a DNA strand. The upper panel shows two conformations, and the lower panel shows the Boltzmann steady-state distribution as well as probability distributions for values of *Δt* ≡ (*t - t*₀) of 0.01*τ*, 0.1*τ*, and 10*τ* with a value of *x*₀ = -650 nm. Thus, the lower panel provides the probability that the MNP 102 will occupy a particular position at a particular time.

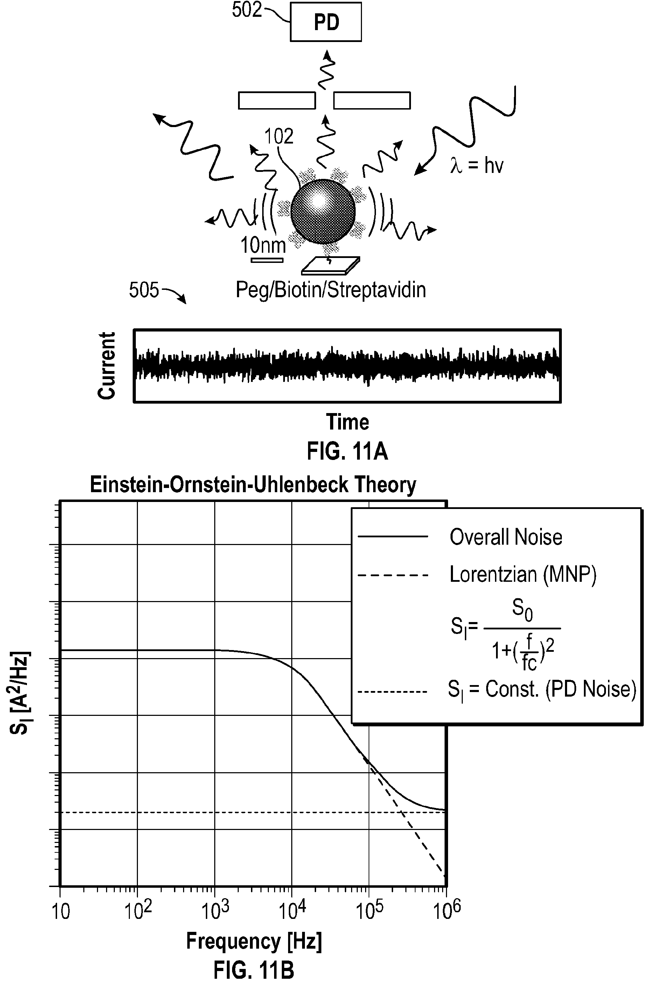

To illustrate how the presence and movement of the MNP 102 affects the sensor signal 207 provided by a magnetic sensor 105, consider first a thought experiment using an optical approach, as illustrated in FIG. 11A. Assume that the MNP 102 has a 20 nm diameter and is bound to the surface of a device by a tether (e.g., peg/biotin/streptavidin). Assume further that a light source exists that can generate light at a wavelength that is comparable to the diameter of the MNP 102, and that a photodiode 502 detects photons reflected in a particular direction by a MNP 102 bound to the surface of the device. If the MNP 102 is stationary and is illuminated by the light source, the intensity of reflected light will remain constant over time. Thus, the PSD of the photodiode 502 signal 505 will provide an indication of the noise contributed by the photodiode 502. In other words, as long as the MNP 102 is not moving, the noise in the photodiode 502 signal will be entirely due to the characteristics of the photodiode 502. Assuming that the noise floor of the photodiode 502 is white (e.g., thermal noise or Johnson-Nyquist noise), the spectrum of the noise is approximately flat at some low level, as shown by the short-dash line in FIG. 11B. When the MNP 102 is allowed to move, the stochastic perturbations cause the MNP 102 to move in confined Brownian motion (because the tether prevents it from floating away). The PSD of confined Brownian motion is a Lorentzian function, which has a PSD in the form of ${S}_{I} = \frac{{S}_{O}}{1 + {\left(\frac{f}{{f}_{c}}\right)}^{2}}$ where, as explained above, the corner frequency ${f}_{c} ≅ \frac{1}{6 {π}^{2} η} \left(\frac{K}{d}\right)$*.* Referring again to FIG. 11B, the overall PSD of the photodiode 502 signal 505 when the MNP 102 is allowed to move in confined Brownian motion is the sum of the white noise of the photodiode 502 itself and the Lorentzian function due to the confined Brownian motion of the MNP 102. The overall noise PSD has a lower-frequency shoulder around 10 kHz (the corner frequency) and a higher-frequency shoulder around 300 kHz where the noise floor of the photodiode 502 begins to dominate the overall noise PSD.

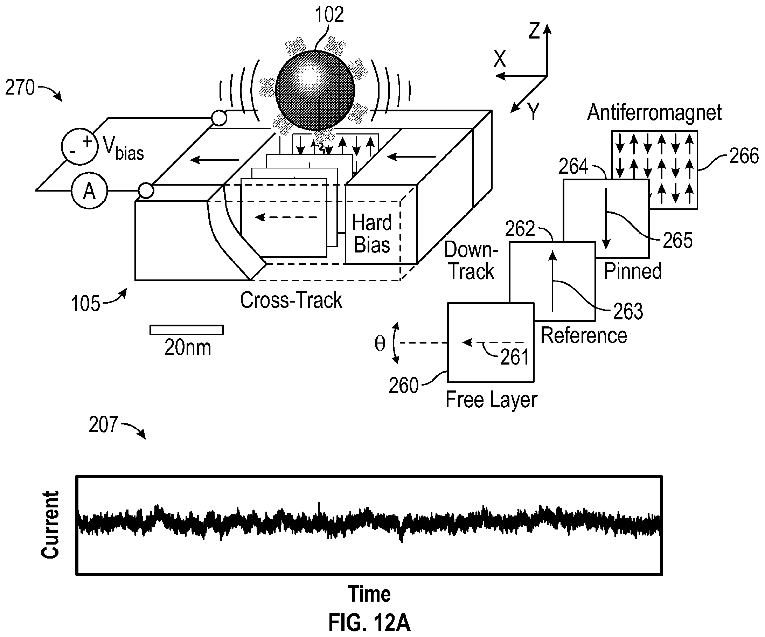

Knowing that the PSD (which may be considered a signature) of confined Brownian motion is a Lorentzian function, the expected PSD of the sensor signal 207 from a magnetic sensor 105 in the absence of a moving MNP 102 and in the presence of a moving MNP 102 can be determined in a similar fashion by first considering the noise PSD of the magnetic sensor 105 without any MNP 102 in its vicinity, and then assessing what the effect of the MNP 102 should be on that noise PSD. FIG. 12A illustrates an exemplary magnetic sensor 105 having a configuration similar to that described previously in the discussion of FIG. 6. The explanation of the components of FIG. 6 that are also shown in FIG. 12A applies to FIG. 12A and is not repeated.

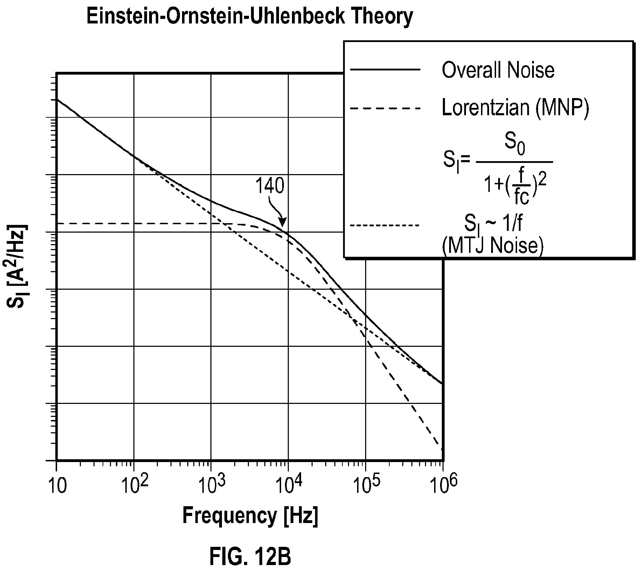

The noise PSD of a perfect MTJ exhibits 1/*f* behavior (it decreases by 10 dB/decade). FIG. 12B plots the expected noise PSD of an example magnetic sensor 105 that is a perfect MTJ, driven by a selected bias voltage (discussed further below), and the Lorentzian function characterizing the PSD of the confined Brownian motion of the MNP 102. In the example of FIG. 12B, the Lorentzian function exceeds the magnetic sensor 105 noise PSD in a frequency range between about 2 kHz and about 70 kHz. As a result, on a log/log scale, the overall PSD has a discernable "bump," labeled 140, in this frequency range. Thus, if a magnetic sensor 105 is sensitive to the presence of a MNP 102, that sensitivity will manifest as a discernible bump 140 in the PSD of the sensor signal 207. As discussed in further detail below, whether and in what frequency range the Lorentzian function exceeds the magnetic sensor 105 noise PSD depends on various factors, including the design of the magnetic sensor 105 and the bias voltage (or current) used to drive the magnetic sensor 105, as well as the factors discussed above that determine the corner frequency of the Lorentzian function (e.g., the spring constant of the molecular tether, the diameter of the MNP 102, the dynamic viscosity of the liquid surrounding the MNP 102).

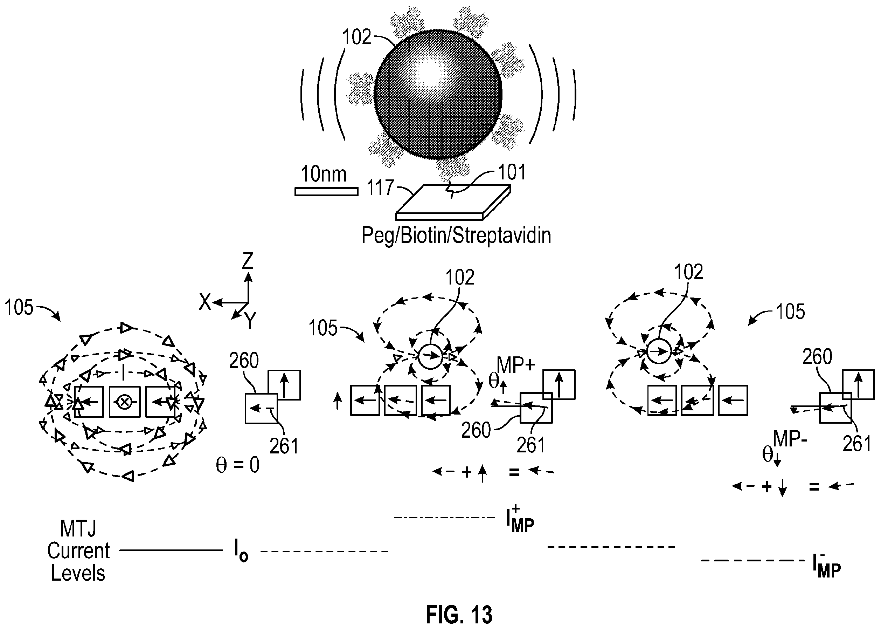

To verify the theoretical analysis presented above, the inventors performed experiments using magnetic sensors 105 in the form of MTJs to determine whether the PSDs of the collected sensor signals 207 do in fact exhibit the behavior derived above. FIG. 13 is a pictorial illustration of the experiments. First, as shown by the left-most panel, an external magnetic field was applied, and the sensor signal 207 was captured to determine the noise PSD of the magnetic sensor 105 in the absence of any MNP 102 (which, as described above, ideally has a 1/*f* profile). Next, the external magnetic field was turned off, and a MNP 102 (20 nm diameter) was tethered to the surface 117 using peg/biotin/streptavidin as described above. A bias voltage was applied to the magnetic sensor 105, which caused a magnetic field in the vicinity of the magnetic sensor 105. In response to this magnetic field, the magnetization of the MNP 102 oriented itself in alignment with the magnetic field and then moved in constrained Brownian motion as described above. The sensor signal 207 was captured to capture the dipole interaction between the magnetic moment of the magnetic sensor 105 and the magnetic moment 261 of the free layer 260 of the magnetic sensor 105 as the MNP 102 moved around, as illustrated graphically in the center and rightmost panels of FIG. 13.

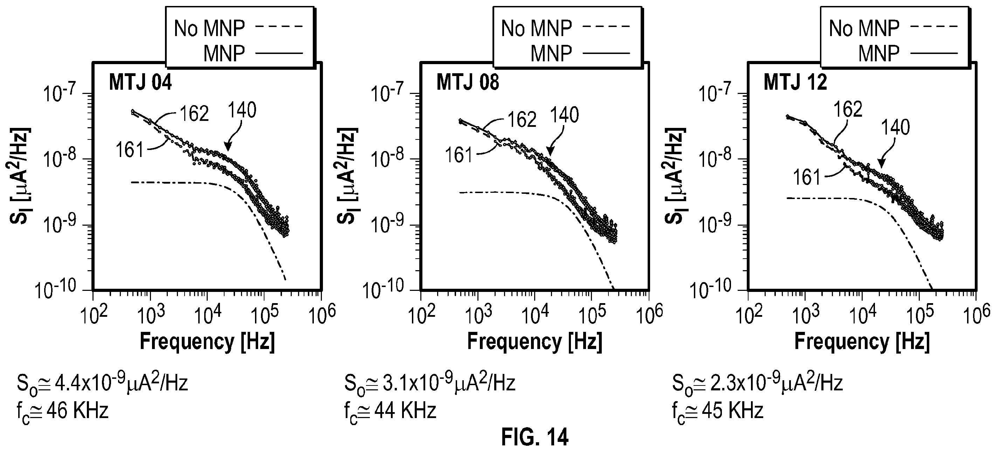

FIG. 14 illustrates the measured PSDs of three tested magnetic sensors 105. Each dash-line with circles, labeled 161, is the noise PSD of one of the tested magnetic sensors 105 (absent any MNP 102), and each solid line with diamonds, labeled 162, is the combined PSD of the MNP 102 and the magnetic sensor 105. As shown by the plots in FIG. 14, each of the combined PSDs has the characteristic bump 140 expected when a MNP 102 is detected. Thus, the experiments confirmed that for a bias voltage of around 10 mV, the tethered MNP 102 behaves like a particle confined in a harmonic potential. In addition, its PSD can be represented by a Lorentzian function in the approximately 488 Hz to 120 kHz range, as shown in FIG. 14. As indicated in FIG. 14, the corner frequency for each of the Lorentzian functions is slightly different for different magnetic sensors 105, but all of them are around 45 kHz. Although FIG. 14 shows data from only three example magnetic sensors 105, other tested magnetic sensors 105 behaved similarly. In all of the experiments, the corner frequency of the Lorentzian function due to the confined Brownian motion of the MNPs 102 was found to be approximately 45 kHz.

As explained above, the corner frequency is dependent on the selected tether (e.g., biopolymer 101) and, specifically, its spring constant. The polymer tether can be considered as an "entropic" spring, as described by P-G. de Gennes in "Scaling Concepts in Polymer Physics" (Cornell University Press, Ithaca, 1979). Stretching or compressing the coil away from its equilibrium size decreases the number of possible conformations and, thus, the entropy. As a result, the free energy increases. The free energy is quadratic in the change of chain size, and the spring constant is given by $K = \frac{3 {k}_{B} T}{{R}^{2}} ≅ 0.5 pN / nm$ where R is the size of the coil, *T* is the temperature, and *k_{B}* is Boltzmann's constant. In some embodiments, it is desirable to use soft and short molecular tethers both to hold the MNP 102 in the sensing region 206 of the magnetic sensor 105, and also to keep the corner frequency (and therefore the sampling rate and associated analog-to-digital complexity of the system) reasonable for small MNP 102. In addition to the peg/biotin/streptavidin tethers described previously, RNA, neutrophil microvilli, PEG₃₃₀₀, PEG₆₂₆₀, and poly(styrene) are all examples of suitable tethers.

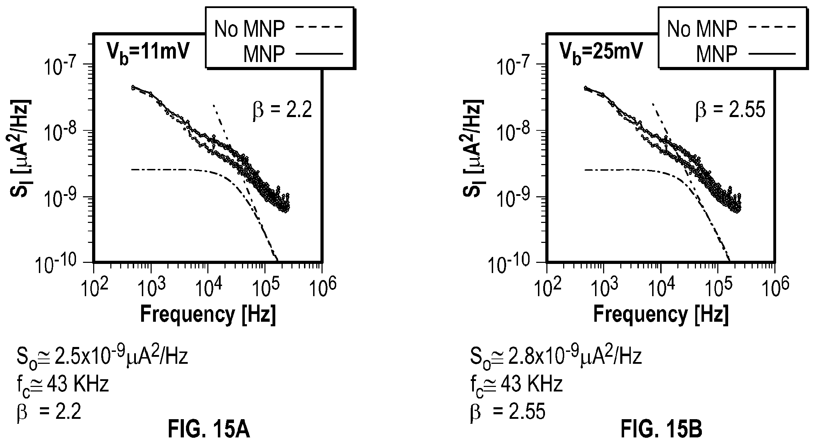

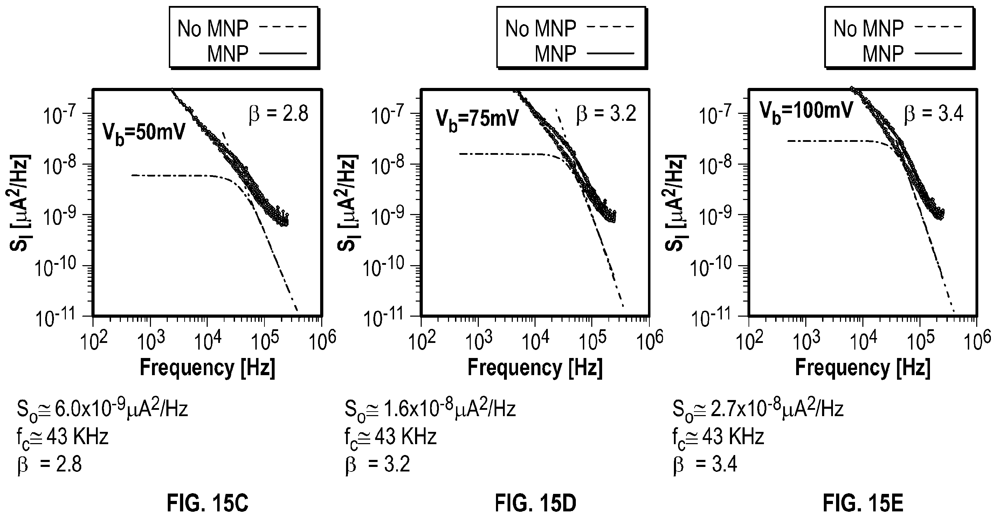

As stated above, the bias voltage applied to a magnetic sensor 105 affects whether and to what extent the characteristic bump 140 in the overall PSD is apparent in the measured sensor signal 207 when a MNP 102 is present. In order to detect the presence and motion of the MNP 102, it is desirable to find a Lorentzian function that can be added to the noise PSD of the magnetic sensor 105 to result in the detected overall PSD. FIGS. 15A, 15B, 15C, 15D, and 15E illustrate the results of experiments conducted to investigate the impact of the bias voltage on this procedure. FIG. 15A shows the results when the bias voltage was 11 mV; FIG. 15B shows the results when the bias voltage was 25 mV; FIG. 15C shows the results when the bias voltage was 50 mV; FIG 15D shows the results when the bias voltage was 75 mV; and FIG. 15E shows the results when the bias voltage was 100 mV.

As a comparison between FIGS. 15A, 15B, 15C, 15D, and 15E indicates, at higher bias voltages, it becomes increasingly difficult to fit a Lorentzian function representing the confined Brownian motion of the MNP 102 to the measured data. The use of higher bias voltages may be triggering the onset of super-diffusion, in which case the motion of the MNP 102 would no longer be confined Brownian motion, but rather driven motion (e.g., the MNP 102 would be influenced by additional forces and would be moving faster than it would be moving in confined Brownian motion). Super-diffusion could result if the magnetic sensor 105 influences (drives) the motion of the MNP 102 rather than merely observing it. The result of higher bias voltages is that the slope of the high-frequency tail of the overall PSD is larger than 2, which is a signature of super-diffusion. It was found in the inventors' experiments that for higher bias voltages, the PSD of the MNP 102 could not be represented by a Lorentzian function, but rather by a function ${S}_{{I}_{MNP}} \left(f\right) = \frac{{S}_{O}}{1 + {\left(\frac{f}{{f}_{c}}\right)}^{β}}$ where *β* is a value greater than 2. The values of *β* for each of the bias voltages in FIGS. 15A, 15B, 15C, 15D, and 15E are shown in the figures. In other words, the experimental results presented in FIGS. 15A, 15B, 15C, 15D, and 15E indicate that the system becomes nonlinear and unpredictable when the bias voltage is too large.

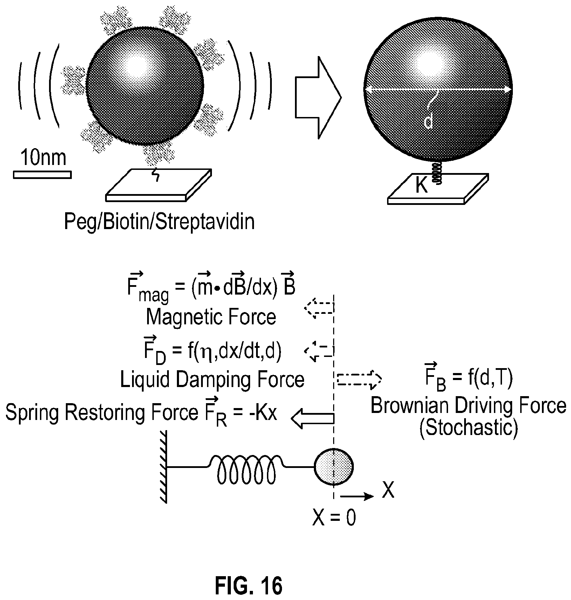

To adjust the mathematical model to account for super-diffusion, the one-dimensional harmonic potential approximation derived above can be modified to include a component representing the magnetic force caused by the magnetic sensor 105 bias voltage. FIG. 16 illustrates how the model can be modified to include a component due to the magnetic sensor 105 influencing the motion of the MNP 102. Once again, the MNP 102 is considered to be a mass on a spring, which is the tether (e.g., biopolymer 101). The Brownian driving force, the liquid damping force, and the spring restoring force are the same as shown in FIG. 10A and described in the discussion of that figure above. In addition to those forces, the model of FIG. 16 adds a magnetic force caused by the magnetic sensor 105, represented as ${\vec{F}}_{mag} = \left(\vec{m}⋅\frac{d \vec{B}}{dx}\right) \vec{B}$ where *̅m̅*̅ is the magnetic moment of the MNP 102, and *̅B̅*̅ is the magnetic field at the position of the MNP 102. The one-dimensional time evolution of the distribution probability P of a diffusing spherical particle at position *x* and time *t,* given its initial position *x*₀ at time *t*₀ in a harmonic potential field in a magnetic field gradient is given by the equation of motion: $\frac{\partial }{\partial t} P \left(x,\left.t\right|{x}_{0},{t}_{0}\right) = \frac{1}{3 πηd} \left({k}_{B}T\frac{{\partial }^{2}}{\partial {x}^{2}}+K\frac{\partial }{\partial x}x+m\frac{\partial }{\partial x}\left[\left(\frac{\partial }{\partial x}B\left(x\right)\right)B\left(x\right)\right]\right) P \left(x,\left.t\right|{x}_{0},{t}_{0}\right)$ This equation has no known analytical solution. Thus, the relationship of the hydrodynamic radius to the corner frequency is not known under these circumstances.

To avoid the onset of super-diffusion and allow the MNP 102 to move in confined Brownian motion without the magnetic sensor 105 substantially affecting its motion, the bias voltage of the magnetic sensor 105 should be kept low enough that the characteristic bump 140 caused by the presence of the MNP 102 is present in the overall PSD, and can be fit with a Lorentzian function representing the confined Brownian motion of the MNP 102 as described above. Stated another way, if it is not possible to fit measured PSD data with a Lorentzian function, the bias voltage used to drive the magnetic sensor 105 may be too high and may need to be reduced.

Although the discussion above focused mainly on MTJ sensors, with some explanation of SV sensors, it is to be understood that the magnetic sensor 105 can be any kind of magnetic sensor. The use of MTJs in experiments and as examples is not intended to be limiting. Suitable magnetic sensors 105 include, but are not limited to, giant magnetoresistive (GMR) sensors, Hall effect devices, spin valves, and spin accumulation sensors. In general, the magnetic sensor 105 can be any magnetic sensor that can allow the presence/absence and/or motion of the MNP 102 to be detected from the sensor signal 207.

### Additional Working Examples

To demonstrate the feasibility and implementation of the dynamic spectral biosensing techniques described herein, conformational changes of an exemplary biopolymer 101, ssDNA, induced by changing the ionic strength of the buffer, have been monitored using magnetic sensors 105 situated in a flow-cell.

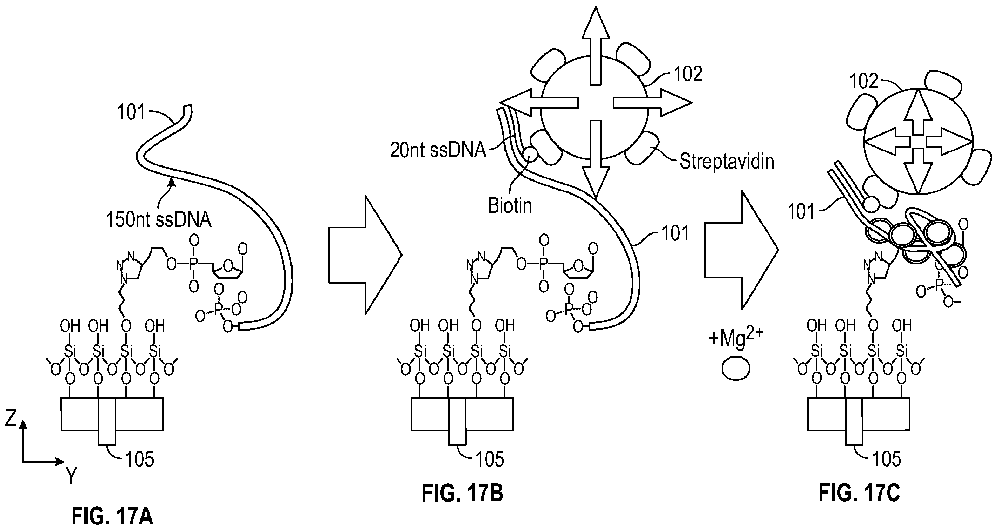

The three stages of experiments conducted are shown schematically in FIGS. 17A, 17B, and 17C. First, as illustrated in FIG. 17A, the 5'-end of 150 nucleotide (nt) ssDNA was first attached to the surface 117 of a device in the sensing region 206 of a magnetic sensor 105 using copper-catalyzed azide-alkyne click chemistry. 3'-end biotinylated 20-mer was then hybridized to the 3'-end of the ssDNA. Thus, FIG. 17A illustrates the exemplary 150 nt ssDNA bound to a surface 117 near a magnetic sensor 105 before the MNP 102 has been attached. The ssDNA is bound to the surface 117 in the vicinity of the magnetic sensor 105 so that the magnetic sensor 105 can detect a MNP 102 bound to the other end of the ssDNA. In the experiments, a uniform 1.5 mT (15 Oersted) external magnetic field was then applied in the direction perpendicular to the exposed surface of the magnetic sensor 105 (along the z-axis in FIG. 17A, both in the positive and the negative direction), and the sensor signal 207 was recorded without any MNP 102 present.

Next, a streptavidin-coated 20 nm MNP 102 was attached to the end of the ssDNA tether (biopolymer 101). FIG. 17B illustrates the ssDNA tether with a streptavidin-coated 20-nm MNP 102 attached. As shown in FIG. 17B, the tethered 20-nm MNP 102 is in the proximity of the magnetic sensor 105 (e.g., generally within its sensing region 206). The MNP 102 is coated in streptavidin to allow it to bind tightly to the ssDNA tether. The arrows overlaying the MNP 102 represent the extent of the stochastic motion of the MNP 102. The sensor signal 207 was recorded in 10 mM Tris buffer.

Addition of, for example, Mg²⁺ ions causes compaction of ssDNA. Thus, the confined stochastic motion of a MNP 102 attached to a ssDNA should become attenuated upon addition of Mg²⁺ ions. (Similar behavior has been observed by TPM on poly-uridine (U) messenger (m)RNA.) Therefore, in the tests, magnesium ions were added to the solution. FIG. 17C illustrates an exemplary state after addition of magnesium ions and subsequent compaction of the ssDNA tether. Relative to FIG. 17B, the stochastic motion of the MNP 102 is attenuated as represented by the shorter arrows overlaying the MNP 102. The sensor signal 207 was recorded in 15 mM Tris-MgCl₂ buffer.

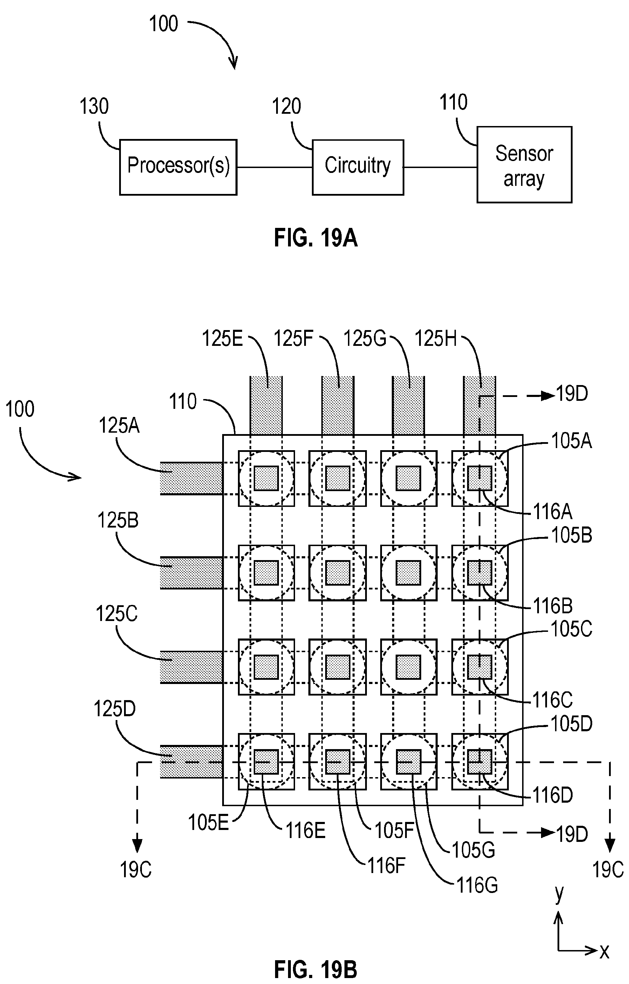

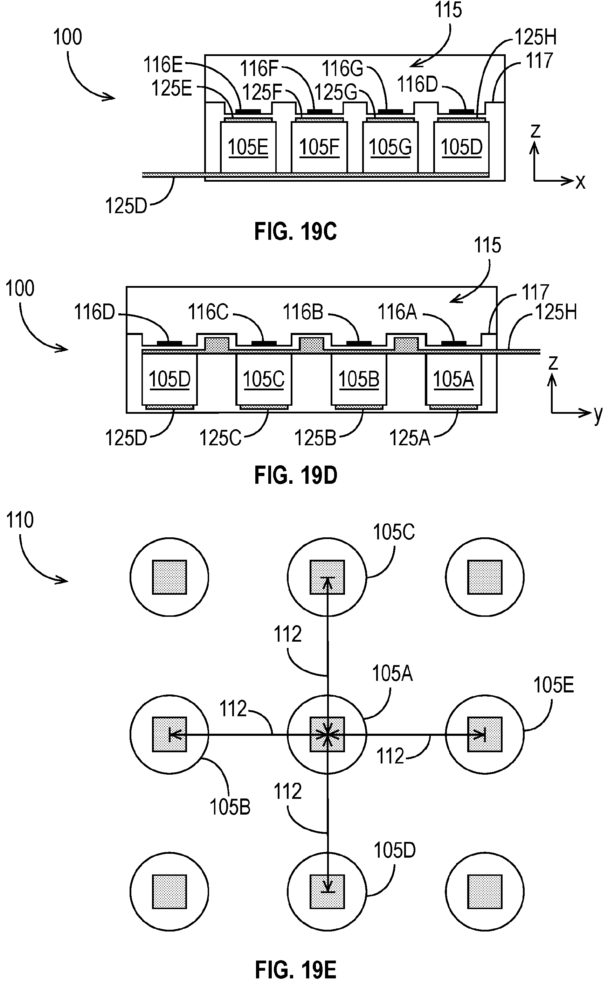

Although the discussion above of FIGS. 17A, 17B, and 17C describes only one MNP 102 and only one magnetic sensor 105, the tests used an array of magnetic sensors 105, a plurality of MNPs 102, and a plurality of ssDNA fragments (biopolymers 101). In the tests, the density of ssDNA immobilized on the surface of the flow cell was not controlled, and it is possible that a particular observed MNP 102 was attached to the surface with more than one DNA strand. (A single-molecule system to mitigate or eliminate this possibility is described below in the context of, for example, FIGS. 19A, 19B, 19C, 19D, and 19E.) Accordingly, the density of attached MNPs 102 was adjusted to ensure that there would be magnetic sensors 105 with a single or only a few MNPs 102 tethered in the proximity of the magnetic sensor 105 so as to ensure the presence of only one MNP 102 within the sensing region 206. Several such magnetic sensors 105 were identified, and the recorded sensor signals 207 of those magnetic sensors 105 were sampled at a moderate sampling rate of 6 kHz. The recorded sensor signal 207 and the corresponding autocorrelation functions for two such representative magnetic sensors 105 are presented in FIGS. 18A, 18B, and 18C.

FIG. 18A illustrates exemplary recorded current fluctuations (e.g., sensor signal 207) of two different exemplary magnetic sensors 105, denoted as "Sensor 1" and "Sensor 2," over a period of two seconds in the presence of an applied external magnetic field H after attachment (immobilization) of 150 nt ssDNA (e.g., each a biopolymer 101) to the surface 117 near each of two magnetic sensor 105, but before the attachment of any MNP 102. The state is illustrated by the uppermost (non-plot) portion of FIG. 18A. In other words, the recorded current fluctuations for each of the two magnetic sensors 105, shown by the plots of intensity versus time, are the background or baseline sensor signals 207 of the two magnetic sensors 105, Sensor 1 and Sensor 2, for the stage depicted in FIG. 17A. The positive and negative autocorrelation functions of the measured sensor signals 207 are also shown in FIG. 18A for each of Sensor 1 and Sensor 2. The smooth, short-dash curve in each of the autocorrelation plots is average autocorrelation of the respective baseline measured sensor signal 207.

FIG. 18B illustrates the measured sensor signals 207 (intensity v. time) of Sensor 1 and Sensor 2 and their autocorrelation functions after attachment of the MNP 102, which, in the tests, was a respective 20 nm Fe₃O₄ particle tethered to the end of each of the DNA strands, and after adding Tris buffer. FIG. 18B provides the results when the ssDNA is in its elongated conformation and corresponds to the stage depicted in FIG. 17B. The stage is illustrated by the uppermost (non-plot) portion of FIG. 18B. The introduction of the MNP 102 causes both the recorded current fluctuations in the respective sensor signals 207 and the autocorrelation functions to change relative to FIG. 18A. For example, as a comparison between FIGS. 18A and 18B indicates, the positive and negative autocorrelation functions of Sensor 1 shift upward relative to the baseline sensor signal 207 for lag times between approximately 1 ms and 200-300 ms, whereas the autocorrelation functions of Sensor 2 generally shift downward relative to the baseline sensor signal 207 for lag times between about 1 ms and around 50 ms. Thus, the presence of a MNP 102 within the sensing region 206 can be inferred from the shifts in the autocorrelation functions relative to the baseline of FIG. 18A (when no MNPs 102 are present).

FIG. 18C illustrates the measured sensor signals 207 (intensity v. time) of Sensor 1 and Sensor 2 and their autocorrelation functions when the DNA tether (e.g., biopolymer 101) is compacted by the introduction of Mg²⁺ ions. In other words, FIG. 18C corresponds to the stage depicted in FIG. 17C. The stage is illustrated by the uppermost (non-plot) portion of FIG. 18C. A comparison of the autocorrelation functions of FIG. 18B to those of FIG. 18C and/or FIG. 18A reveals that the changes in conformation are detectable in the autocorrelation functions. For example, relative to the autocorrelation functions shown in FIG. 18B for Sensor 1, the positive and negative autocorrelation functions shift slightly downward for lag times between 1 ms and around 60-70 ms after addition of the Mg²⁺ ions and they also hew closer to the average autocorrelation function for lag times above about 300 ms. Similarly, relative to the autocorrelation functions shown in FIG. 18B for Sensor 2, the change in conformation of the ssDNA caused by the addition of Mg²⁺ ions manifests as a shift downward of the positive and negative autocorrelation functions for lag times between about 1 ms and about 50 ms, and a shift upward for lag times around 200-300 ms. Thus, as illustrated by FIGS. 18A, 18B, and 18C, significant changes in the noise autocorrelation functions are observed between the three states, thereby allowing both the presence/absence and motion of the MNPs 102 within the sensing regions 206 of Sensor 1 and Sensor 2 to be detected and/or monitored.

The results described and shown in FIGS. 18A, 18B, and 18C confirm that magnetic sensors 105 can detect not only the changes of the MNP 102 average equilibrium position, but they can also monitor small reversible variations in noise fluctuations induced by single-molecule processes. Billions of such magnetic sensors 105 with single-molecule sensitivity can potentially be integrated on CMOS platforms (e.g., similar to Toshiba's 4-Gbit density STT-MRAM chip) to create the next generation of high-throughput systems for diagnostics and drug-discovery while utilizing existing mature technologies and high-volume manufacturing capabilities developed by the semiconductor and data storage industries.

The coupling between the pinned and free layers of certain tested magnetic sensors 105 is appropriate for biosensing, as the experiments described herein indicate. These magnetic sensors 105 are one example of suitable magnetic sensors 105. Other magnetic sensors 105 having coupling between FM1 and FM2 that is optimized for biosensing applications or for a particular type of MNP 102 can also be used and may perform better than the exemplary magnetic recording sensors used in the experiments.

### Monitoring Devices and Systems

As described further below, in some embodiments, a system 100 for monitoring motion of a MNP 102 coupled to a biopolymer 101 can comprise a fluid chamber 115, at least one processor 130, and a magnetic sensor 105. The fluid chamber includes a binding site 116 that is configured to affix an end of the biopolymer 101 to a surface of the fluid chamber 115 and to allow the MNP 102 to move (e.g., as it is bombarded by the molecules of a surrounding fluid). The binding site 116 may include a structure (e.g., a cavity or ridge) configured to anchor the biopolymer 101 to the binding site 116.

The magnetic sensor 105 may comprise, for example, a MTJ or a STO. The magnetic sensor 105 has a sensing region 206 within the fluid chamber 115, in which it can detect the MNP 102. The sensing region 206 may have a volume, for example, between about 10⁵ nm³ and about 5 × 10⁵ nm³. The sensing region 206 includes the binding site 116. The magnetic sensor 105 is configured to generate a sensor signal 207 characterizing the magnetic environment (e.g., presence, absence, and/or position of the MNP 102) within the sensing region 206 and to provide the sensor signal 207 to the at least one processor 130. The sensor signal 207 may convey (e.g., report) one or more of a current, a voltage, a resistance, a noise (e.g., a frequency noise or phase noise), a frequency or change in frequency (e.g., an oscillation frequency or a Lorentzian corner frequency), etc.

In some embodiments, the at least one processor 130 is configured to execute machine-executable instructions that allow it to (a) obtain a first portion of the sensor signal 207 representing the magnetic environment within the sensing region 206 during a first detection period, (b) obtain a second portion of the sensor signal 207 representing the magnetic environment within the sensing region 206 during a second detection period that is after the first detection period, and (c) analyze the first and second portions of the sensor signal 207 to detect motion of the tethered MNP 102. For example, as described further below, the at least one processor 130 may determine a first autocorrelation function of the first portion of the signal, determine a second autocorrelation function of the second portion of the signal, and analyze the first autocorrelation function and the second autocorrelation function (e.g., compare the first and second autocorrelation functions) to detect motion of the tethered MNP 102. The at least one processor 130 may process the sensor signal 207, or portions of it, in the time domain, frequency domain, or both. In some embodiments, the at least one processor 130 is configured to determine a Lorentzian function characterizing the confined Brownian motion of a MNP 102.

The system 100 may further include detection circuitry 120 coupled to the magnetic sensor 105 and to the at least one processor 130. The circuitry 120 may include, for example, one or more lines that allow the at least one processor 130 to read or interrogate the magnetic sensor 105. The circuitry 120 may include components such as an analog-to-digital converter and/or an amplifier.

In some embodiments, a monitoring system 100 comprises a plurality of magnetic sensors 105 that, in use, are each functionalized with individual, single biomolecules such that the monitoring system 100 is capable of detecting single-molecule processes at each magnetic sensor 105. FIG. 19A is a block diagram showing components of an exemplary monitoring system 100 in accordance with some embodiments. As illustrated, the exemplary monitoring system 100 includes a sensor array 110, which is coupled to circuitry 120, which is coupled to at least one processor 130. The sensor array 110 comprises a plurality of magnetic sensors 105 that may be arranged in any suitable way, as described further below. (It is to be understood that the sensor array 110 includes at least one magnetic sensor 105.)

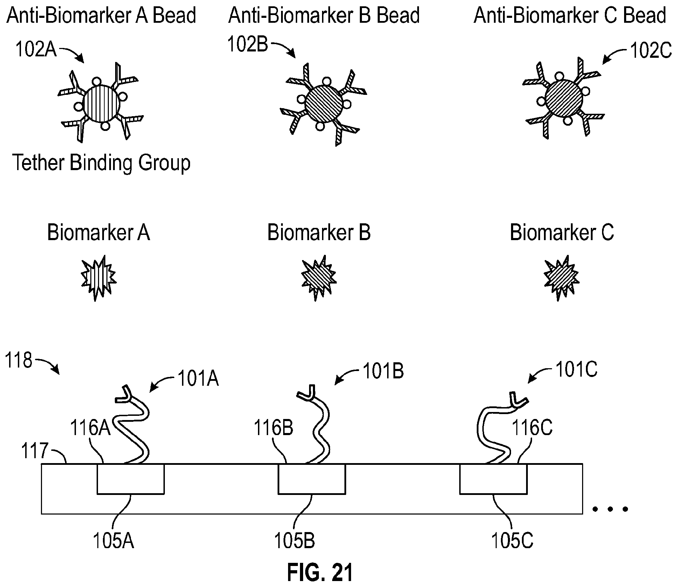

The circuitry 120 can include, for example, one or more lines that allow magnetic sensors 105 in the sensor array 110 to be interrogated by the at least one processor 130 (e.g., with the assistance of other components that are well known in the art, such as a current or voltage source, amplifier, analog-to-digital converter, etc.). For example, in operation, the processor(s) 130 can cause the circuitry 120 to apply a bias voltage or current to such lines to detect a sensor signal 207 that reports the magnetic environment of at least one magnetic sensor 105 in the sensor array 110. The sensor signal 207 indicates the presence, absence, position, and/or movement of a MNP 102 within the sensing region 206. In other words, the sensor signal 207 indicates some characteristic (e.g., magnetic field, resistance, voltage, current, oscillation frequency, signal level, noise level, frequency noise, phase noise, etc.) of the magnetic sensor 105. The sensor signal 207 can be inspected and/or processed to determine whether the magnetic sensor 105 has detected a MNP 102 or motion (e.g., changes in position) of a MNP 102 as time passes. For example, the at least one processor 130 may monitor one or more time-domain, frequency-domain, deterministic, and/or statistical properties (e.g., peak or average amplitude, fluctuations, excursions from a mean or expected peak, autocorrelation, power spectral density, etc.) of the sensor signal 207 and determine that a MNP 102 or movement of a MNP 102 was (or was not) detected. As a specific example, the at least one processor 130 may compare a form (e.g., autocorrelation, PSD, etc.) of the sensor signal 207 of a magnetic sensor 105 at a selected time or over a selected time period to a form of the sensor signal 207 at an earlier time or over an earlier or different period of time (e.g., a baseline autocorrelation, as described above in the discussion of FIGS. 17A, 17B, and 17C or a baseline noise PSD as described below in the discussion of, e.g., FIGS. 21-26) and base the determination of whether a MNP 102 was or was not detected, or has or has not moved, on changes in the sensor signal 207. For example, the at least one processor 130 may determine a first overall noise PSD of the sensor signal 207 during a first detection period and a second overall noise PSD of the sensor signal 207 during a second detection period, and analyze whether a MNP 102 is present and/or has moved. In some embodiments, the at least one processor 130 determines a Lorentzian function that, when added to a baseline noise PSD of the magnetic sensor 105, results in the overall noise PSD of the sensor signal 207 during one or both of the first and second detection periods.

The sensor signal 207 and the information it conveys to characterize the magnetic environment of the magnetic sensor 105 may depend on the type of magnetic sensor 105 used in the monitoring system 100. In some embodiments, the magnetic sensors 105 are magnetoresistive (MR) sensors (e.g., MTJs, SVs, etc.) that can detect, for example, a magnetic field or a resistance, a change in magnetic field or a change in resistance, or a noise level. In some embodiments, each of the magnetic sensors 105 of the sensor array 110 is a thin film device that is capable of using the MR effect to detect a MNP 102 attached to a biopolymer 101 bound to a respective binding site 116 associated with the magnetic sensor 105. The magnetic sensor 105 may operate as a potentiometer with a resistance that varies as the strength and/or direction of the sensed magnetic field changes. In some embodiments, the magnetic sensor 105 comprises a magnetic oscillator (e.g., STO), and the sensor signal 207 reports a frequency generated by the magnetic oscillator, or a change in frequency, frequency noise, or phase noise.

In some embodiments, the at least one processor 130, with help from the circuitry 120, detects deviations or fluctuations in the magnetic environment of some or all of the magnetic sensors 105 in the sensor array 110. For example, a magnetic sensor 105 of the MR type in the absence of a MNP 102 should have relatively small noise above a certain frequency as compared to a magnetic sensor 105 in the presence of a MNP 102, because the field fluctuations from the MNP 102 will cause fluctuations of the moment of the sensing ferromagnet. These fluctuations can be measured, for example, using heterodyne detection (e.g., by measuring noise power density) or by directly measuring the current or voltage of the magnetic sensor 105 and evaluated using a comparator circuit to compare to another sensor element that does not sense the binding site 116. In some embodiments, the magnetic sensors 105 include STO elements, and fluctuating magnetic fields from MNPs 102 cause jumps in phase for the magnetic sensors 105 due to instantaneous changes in frequency, which can be detected using a phase detection circuit.

It is to be understood that the examples of MNPs 102 and magnetic sensors 105 provided herein are merely exemplary. In general, any type of MNP 102 that can be attached to biopolymers 101 may be used along with an array 110 of any type of magnetic sensor 105 that can detect that type of MNP 102.

It is also to be understood that the components of the monitoring system 100 may be distributed, or they may be included in a single physical device. For example, if the at least one processor 130 includes more than one processor, a first processor may be part of a device (e.g., a chip) that includes the sensor array 110 of at least one magnetic sensor 105, and a second processor may be in a different physical location (e.g., off-chip in an attached computer). As a specific example, a first processor within the monitoring system 100 can be configured to retrieve the sensor signal 207 from a magnetic sensor 105, and a second processor within the monitoring system 100, not necessarily part of the same physical apparatus as the first processor, can process the sensor signal 207 (e.g., compute autocorrelation functions, PSDs, Lorentzian functions, etc., and/or perform signal processing and/or analysis, etc.) to detect the presence/absence and/or motion of the MNP 102. Accordingly, the components illustrated in FIG. 19A can be co-located or distributed. Stated a different way, a system may comprise the components illustrated in FIG. 19A in a single physical device, or the components of FIG. 19A can be distributed. Likewise, the monitoring system 100 can include other components, such as, for example, memory to store the sensor signal 207 or a sampled or processed version of the sensor signal 207, or instructions for execution by the at least one processor 130, among other things.

FIGS. 19B, 19C, and 19D illustrate portions of an exemplary monitoring system 100 for detection and monitoring of single-molecule processes in accordance with some embodiments. FIG. 19B is a top view of the portion of the monitoring system 100. FIG. 19C is a cross-section view at the position indicated by the long-dash line labeled "19C" in FIG. 19B, and FIG. 19D is a cross-section view at the position indicated by the long-dash line labeled "19D" in FIG. 19B.

The exemplary portion of the monitoring system 100 shown in FIGS. 19B, 19C, and 19D comprises a sensor array 110 for sensing MNPs 102 within a fluid chamber 115 of the monitoring system 100. The sensor array 110 includes a plurality of magnetic sensors 105, with sixteen magnetic sensors 105 shown in the array 110 of FIG. 19B. It is to be appreciated that an implementation of a monitoring system 100 may include any number of magnetic sensors 105 (e.g., as few as one, or hundreds, thousands, millions, or even billions of magnetic sensors 105). To avoid obscuring the drawing, only seven of the magnetic sensors 105 are labeled in FIG. 19B, namely the magnetic sensors 105A, 105B, 105C, 105D, 105E, 105F, and 105G. (For simplicity, this document refers generally to the magnetic sensor 105 by the reference number 105. Individual magnetic sensors 105 are given the reference number 105 followed by a letter.) As explained above, the magnetic sensors 105 can detect the presence or absence of MNPs 102 and movement of the MNPs 102 within their respective sensing regions 206. In other words, each of the magnetic sensors 105 can detect whether there is a MNP 102 in its vicinity (e.g., in the sensing region 206), and the sensor signal 207 provided by the magnetic sensor 105 also provides an indication of whether and how the MNP 102 is moving.

Referring now to FIGS. 19C and 19D in conjunction with FIG. 19B, each magnetic sensor 105 is illustrated in the exemplary embodiment of the monitoring system 100 as having a cylindrical shape. It is to be understood, however, that in general the magnetic sensors 105 can have any suitable shape. For example, the magnetic sensor 105 may be cuboid in three dimensions. Moreover, different magnetic sensors 105 can have different shapes (e.g., some may be cuboid and others cylindrical, etc.). It is to be appreciated that the drawings are merely exemplary.

As shown in FIGS. 19C and 19D, the monitoring system 100 includes a fluid chamber 115. The fluid chamber 115 comprises a plurality of binding sites 116 on the surface 117. The fluid chamber 115 holds fluids (e.g., buffers, nucleotide precursors, other fluids or solutions). In the illustrated embodiment, each magnetic sensor 105 is associated with a respective binding site 116. (For simplicity, this document refers generally to the binding sites by the reference number 116. Individual binding sites are given the reference number 116 followed by a letter.) In other words, the magnetic sensors 105 and the binding sites 116 are in a one-to-one relationship. As shown in FIG. 19B, the magnetic sensor 105A is associated with the binding site 116A, the magnetic sensor 105B is associated with the binding site 116B, the magnetic sensor 105C is associated with the binding site 116C, the magnetic sensor 105D is associated with the binding site 116D, the magnetic sensor 105E is associated with the binding site 116E, the magnetic sensor 105F is associated with the binding site 116F, and the magnetic sensor 105G is associated with the binding site 116G. Each of the other, unlabeled magnetic sensors 105 shown in FIG. 19B is also associated with a respective binding site 116. In the example embodiment of FIGS. 19B, 19C, and 19D, each magnetic sensor 105 is shown disposed below its respective binding site 116, but it is to be appreciated that the binding sites 116 may be in other locations relative to their respective magnetic sensors 105. For example, the binding sites 116 may be to the sides of their respective magnetic sensors 105.

Each of the binding sites 116 is configured to bind no more than one biopolymer 101 (e.g., ssDNA, RNA, protein, etc.) to the surface 117 within the fluid chamber 115. In other words, each binding site 116 has characteristics and/or features intended to allow one, and only one, biopolymer 101 to be bound to it for sensing and monitoring by a respective magnetic sensor 105 (or multiple magnetic sensors 105, as discussed below), thereby making the system 100 a single-molecule system. The respective magnetic sensor 105 can thereafter detect and monitor movement of a MNP 102 attached to the biopolymer 101 bound to the binding site 116. In some embodiments, the binding site 116 has a structure (or multiple structures) configured to anchor the biopolymer 101 to the binding site 116. For example, the structure (or structures) may include a cavity or a ridge. FIGS. 19C and 19D illustrate the binding sites 116 as extending from the surface 117 of the fluid chamber 115, but it is to be recognized that the binding sites 116 may be flush with or etched into the surface 117 of the fluid chamber 115.

The binding sites 116 can have any suitable size and shape that facilitates the attachment of one, and only one, biopolymer 101 to each binding site 116. For example, the shapes of the binding sites 116 can be similar or identical to the shapes of the magnetic sensors 105 (e.g., if the magnetic sensors 105 are cylindrical in three dimensions, the binding sites 116 can also be cylindrical, either protruding from the surface 117 of the fluid chamber 115 or forming a fluid container within the surface 117 of the fluid chamber 115, with a radius that can be larger, smaller, or the same size as the radius of the respective magnetic sensor 105; if the magnetic sensors 105 are cuboid in three dimensions, the binding sites 116 can also be cuboid and larger, smaller, or the same size as the closest part of the magnetic sensors 105, etc.). In general, the binding sites 116 and the surface 117 of the fluid chamber 115 can have any shapes and characteristics that facilitate the attachment of a single biopolymer 101 to each binding site 116 and allow the magnetic sensors 105 to detect the presence and motion of MNPs 102 attached to biopolymers 101 bound to their respective binding sites 116.

FIGS. 19C and 19D illustrate an enclosed fluid chamber 115 with a top portion that extends in the x-y plane, but there is no requirement for the fluid chamber 115 to be enclosed. In some embodiments, the surface 117 of the fluid chamber 115 has properties and characteristics that protect the sensors 105 from whatever fluids are in the fluid chamber 115, while still allowing the biopolymers 101 to bind to the binding sites 116 and the magnetic sensors 105 to detect MNPs 102 that are attached to the biopolymers 101 attached to the binding sites 116. The material of the fluid chamber 115 (and possibly of the binding sites 116) may be or comprise an insulator. In some embodiments, the surface 117 of the fluid chamber 115 comprises an organic polymer, a metal, or a silicate. The surface 117 of the fluid chamber 115 may include, for example, a metal oxide, silicon dioxide, polypropylene, gold, glass, or silicon. The thickness of the surface 117 of the fluid chamber 115 may be selected so that the magnetic sensors 105 can detect MNPs 102 attached to biopolymers 101 bound to the binding sites 116 within the fluid chamber 115. In some embodiments, the surface 117 is approximately 3 to 20 nm thick so that each magnetic sensor 105 is between approximately 5 nm and approximately 50 nm from any MNP 102 attached to the biopolymer 101 bound to the respective binding site 116. It is to be understood that these values are merely exemplary. It will be appreciated that an implementation may have a fluid chamber 115 with a thicker or thinner surface 117, and, as explained above, the sensing region 206 can be of any suitable size.

The circuitry 120 of the monitoring system 100 may include, or be attached to the sensor array 110 by, one or more lines 125. In some embodiments, each magnetic sensor 105 is coupled to at least one line 125. In the example shown in FIGS. 19B, 19C, and 19D, the monitoring system 100 includes eight lines 125A, 125B, 125C, 125D, 125E, 125F, 125G, and 125H. (For simplicity, this document refers generally to the lines by the reference number 125. Individual lines are given the reference number 125 followed by a letter.) In the exemplary embodiment of FIGS. 19B, 19C, and 19D, pairs of lines 125 can be used to access (e.g., read or interrogate) individual magnetic sensors 105. In the exemplary embodiment shown in FIGS. 19B, 19C, and 19D, each magnetic sensor 105 of the sensor array 110 is coupled to two lines 125. For example, the magnetic sensor 105A is coupled to the lines 125A and 125H; the magnetic sensor 105B is coupled to the lines 125B and 125H; the magnetic sensor 105C is coupled to the lines 125C and 125H; the magnetic sensor 105D is coupled to the lines 125D and 125H; the magnetic sensor 105E is coupled to the lines 125D and 125E; the magnetic sensor 105F is coupled to the lines 125D and 125F; and the magnetic sensor 105G is coupled to the lines 125D and 125G. In the exemplary embodiment of FIGS. 19B, 19C, and 19D, the lines 125A, 125B, 125C, and 125D are shown residing under the magnetic sensors 105, and the lines 125E, 125F, 125G, and 125H are shown residing above the magnetic sensors 105. FIG. 19C shows the magnetic sensor 105E in relation to the lines 125D and 125E, the magnetic sensor 105F in relation to the lines 125D and 125F, the magnetic sensor 105G in relation to the lines 125D and 125G, and the magnetic sensor 105D in relation to the lines 125D and 125H. FIG. 19D shows the magnetic sensor 105D in relation to the lines 125D and 125H, the magnetic sensor 105C in relation to the lines 125C and 125H, the magnetic sensor 105B in relation to the lines 125B and 125H, and the magnetic sensor 105A in relation to the lines 125A and 125H.

The magnetic sensors 105 of the exemplary monitoring system 100 shown in FIGS. 19B, 19C, and 19D are arranged in a sensor array 110 that has a rectangular pattern. (It is to be appreciated that a square pattern is a special case of a rectangular pattern.) Each of the lines 125 identifies a row or a column of the sensor array 110. For example, each of the lines 125A, 125B, 125C, and 125D identifies a different row of the sensor array 110, and each of the lines 125E, 125F, 125G, and 125H identifies a different column of the sensor array 110. As shown in FIG. 19C, each of the lines 125E, 125F, 125G, and 125H is in contact with one of the magnetic sensors 105 along the cross-section (namely, line 125E is in contact with the top of magnetic sensor 105E, line 125F is in contact with the top of magnetic sensor 105F, line 125G is in contact with the top of magnetic sensor 105G, and line 125H is in contact with the top of magnetic sensor 105D), and the line 125D is in contact with the bottom of each of the sensors 105E, 105F, 105G, and 105D. Similarly, and as shown in FIG. 19D, each of the lines 125A, 125B, 125C, and 125D is in contact with the bottom of one of the sensors 105 along the cross-section (namely, line 125A is in contact with the bottom of magnetic sensor 105A, line 125B is in contact with the bottom of magnetic sensor 105B, line 125C is in contact with the bottom of magnetic sensor 105C, and line 125D is in contact with the bottom of magnetic sensor 105D), and the line 125H is in contact with the top of each of the magnetic sensors 105D, 105C, 105B, and 105A.

The magnetic sensors 105 and portions of the lines 125 connecting to the sensor array 110 are illustrated in FIG. 19B using dashed lines to indicate that they may be embedded within the monitoring system 100. As explained above, the magnetic sensors 105 may be protected (e.g., by an insulator) from the contents of the fluid chamber 115, which itself might be enclosed. Accordingly, it is to be understood that the various illustrated components (e.g., lines 125, magnetic sensors 105, binding sites 116, etc.) are not necessarily visible in a physical instantiation of the monitoring system 100 (e.g., they may be embedded in or covered by protective material, such as an insulator).

In some embodiments, some or all of the binding sites 116 reside in nanowells or trenches in lines 125 passing over the magnetic sensors 105. For example, as shown in the example of FIG. 19D, the line 125H may be thinner over the magnetic sensors 105 than it is between the magnetic sensors 105. For example, the line 125H has a first thickness above the magnetic sensor 105D, a second, larger thickness between the magnetic sensors 105D and 105C, and the first thickness above the magnetic sensor 105C. Such a configuration may be advantageously fabricated using conventional thin-film fabrication methods (e.g., by depositing material, applying a mask to the deposited material, and removing (e.g., by etching) some of the deposited material in accordance with the mask). Both the binding sites 116 and, if present, nanowells may be fabricated using conventional techniques.

To simplify the explanation, FIGS. 19B, 19C, and 19D illustrate an exemplary monitoring system 100 with only sixteen magnetic sensors 105 in the sensor array 110, only sixteen corresponding binding sites 116, and eight lines 125. It is to be appreciated that the monitoring system 100 may have fewer or more magnetic sensors 105 in the sensor array 110, and, accordingly, it may have more or fewer binding sites 116. Similarly, embodiments that include lines 125 may have more or fewer lines 125. In general, any configuration of magnetic sensors 105, binding sites 116, and circuitry 120 (e.g., including lines 125) that allows the magnetic sensors 105 to detect MNPs 102 attached to biopolymers 101 bound to the binding sites 116 may be used. Similarly, any configuration of one or more lines 125 or some other mechanism that allows the sensor signals 207 to be retrieved from the magnetic sensors 105 may be used. The examples presented herein are not intended to be limiting.

The magnetic sensors 105 shown in FIGS. 19B, 19C, and 19D are in close proximity to the binding sites 116 and, therefore, they are also in close proximity to the biopolymers 101 and MNPs 102 that are bound to the binding sites 116.

Although FIGS. 19B, 19C, and 19D illustrate magnetic sensors 105 and binding sites 116 in a one-to-one relationship, it is to be appreciated that each binding site 116 can be sensed by more than one magnetic sensor 105. For example, if a monitoring system 100 has more magnetic sensors 105 than binding sites 116, it may be possible for at least some MNPs 102 to be sensed by multiple magnetic sensors 105 (e.g., to improve the accuracy of detection of the MNPs 102 and the motion thereof). Such an approach can improve the SNR by providing diversity of observations.

The exemplary sensor array 110 shown and described in the context of FIGS. 19B, 19C, and 19D is a rectangular array, with the magnetic sensors 105 arranged in rows and columns. In other words, the plurality of magnetic sensors 105 of the sensor array 110 is arranged in a rectangular grid pattern. In some embodiments, adjacent rows and columns of the rectangular grid pattern are equidistant from each other, which results in the magnetic sensors 105 being arranged in a square grid (or lattice) pattern as illustrated in FIG. 19E. In embodiments in which the magnetic sensors 105 are arranged in a square grid pattern, each magnetic sensor 105 has up to four nearest neighbors. For example, as shown in FIG. 19E, the magnetic sensor 105A has the four nearest neighbors labeled as 105B, 105C, 105D, and 105E. The closest sensors 105 are a nearest-neighbor distance 112 away, as shown in FIG. 19E. Thus, each of the sensors 105B, 105C, 105D, and 105E is a nearest-neighbor distance 112 away from the magnetic sensor 105A.

In accordance with some embodiments, an example monitoring system 100 may use high-precision nanoscale fabrication of densely-packed nanoscale magnetic sensors 105 capable of detecting individual MNPs 102, as described above in the discussion of FIGS. 18A, 18B, and 18C. The sizes of the functionalized binding sites 116 can be similar to the size of, for example, the biopolymer 101 with a MNP 102 attached so that multiple biopolymers 101 cannot bind to the same binding site 116 or be sensed by the same magnetic sensor 105 (e.g., so that each magnetic sensor 105 detects/senses only one MNP 102). The appropriate value of the nearest-neighbor distance 112, which may then be used to determine the size of the sensor array 110 and/or the maximum number of magnetic sensors 105 that can fit within a sensor array 110 of a selected size, can be determined based on the properties of the magnetic sensor 105 (e.g., sensitivity, size, etc.), the properties (e.g., lengths, softness, etc.) of biopolymers 101 the monitoring system 100 is intended to monitor, and the properties (e.g., size, type, etc.) of the MNP(s) 102 being used. For example, the combined length of the biopolymer 101 and the size of the MNP 102 to be used can provide a physical limitation on how closely two magnetic sensors 105 in a sensor array 110 can be positioned. In some embodiments, the size of the magnetic sensors 105 may be limited by the nanoscale patterning capabilities of a process used to manufacture the sensor array 110. For example, using technology available at the time of writing, the size of each magnetic sensor 105 (e.g., assuming cylindrical sensors 105, the diameter of the sensors 105 in the x-y plane) may be around 20 nm. Assuming the type of biopolymer 101 to be monitored is ssDNA, and it is desirable to monitor fragments up to 150 nt in length, the maximum length of biopolymer 101 to be sequenced is approximately 50 nm in the elongated state, although ssDNA conformation can vary between elongated and coiled, depending on the ionic strength of the buffer. Because the MNP 102 participates in single-molecule reactions, the MNP 102 should have molecular dimensions. As explained above, the MNPs 102 can be, for example, superparamagnetic nanoparticles, organometallic compounds, or any other functional molecular group that can be detected by nanoscale magnetic sensors 105.

As explained above, an example monitoring system 100 can be implemented using magnetic sensors 105 in various configurations. For example, in some embodiments of the monitoring system 100, the magnetic sensors 105 (e.g., MTJs) are arranged in a square lattice that is compatible with existing crosspoint MRAM sensor geometries. As a specific example, a sensor array 110 having a configuration similar to the single Toshiba 4 G-bit density STT-MRAM chip first introduced at the International Electron Devices Meeting (IEDM) in 2016 can be used. In this case, the area of each nanoscale magnetic sensor 105 or its immediate proximity can be functionalized to serve as a respective binding site 116. The minimum nearest-neighbor distance 112 between magnetic sensors 105 of the Toshiba platform is 90 nm, which is sufficient spacing assuming the MNP 102 are superparamagnetic nanoparticles (e.g., iron oxide, iron platinum, etc.), the biopolymer 101 is 150 nt in length, and the sensor array 110 is a rectangular (e.g., square) array of magnetic tunnel junctions (MTJs) similar to those used in non-volatile data storage applications.

It is to be understood that the arrangement of magnetic sensors 105 in a grid pattern (e.g., a square lattice as shown in FIG. 19B) is one of many possible arrangements. It will be appreciated by those having ordinary skill in the art that other arrangements of the magnetic sensors 105 are possible and are within the scope of the disclosures herein. For example, the magnetic sensors 105 may be arranged in a hexagonal pattern, in which case each magnetic sensor 105 has up to six nearest neighbors, all at a nearest-neighbor distance 112. As will be appreciated by those having ordinary skill in the art, the sensor-packing limit (e.g., the minimum value of the nearest-neighbor distance 112) for a monitoring system 100 with a hexagonal arrangement of binding sites 116 and magnetic sensors 105 can be derived from knowledge of the size, shape, and properties of the magnetic sensors 105, the expected length of the biopolymer 101, and the size and type of the MNP 102 to be used.

### Example Monitoring Methods

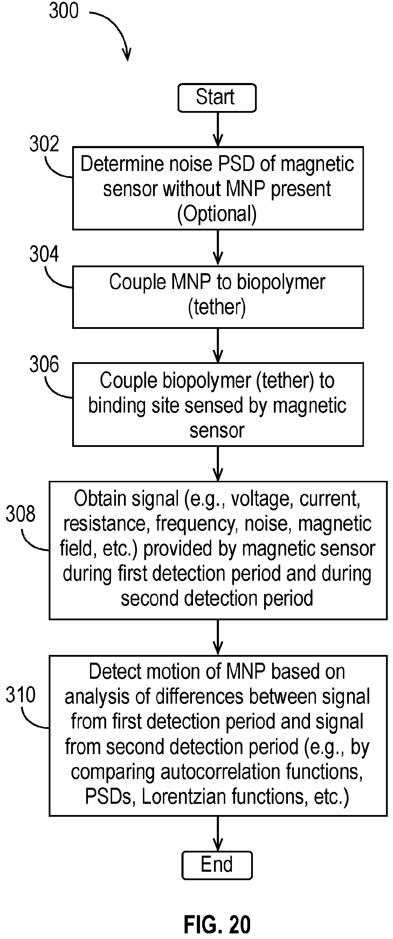

As described above (e.g., in the discussion of FIGS. 17A, 17B, 17C, 18A, 18B, and 18C), the magnetic sensors 105 described herein can be used in methods for monitoring single-molecule processes. FIG. 20 is a flow diagram of an exemplary method 300 of sensing motion of a tethered MNP 102 in accordance with some embodiments. At 302, optionally, the noise PSD of the magnetic sensor 105 is determined without any MNP 102 in its vicinity. As explained above, this step, if performed, establishes a baseline sensor PSD that can be compared to other PSDs to determine whether a MNP 102 is present.

At 304, a MNP 102 is coupled to a first end of a biopolymer 101 (e.g., a nucleic acid, protein, etc.). As explained above, the MNP 102 may be any suitable particle, including, for example, a superparamagnetic particle and/or a particle having a diameter of a few nanometers (e.g., less than approximately 5 nm). The MNP 102 may be of a different size (e.g., 20 nm). The MNP 102 may comprise or be any suitable material that can be detected by a magnetic sensor 105. For example, the MNP 102 may be or comprise iron oxide (FeO), Fe₃O₄, or FePt.

At 306, a second end (the other end) of the biopolymer 101 is coupled to a binding site 116 that is sensed by a magnetic sensor 105. As described above, the binding site 116 may be within a fluid chamber 115 of a monitoring system 100. As also described above, the magnetic sensor 105 may be any suitable sensor. For example, the magnetic sensor 105 may comprise a MTJ or STO.

At 308, a sensor signal 207 is obtained from the magnetic sensor 105 during a first detection period and during a second detection period. As explained above, the sensor signal 207 may be or indicate, for example, a current, voltage, resistance, noise (e.g., frequency noise or phase noise), frequency (e.g., oscillation frequency of a STO), magnetic field, etc. The first and second detection periods may be partially overlapping time periods, or they may be nonoverlapping, in which case a solution (e.g., containing Mg²⁺ ions) may be added (e.g., to a detection device fluid chamber 115) between the first and second time periods (e.g., as discussed above in the explanation of FIGS. 17B and 17C and FIGS. 18B and 18C).

At 310, motion of the MNP 102 is detected based on an analysis of changes in the sensor signal 207 between the first detection period and the second detection period. Changes in the sensor signal 207 between the first detection period and the second detection period may be detected, for example, by obtaining a first autocorrelation of a portion of the signal corresponding to the first detection period, obtaining a second autocorrelation of a portion of the signal corresponding to the second detection period, and identifying at least one difference between the first autocorrelation and the second autocorrelation (e.g., by comparing the autocorrelation functions as described above in the discussion of FIGS. 18A, 18B, and 18C). As another example, changes in the sensor signal 207 between the first detection period and the second detection period may be detected, in part, by determining at least one Lorentzian function that, when added to the noise PSD of the magnetic sensor 105, results in the PSD of the sensor signal 207 during the first detection period and/or the second detection period. Motion of the MNP 102 can be determined based on a comparison of the Lorentzian function that fits the sensor signal 207 captured during the first detection period to the Lorentzian function that fits the sensor signal 207 captured during the second detection period. Processing and/or analysis of the sensor signal 207 can be performed in the time domain, the frequency domain, or a combination of the two. For example, as described above, autocorrelation functions of portions of the sensor signal 207 taken at different times can reveal movement of a MNP 102 being sensed by a magnetic sensor 105. In some circumstances, time-domain processing may be preferred for this analysis. As another example, as described above, the PSD of the sensor signal 207 may be processed and/or fit with a Lorentzian function, and/or different Lorentzian functions can be compared. In some circumstances, this processing may be more convenient in the frequency domain. As yet another example, if the sensor signal 207 conveys a frequency (e.g., the oscillation frequency of a STO of the magnetic sensor 105), frequency-domain processing (e.g., following Fourier transforms of time-domain data) may be preferred. As yet another example, an autocorrelation function may be computed or determined and transformed into the frequency domain for further analysis.

It will be appreciated that the steps of the method 300 are illustrated in an exemplary order, but at least some of the steps can be performed in a different order. As just one example, step 306 can be performed before step 304 (as, e.g., described above in the discussion of FIGS. 17A, 17B, and 17C). It will also be appreciated that certain of the steps illustrated in FIG. 20 can either be performed in real-time (or near real-time) or at a later time. For example, step 302, if performed at all, can be performed much earlier than any of the other steps, or even after all of the other steps have been completed (e.g., after the MNPs 102 have been rinsed away). As another example, one or more signals collected during step 308 can be recorded, and step 310 can be performed on recorded data. Specifically, the magnetic sensor 105 can be read/interrogated during a test or experiment, and the collected sensor signal 207, either in its native form or in another format (e.g., sampled, amplified, normalized, etc.), can be recorded (e.g., saved to memory). At some later time, one or more processors (e.g., the at least one processor 130) can retrieve and process the recorded sensor signal 207 and determine whether and/or when and/or how the MNP 102 being monitored by the magnetic sensor 105 moved during the test or experiment.

### Multiplexed Magnetic Digital Homogeneous, Non-Enzymatic (HoNon) ELISA

As explained above, traditional ELISA (analog) readout systems require large volumes that ultimately dilute reaction product, requiring millions of enzyme labels to generate signals that are detectable utilizing conventional plate readers. Traditional ELISA sensitivity is limited to the picomolar (e.g., pg/mL) range and above.

In contrast, single-molecule measurements are digital in nature. Each molecule generates a signal that can be detected and counted. It is easier to measure the presence or absence of signal (1s and 0s) than to detect the absolute amount of signal. Digital ELISA sensitivity is on the order of attomolar (aM) to sub-femtomolar (fM).

One example of a single-molecule digital ELISA technology is the Simoa bead-based assay from Quanterix. (See https://www.quanterix.com/simoa-technology/, last visited June 30, 2021.) In Simoa, paramagnetic particles are coupled to antibodies that are designed to bind to specific targets. These particles are added to a sample. Detection antibodies that are capable of generating fluorescence are then added, with the objective being to form an immunocomplex consisting of the bead, bound protein, and detection antibody. If the concentration is low enough, each bead will contain either one bound protein or zero bound proteins. The sample is then loaded into an array having many microwells, each large enough to hold one bead. After enzymatic signal amplification with fluorescent substrate and fluorescence imaging, the data can be analyzed.

Both traditional and digital ELISA are heterogeneous assays that involve enzymatic signal amplification and multiple time-consuming incubation, reaction, and washing steps, usually lasting several hours. A homogeneous assay is an assay format allowing assay measurement by a simple mix-and-read procedure without the need to process samples by separation or washing steps, which considerably shortens the analysis time. Short detection times, however, usually correlate with decreased sensitivities and dynamic ranges.

It is possible to obtain highly-sensitive detection comparable to digital ELISA with the simplicity of homogeneous assay. For example, homogeneous entropy-driven biomolecular assay (HEBA) achieves one-pot, catalytically amplified signal generation with no use of enzymes or precise temperature cycling. (See, e.g., Donghyuk Kim, et al., "Homogeneous Entropy-Driven Amplified Detection of Biomolecular Interactions," ACS Nano, July 2016, 10 (8), 7467-75.)

Digital homogeneous, non-enzymatic (HoNon) immunosorbent assay ELISA with no signal amplification has been demonstrated. (See, e.g., Kenji Akama, et al., "Wash- and Amplification-Free Digital Immunoassay Based on Single-Particle Motion Analysis," ACS Nano, November 2019 13 (11), 13116-26; Kenji Akama and Hiroyuki Noji, "Multiplexed homogeneous digital immunoassay based on single-particle motion analysis," Lab on a Chip, Issue 12, 2020; Kenji Akama and Hiroyuki Noji, "Multiparameter single-particle motion analysis for homogeneous digital immunoassay," Lab on a Chip, Issue 12, 2020,)

Compared to optical, plasmonic, and electrochemical biosensors, magnetic biosensors (e.g., the magnetic sensors 105 described herein) exhibit low background noise because most of the biological environment is non-magnetic. The sensor signal 207 is also less influenced by the types of the sample matrix, thereby allowing accurate and reliable detection processes. Thus, embodiments of the systems (e.g., system 100), devices, and methods described herein can be used to provide what can be referred to as "multiplexed magnetic digital HoNon ELISA."

FIG. 21 illustrates several components involved in multiplexed magnetic digital HoNon ELISA in accordance with some embodiments. For the sake of example, it is assumed that there are three biomarkers to be tested, A, B, and C, as shown in FIG. 21. To test for these three biomarkers, three anti-biomarker beads, A, B, and C, are also illustrated. Each bead includes a MNP 102 and a tether binding group (illustrated as a small circle) to allow it to bind to a flexible molecular tether. The same type of MNP 102 can be used for each bead, or different beads can include different types of MNP 102. For example, the MNP 102 included in anti-biomarker beads, A, B, and C can be of the same type (e.g., a single type of MNP 102 having the same chemical composition (e.g., FeO, Fe₃O₄, FePt, etc.) can be used for all of anti-biomarker beads, A, B, and C). Alternatively, two or more MNP 102 types can be used for different anti-biomarker beads (e.g., FeO can be used for anti-biomarker beads A, FePt can be used for anti-biomarker beads B, etc.). In FIG. 21, anti-biomarker A bead includes MNP 102A of a first type, anti-biomarker B bead includes MNP 102B of a second type, which may be the same as or different from the first type, and anti-biomarker C bead includes MNP 102C of a third type, which may be the same as or different from the first type and/or the second type. The different anti-biomarker types are shaded differently in the drawings to allow them to be distinguished from each other, but it is to be appreciated that the shading in the drawings does not necessarily mean that the chemical compositions of the MNPs 102 in use are different.

As described above, the monitoring system 100 may include a sensor array 110. FIG. 21 illustrates a portion 118 of such a sensor array 110 in accordance with some embodiments. The portion 118 includes three magnetic sensors 105, namely magnetic sensor 105A, magnetic sensor 105B, and magnetic sensor 105C. Each magnetic sensor 105 has a respective binding site 116 on the surface 117 of the sensor array 110 (i.e., magnetic sensor 105A has binding site 116A, magnetic sensor 105B has binding site 116B, and magnetic sensor 105C has binding site 116C), which may be within a fluid chamber 115. Attached to the surface 117 at each binding site 116 is a respective flexible molecular tether (e.g., a biopolymer 101). For example, at binding site 116A is the tether 101A, at binding site 116B is the tether 101B, and at binding site 116C is the tether 101C.

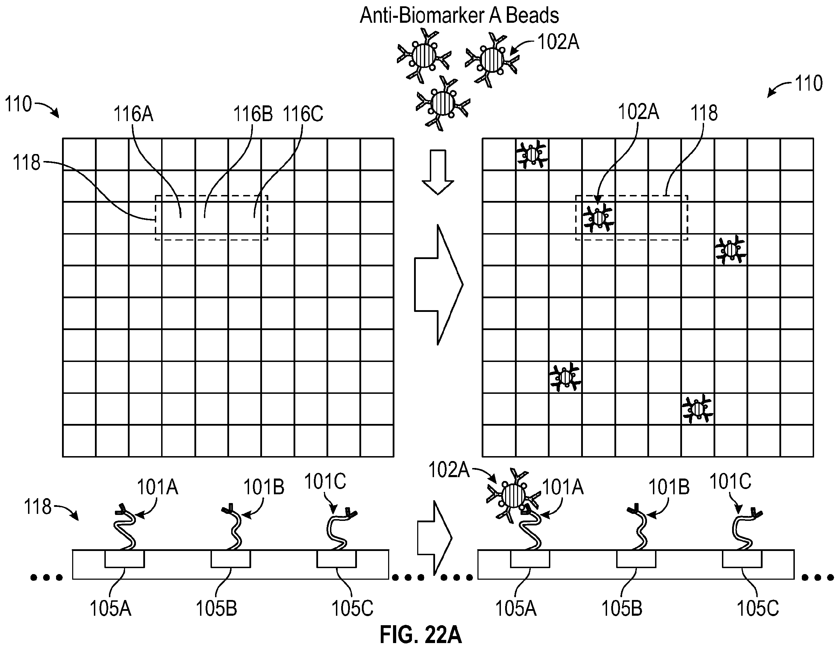

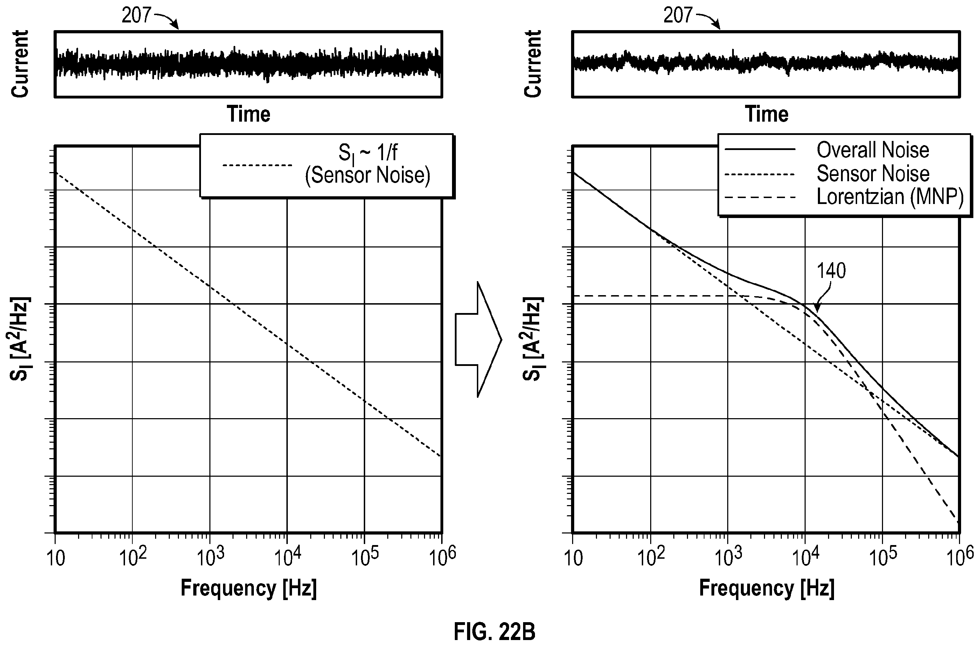

FIGS. 22A and 22B illustrate a portion of an exemplary procedure for multiplexed magnetic digital HoNon ELISA in accordance with some embodiments. FIG. 22A illustrates the introduction of a plurality of anti-biomarker A beads, including the MNP 102A, to the sensor array 110 (e.g., by adding a solution to a fluid chamber 115 of a monitoring system 100). As shown on the right-hand side of FIG. 22A, the anti-biomarker A bead that includes the MNP 102A binds to the tether 101A at the binding site 116A sensed by the magnetic sensor 105A. FIG. 22B illustrates how the binding of the MNP 102A to the tether 101A affects the sensor signal 207 detected by the magnetic sensor 105 (assumed for the sake of example to be a MTJ). As shown by the sensor signal 207 and plot on the left-hand side of FIG. 22B, before the anti-biomarker A bead that includes MNP 102A has bound to the tether 101A, the noise PSD of the sensor signal 207 exhibits the 1/*f* characteristic that is expected of a MTJ sensor when no MNP 102 is present. The right-hand side of FIG. 22B illustrates that after the MNP 102A has bound to the tether 101A, the noise PSD of the sensor signal 207 exhibits the characteristic bump 140 that is expected due to the presence of a Lorentzian function in the overall noise. The presence of the bump 140 in the overall noise PSD indicates that a MNP 102 has bound to the tether 101A at the magnetic sensor 105A. Because only the anti-biomarker A beads have been added at this point, all of the magnetic sensors 105 in the sensor array 110 can be interrogated to identify which of their overall PSDs have bumps 140 and thereby determine the locations of anti-biomarker A beads (e.g., to determine which of all of the tethers 101 have incorporated anti-biomarker beads of type A).

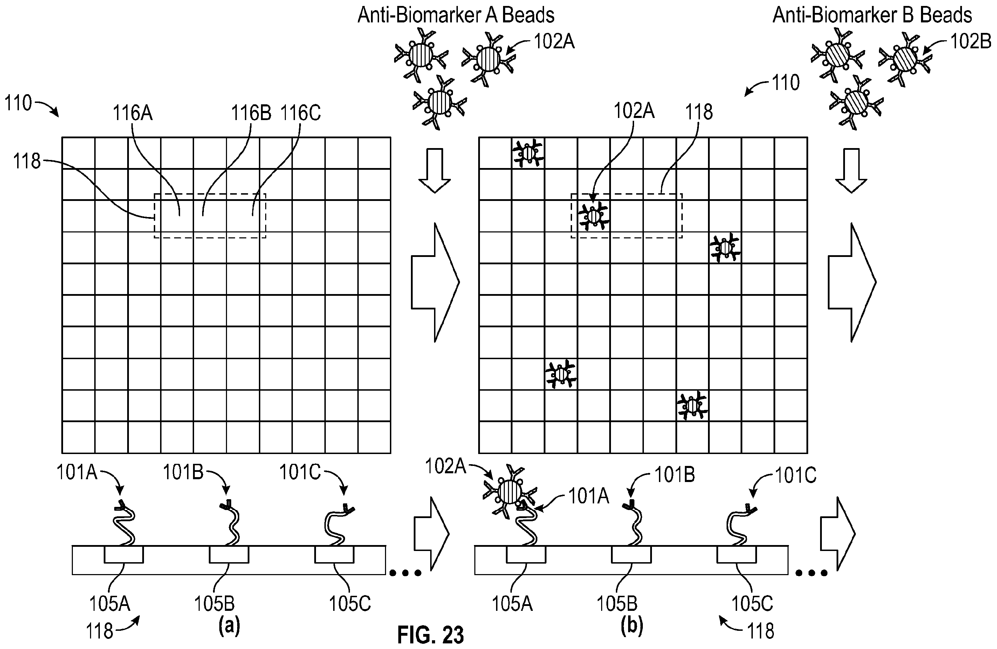

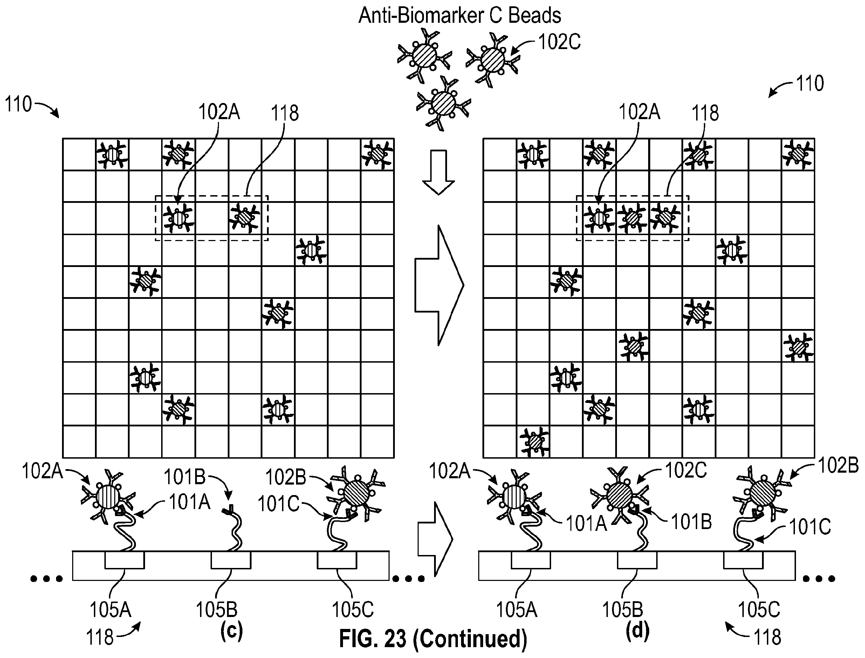

FIG. 23 illustrates additional possible steps in the exemplary procedure depicted in FIGS. 22A and 22B. The portions of FIG. 23 labeled "(a)" and "(b)" were described above in the discussion of FIGS. 22A and 22B. That discussion applies to FIG. 23 and is not repeated. After recording the locations of anti-biomarker A beads in the sensor array 110, another plurality of anti-biomarker beads can optionally be added. For example, FIG. 23 illustrates adding a plurality of anti-biomarker B beads, one of which includes MNP 102B, next. As shown in the portion of FIG. 23 labeled "(c)," the anti-biomarker B bead that includes MNP 102B binds to the tether 101C at magnetic sensor 105C. As explained above, the presence of the MNP 102B can be detected in the sensor signal 207 of the magnetic sensor 105C: the overall noise PSD will have a bump 140 due to the Lorentzian component contributed by the MNP 102B. Thus, the locations of anti-biomarker B beads within the sensor array 110 can be determined by interrogating the magnetic sensors 105 of the sensor array 110 that did not previously sense anti-biomarker A beads. After the identities of the magnetic sensors 105 sensing anti-biomarker B beads have been determined, the identities/locations of magnetic sensors 105 detecting anti-biomarker A beads and the identities/locations of magnetic sensors 105 detecting anti-biomarker B beads within the sensor array 110 are known.

Next, optionally, another plurality of anti-biomarker beads can be added. For example, FIG. 23 illustrates adding a plurality of anti-biomarker C beads, one of which includes MNP 102C, next. As shown in the portion of FIG. 23 labeled "(d)," the anti-biomarker C bead that includes MNP 102C binds to the tether 101B at magnetic sensor 105B. As explained above, the presence of the MNP 102C can be detected in the sensor signal 207 of the magnetic sensor 105B: the overall noise PSD will have a bump 140 due to the Lorentzian component contributed by the MNP 102C. Thus, the locations of anti-biomarker C beads can be determined by interrogating the magnetic sensors 105 of the sensor array 110 that did not sense anti-biomarker A beads or anti-biomarker B beads before. After the identities of the magnetic sensors 105 sensing anti-biomarker C beads have been determined, the identities/locations of magnetic sensors 105 detecting anti-biomarker A beads, the identities/locations of magnetic sensors 105 detecting anti-biomarker B beads, the identities/locations of magnetic sensors 105 detecting anti-biomarker C beads, and the locations/identities of magnetic sensors 105 that did not sense any MNP 102 within the sensor array 110 are all known.

Optionally, additional types of anti-biomarker beads can be added (e.g., more or fewer than three types of biomarkers can be tested), and the locations of these additional anti-biomarker beads determined as described above.

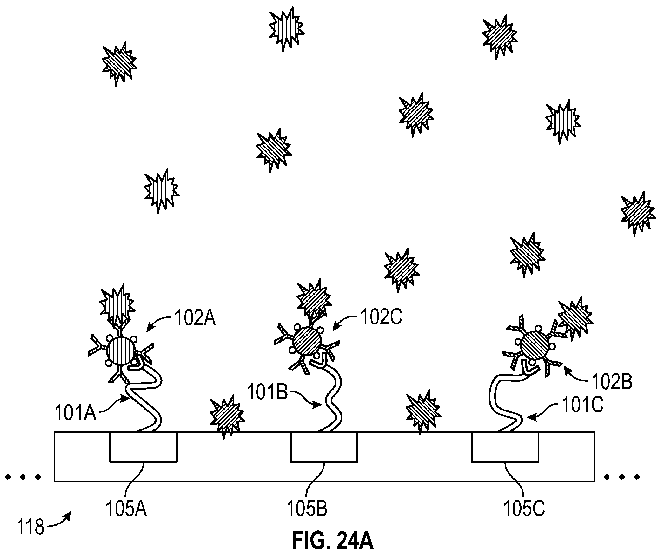

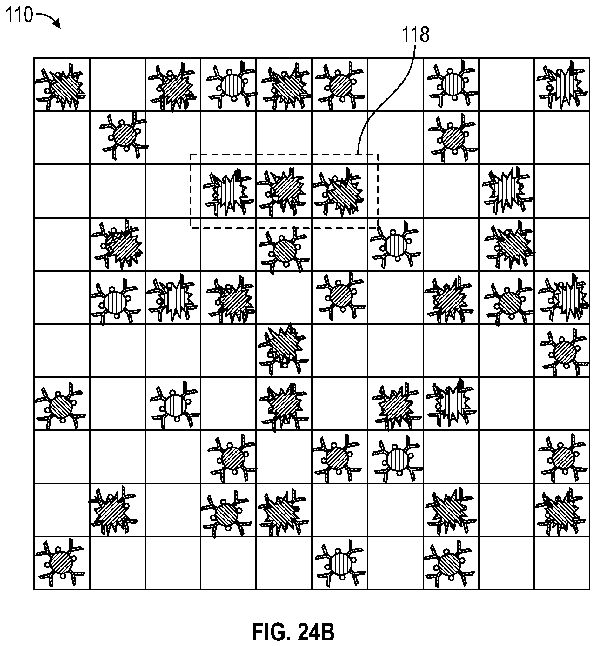

Next, as illustrated in FIG. 24A, biomarkers corresponding to the previously-added anti-biomarker beads can be added (e.g., to the fluid chamber 115 of a monitoring system 100). FIG. 24A illustrates the addition of a complex biological solution containing all of biomarkers A, B, and C. Because the locations of anti-biomarker A beads, anti-biomarker B beads, and anti-biomarker C beads are known, and because each biomarker type will bind only to the anti-biomarker bead of the same type, all of the biomarkers to be tested can be added at the same time without interference. As illustrated in the example of FIG. 24A, a biomarker of type A binds to the anti-biomarker A bead that includes MNP 102A attached to the tether 101A. Similarly, a biomarker of type B binds to the anti-biomarker B bead that includes MNP 102B attached to the tether 101C, and biomarker of type C binds to the anti-biomarker C bead that includes MNP 102C attached to the tether 101B. FIG. 24B shows an example of how the entire sensor array 110 might look following the addition of a complex biological solution containing all three biomarkers A, B, and C. (It is to be appreciated that, as explained above, a sensor array 110 implementation could have many more magnetic sensors 105 (e.g., thousands, millions, etc.) than shown in the figures herein.)

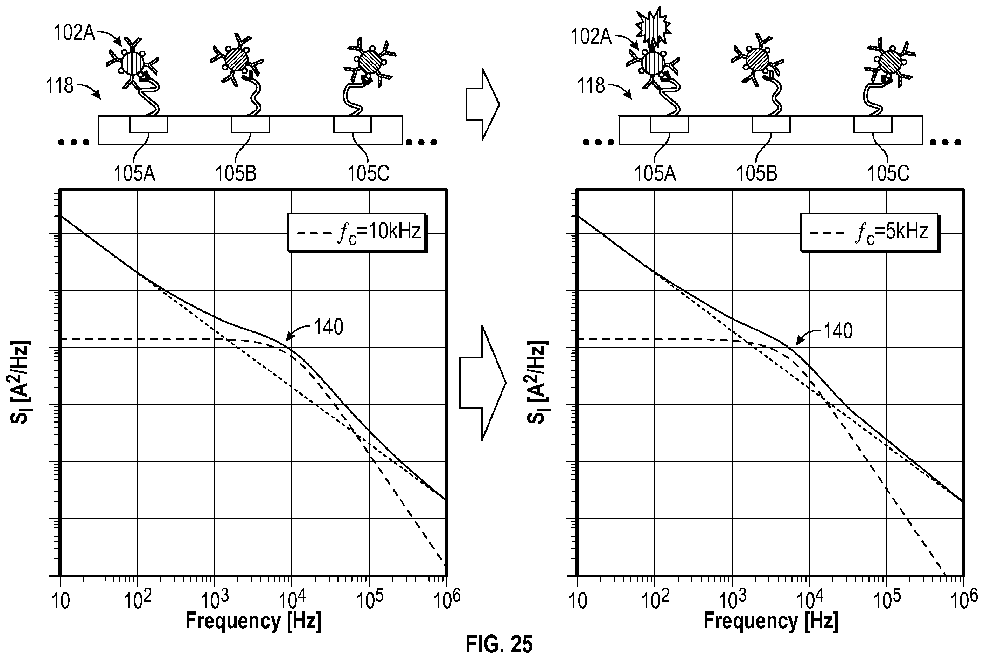

FIG. 25 illustrates how the binding of a biomarker can be detected from the detected noise PSD of the sensor signal 207 of a particular magnetic sensor 105. The left-hand side of FIG. 25 illustrates an example noise PSD for the magnetic sensor 105A after the MNP 102A has bound to the tether 101A (e.g., corresponding to the state of the sensor array 110 shown on the right-hand side of FIG. 22A). The left-hand size of FIG. 25 shows the component sensor noise PSD (caused by magnetic sensor 105A) and Lorentzian function (caused by MNP 102A) that, when added to the sensor noise PSD, produces the PSD of the overall noise in the sensor signal 207. In the illustrated example, the corner frequency of the Lorentzian function is approximately 10 kHz, which, as described above, is a function of the diameter of the MNP 102A: ${f}_{c} ≅ \frac{1}{6 {π}^{2} η} \left(\frac{K}{d}\right)$ where, as described above, *η* is the dynamic viscosity of the surrounding liquid (for water at room temperature, it is approximately $8.9 \times {10}^{- 4} \frac{N}{s \times {m}^{2}}$), *d* is the diameter of the MNP 102A, and *K* is the spring constant of the molecular tether 101A.

The right-hand side of FIG. 25 illustrates an example noise PSD for the magnetic sensor 105A after the addition of the complex biological solution and after a biomarker of type A has bound to the anti-biomarker A bead containing MNP 102A, which is bound to the tether 101A at the magnetic sensor 105A. Also shown are the component sensor noise PSD and Lorentzian function that, when added to the sensor noise PSD, produces the PSD of the overall noise in the sensor signal 207. The sensor noise PSD is the same as on the left-hand side of FIG. 25, but the Lorentzian function has changed due to the incorporation of the biomarker of type A. Assuming that the diameter of the biomarker of type A is approximately the same as the diameter of the MNP 102A, the corner frequency of the Lorentzian function will shift to a lower frequency that is given by ${f}_{c} ≅ \frac{1}{6 {π}^{2} η} \left(\frac{K}{2 d}\right)$ Thus, the presence of the biomarker A at the magnetic sensor 105A makes the apparent diameter of the MNP 102A approximately double, which causes a non-negligible shift in the corner frequency of the Lorentzian function. By detecting this shift in the corner frequency, the presence of the biomarker A at the magnetic sensor 105A can be detected. The presence of biomarkers (of whatever type) at other magnetic sensors 105 can be detected similarly.

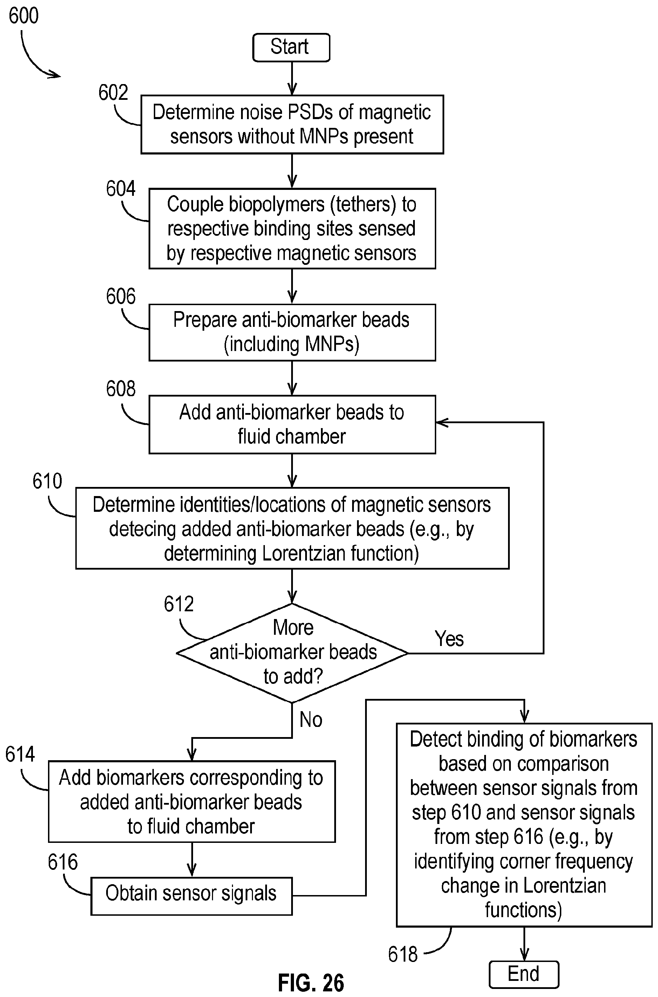

FIG. 26 is a flow diagram of a process 600 to detect binding of biomarkers in accordance with some embodiments. For example, the process 600 can be used to detect biological events such as those discussed in the context of FIG. 2A, among others. At 602, the noise PSDs of magnetic sensors 105 of a sensor array 110 are determined without any MNPs 102 present (e.g., without any MNP 102 in the sensing regions 206). At 604, biopolymers 101 (tethers) are coupled to respective binding sites 116 that are sensed by respective magnetic sensors 105. At 606, a plurality of anti-biomarker beads are prepared. As described above in the discussion of FIG. 21, the anti-biomarker beads include MNPs 102. At 608, a first set of anti-biomarker beads (e.g., of a first type to be tested) is added to the fluid chamber 115 of a monitoring system 100. At 610, the identities (or locations) of the magnetic sensors 105 detecting an anti-biomarker bead are determined. As explained above (e.g., in the discussion of FIGS. 22A and 22B), the presence of an anti-biomarker bead at a particular magnetic sensor 105 can be detected by determining whether an overall noise PSD of the sensor signal 207 after addition of the anti-biomarker beads (and, thus, the MNPs 102) has the bump 140 due to the addition of the Lorentzian function that characterizes the PSD of the noise caused by a MNP 102.

At 612, it is determined whether there are more anti-biomarker beads to be tested (e.g., referring to FIG. 23, whether there are anti-biomarker B beads or anti-biomarker C beads). If so, then the process 600 repeats steps 608 and 610. Once there are no more anti-biomarker beads to be added, the monitoring system 100 has a map of which magnetic sensors 105 of the sensor array 110 are sensing tethers 101 that have incorporated anti-biomarker beads, and, in the case of multiple types of anti-biomarker beads, which magnetic sensors 105 are sensing which types of anti-biomarker beads.

At 614, a solution containing biomarkers corresponding to the anti-biomarker beads in the fluid chamber 115 is added to the fluid chamber 115. As explained above, one benefit of some embodiments is that multiple biomarkers can be tested at once. Thus, if the fluid chamber 115 contains more than one type of anti-biomarker bead, the added solution can include multiple types of biomarkers, all of which can be added to the fluid chamber 115 at the same time. (Of course, it is to be appreciated that if there are multiple biomarkers to be tested, they can be added separately.)

At 616, the sensor signals 207 are obtained from at least those magnetic sensors 105 sensing respective MNPs 102. At 618, the binding of biomarkers is detected based on a comparison between the sensor signals 207 collected at step 610 and those collected at step 616. For example, as explained above in the discussion of FIG. 25, the corner frequency of the Lorentzian function that fits the overall noise PSD of the sensor signal 207 from step 610 can be compared to the corner frequency of the Lorentzian function that fits the overall noise PSD of the sensor signal 207 from step 616 to see if the corner frequency has changed. Specifically, and as explained above, incorporation of a biomarker can be detected from a reduction in the corner frequency due to the effective diameter of the MNP 102 increasing (e.g., the effective mass of the biopolymer 101 increases, and the frequency of motion of the MNP 102 decreases).

It is to be appreciated that the steps of the process 600 are shown in an exemplary order, but some steps can be performed in a different order. As just one example, the order of steps 602, 604, and 606 can be different (e.g., step 604 can be performed before step 602 or after step 606; step 606 can be performed before step 602 and/or before step 604; etc.).

In the foregoing description and in the accompanying drawings, specific terminology has been set forth to provide a thorough understanding of the disclosed embodiments. In some instances, the terminology or drawings may imply specific details that are not required to practice the invention.

To avoid obscuring the present disclosure unnecessarily, well-known components are shown in block diagram form and/or are not discussed in detail or, in some cases, at all.

Unless otherwise specifically defined herein, all terms are to be given their broadest possible interpretation, including meanings implied from the specification and drawings and meanings understood by those skilled in the art and/or as defined in dictionaries, treatises, etc. As set forth explicitly herein, some terms may not comport with their ordinary or customary meanings.

As used herein, the singular forms "a," "an" and "the" do not exclude plural referents unless otherwise specified. The word "or" is to be interpreted as inclusive unless otherwise specified. Thus, the phrase "A or B" is to be interpreted as meaning all of the following: "both A and B," "A but not B," and "B but not A." Any use of "and/or" herein does not mean that the word "or" alone connotes exclusivity.

As used herein, phrases of the form "at least one of A, B, and C," "at least one of A, B, or C," "one or more of A, B, or C," and "one or more of A, B, and C" are interchangeable, and each encompasses all of the following meanings: "A only," "B only," "C only," "A and B but not C," "A and C but not B," "B and C but not A," and "all of A, B, and C."

To the extent that the terms "include(s)," "having," "has," "with," and variants thereof are used herein, such terms are intended to be inclusive in a manner similar to the term "comprising," i.e., meaning "including but not limited to." The terms "exemplary" and "embodiment" are used to express examples, not preferences or requirements. The term "coupled" is used herein to express a direct connection/attachment as well as a connection/attachment through one or more intervening elements or structures. The terms "over," "under," "between," and "on" are used herein refer to a relative position of one feature with respect to other features. For example, one feature disposed "over" or "under" another feature may be directly in contact with the other feature or may have intervening material. Moreover, one feature disposed "between" two features may be directly in contact with the two features or may have one or more intervening features or materials. In contrast, a first feature "on" a second feature is in contact with that second feature.

The term "substantially" is used to describe a structure, configuration, dimension, etc. that is largely or nearly as stated, but, due to manufacturing tolerances and the like, may in practice result in a situation in which the structure, configuration, dimension, etc. is not always or necessarily precisely as stated. For example, describing two lengths as "substantially equal" means that the two lengths are the same for all practical purposes, but they may not (and need not) be precisely equal at sufficiently small scales. As another example, a structure that is "substantially vertical" would be considered to be vertical for all practical purposes, even if it is not precisely at 90 degrees relative to horizontal.

The drawings are not necessarily to scale, and the dimensions, shapes, and sizes of the features may differ substantially from how they are depicted in the drawings.

## Claims

1. A method (300) for monitoring single-molecule biological processes using a magnetic sensor (105) having a sensing region, the method comprising:
coupling (304) a biopolymer to a binding site sensed by the magnetic sensor;
coupling (306) a magnetic particle to the biopolymer; and
obtaining (308) a signal from the magnetic sensor during a first detection period and during a second detection period, wherein the signal characterizes a magnetic environment within a sensing region of the magnetic sensor,
**characterised by** further comprising:
determining a first power spectral density, PSD, of the signal obtained during the first detection period;
fitting a first Lorentzian function **characterized by** a first corner frequency to the first PSD;
determining a second PSD of the signal obtained during the second detection period;
fitting a second Lorentzian function **characterized by** a second corner frequency to the second PSD; and
detecting (310) motion of the magnetic particle based on a change in the signal between the first detection period and the second detection period, wherein detecting the motion of the magnetic particle based on the change in the signal between the first detection period and the second detection period comprises identifying a difference between the first corner frequency and the second corner frequency.

2. The method recited in claim 1, wherein detecting the motion of the magnetic particle based on the change in the signal between the first detection period and the second detection period comprises:
obtaining a first autocorrelation of a portion of the signal corresponding to the first detection period;
obtaining a second autocorrelation of a portion of the signal corresponding to the second detection period; and
identifying at least one difference between the first autocorrelation and the second autocorrelation.

3. The method recited in claim 1 or claim 2, wherein the binding site is situated in fluid chamber of a detection system, and further comprising adding a solution to the fluid chamber between the first detection period and the second detection period.

4. The method recited in any one of claims 1 to 3, further comprising applying a magnetic field to the magnetic particle.

5. The method recited in any one of claims 1 to 4, further comprising obtaining the signal from the magnetic sensor during a third detection period, wherein the third detection period takes place while the magnetic particle is outside of the sensing region.

6. The method recited in claim 5, further comprising determining a baseline noise power spectral density, PSD, of the magnetic sensor using the signal detected during the third detection period.

7. The method recited in claim 6, wherein a sum of the first Lorentzian function and the baseline noise PSD of the magnetic sensor is approximately equal to a PSD of the signal from the magnetic sensor during the first detection period, and wherein a sum of the second Lorentzian function and the baseline noise PSD of the magnetic sensor is approximately equal to a PSD of the signal from the magnetic sensor during the second detection period.

8. The method recited in claim 7, further comprising:
concluding that a biological process has occurred based on the first corner frequency being different from the second corner frequency.

9. The method recited in claim 8, wherein the biological process comprises coupling of a biomarker to the biopolymer, and the second detection period follows addition of a complex biological solution comprising a plurality of biomarkers, and wherein the first corner frequency is greater than the second corner frequency.

10. The method recited in any one of claims 1 to 9, wherein the second detection period follows addition of a complex biological solution comprising a plurality of biomarkers, and wherein the first corner frequency is greater than the second corner frequency.

11. A system (100) for monitoring motion of a magnetic particle (102) coupled to a biopolymer (101), the system comprising:
a fluid chamber (115) comprising a binding site (116) for holding no more than a single biopolymer at a time, and wherein the binding site is configured to affix an end of the biopolymer to a surface (117) of the fluid chamber and to allow the magnetic particle to move;
at least one processor (130); and
a magnetic sensor (105) having a sensing region (206) within the fluid chamber, wherein the sensing region includes the binding site but no other binding site, and wherein the magnetic sensor is configured to generate a signal (207) characterizing a magnetic environment within the sensing region and to provide the signal to the at least one processor,
**characterised in that** the at least one processor is configured to:
obtain a first portion of the signal, the first portion of the signal representing the magnetic environment within the sensing region during a first detection period,
determine a first power spectral density, PSD, of the first portion of the signal,
obtain a second portion of the signal, the second portion of the signal representing the magnetic environment within the sensing region during a second detection period, the second detection period being after the first detection period,
determine a second PSD of the second portion of the signal;
analyze the first portion of the signal and the second portion of the signal to detect motion of the magnetic particle by:
fitting a first Lorentzian function to the first PSD,
fitting a second Lorentzian function to the second PSD, and
comparing a first corner frequency of the first Lorentzian function to a second corner frequency of the second Lorentzian function.

12. The system recited in claim 11, wherein the signal conveys a frequency noise, a phase noise, or an oscillation frequency of the magnetic sensor.

13. The system recited in claim 11 or claim 12, wherein the at least one processor is further configured to:
determine a first autocorrelation function of the first portion of the signal; and
determine a second autocorrelation function of the second portion of the signal;
and wherein analyzing the first portion of the signal and the second portion of the signal to detect motion of the magnetic particle comprises comparing the first autocorrelation function to the second autocorrelation function.

14. The system recited in any one of claims 11 to 13 , further comprising detection circuitry coupled to the magnetic sensor and to the at least one processor.

15. The system recited in any one of claims 11 to 14, wherein the magnetic particle is a first magnetic particle, the biopolymer is a first biopolymer, the magnetic sensor is a first magnetic sensor, the sensing region is a first sensing region, and the signal is a first signal, and wherein the fluid chamber further comprises a second binding site for holding no more than a single biopolymer at a time, and wherein the second binding site is configured to affix an end of a second biopolymer to the surface of the fluid chamber and to allow a second magnetic particle coupled to the second biopolymer to move, and further comprising:
a second magnetic sensor having a second sensing region within the fluid chamber, wherein the second sensing region includes the second binding site but no other binding site, and wherein the second magnetic sensor is configured to generate a second signal characterizing a magnetic environment within the second sensing region and to provide the second signal to the at least one processor,
and wherein the at least one processor is further configured to:
obtain a first portion of the second signal, the first portion of the second signal representing the magnetic environment within the second sensing region during a third detection period,
obtain a second portion of the second signal, the second portion of the second signal representing the magnetic environment within the second sensing region during a fourth detection period, and
analyze the first portion of the second signal and the second portion of the second signal to detect motion of the second magnetic particle.

16. The system recited in claim 15, wherein the first and third detection periods are identical, and the second and fourth detection periods are identical.

17. The system recited in any one of claims 11 to 16, wherein the magnetic sensor is one of a plurality of magnetic sensors disposed in a sensor array (110).

18. The system recited in claim 17, further comprising at least one line coupling the sensor array to the at least one processor, wherein the binding site is situated in a trench in a first line of the at least one line.

19. The system recited in any one of claims 11 to 18, wherein the at least one processor comprises at least two processors, wherein a first processor of the at least two processors is configured to obtain the first and second portions of the signal, and a second processor of the at least two processors is configured to analyze the first and second portions of the signal to detect the motion of the magnetic particle.

20. The system recited in any one of claims 11 to 19, wherein the at least one processor is further configured to determine, based on comparing the first corner frequency to the second corner frequency, that a particular biomarker has coupled to the biopolymer.

## Patentansprüche

1. Verfahren (300) zum Überwachen von biologischen Einzelmolekülprozessen unter Verwendung eines Magnetsensors (105) mit einem Abfühlbereich, wobei das Verfahren Folgendes umfasst:
Koppeln (304) eines Biopolymers an eine Bindungsstelle, die durch den Magnetsensor abgefühlt wird;
Koppeln (306) eines magnetischen Partikels an das Biopolymer; und
Erhalten (308) eines Signals vom Magnetsensor während eines ersten Detektionszeitraums und während eines zweiten Detektionszeitraums, wobei das Signal eine magnetische Umgebung in einer Abfühlregion des Magnetsensors **kennzeichnet,**
**dadurch** gekennzeichnet, dass es ferner Folgendes umfasst:
Bestimmen einer ersten Leistungsspektrumsdichte, PSD, des während des ersten Detektionszeitraums erhaltenen Signals;
Anpassen einer ersten Lorentz-Funktion, die durch eine erste Grenzfrequenz gekennzeichnet ist, an die erste PSD;
Bestimmen einer zweiten PSD des während des zweiten Detektionszeitraums erhaltenen Signals;
Anpassen einer zweiten Lorentz-Funktion, die durch eine zweite Grenzfrequenz gekennzeichnet ist, an die zweite PSD; und
Detektieren (310) einer Bewegung des magnetischen Partikels basierend auf einer Änderung des Signals zwischen dem ersten Detektionszeitraum und dem zweiten Detektionszeitraum, wobei das Detektieren der Bewegung des magnetischen Teilchens basierend auf der Änderung des Signals zwischen dem ersten Detektionszeitraum und dem zweiten Detektionszeitraum das Identifizieren einer Differenz zwischen der ersten Grenzfrequenz und der zweiten Grenzfrequenz umfasst.

2. Verfahren nach Anspruch 1, wobei das Detektieren der Bewegung des magnetischen Partikels basierend auf der Änderung des Signals zwischen dem ersten Detektionszeitraum und dem zweiten Detektionszeitraum Folgendes umfasst;
Erhalten einer ersten Autokorrelation eines Abschnitts des Signals, das dem ersten Detektionszeitraum entspricht;
Erhalten einer zweiten Autokorrelation eines Abschnitts des Signals, das dem zweiten Detektionszeitraum entspricht; und
Identifizieren zumindest einer Differenz zwischen der ersten Autokorrelation und der zweiten Autokorrelation.

3. Verfahren nach Anspruch 1 oder 2, wobei die Bindungsstelle sich in einer Fluidkammer eines Detektionssystems befindet und ferner umfassend das Zusetzen einer Lösung zu der Fluidkammer zwischen dem ersten Detektionszeitraum und dem zweiten Detektionszeitraum.

4. Verfahren nach einem der Ansprüche 1 bis 3, ferner umfassend das Anlegen eines Magnetfelds an die magnetischen Partikel.

5. Verfahren nach einem der Ansprüche 1 bis 4, ferner umfassend das Erhalten des Signals vom Magnetsensor während eines dritten Detektionszeitraums, wobei der dritte Detektionszeitraum stattfindet, während das magnetische Partikel außerhalb der Abfühlregion ist.

6. Verfahren nach Anspruch 5, ferner umfassend das Bestimmen einer Grundlinienrauschen-Leistungsspektrumsdichte, PSD, des Magnetsensors unter Verwendung des während des dritten Detektionszeitraums detektierten Signals.

7. Verfahren nach Anspruch 6, wobei eine Summe der ersten Lorentz-Funktion und der Grundlinienrauschen-PSD des Magnetsensors ungefähr einer PSD des Signals von dem Magnetsensor während des ersten Detektionszeitraums entspricht und wobei eine Summe der zweiten Lorentz-Funktion und der Grundlinienrauschen-PSD des Magnetsensors ungefähr einer PSD des Signals von dem Magnetsensor während des zweiten Detektionszeitraums entspricht.

8. Verfahren nach Anspruch 7, ferner umfassend:
Schlussfolgern, dass ein biologischer Prozess eingetreten ist, basierend auf einem Unterschied zwischen der ersten Grenzfrequenz und der zweiten Grenzfrequenz.

9. Verfahren nach Anspruch 8, wobei der biologische Prozess das Koppeln eines Biomarkers an das Biopolymer umfasst und der zweite Detektionszeitraum auf das Zusetzen einer komplexen biologischen Lösung folgt, die eine Vielzahl von Biomarkern umfasst, und wobei die erste Grenzfrequenz größer ist als die zweite Grenzfrequenz.

10. Verfahren nach einem der Ansprüche 1 bis 9, wobei der zweite Detektionszeitraum auf das Zusetzen einer komplexen biologischen Lösung folgt, die eine Vielzahl von Biomarkern umfasst, und wobei die erste Grenzfrequenz größer ist als die zweite Grenzfrequenz.

11. System (100) zum Überwachen einer Bewegung eines magnetischen Partikels (102), das an ein Biopolymer (101) gekoppelt ist, wobei das System Folgendes umfasst:
eine Fluidkammer (115), umfassend eine Bindungsstelle (116) zum Enthalten von nicht mehr als einem einzigen Biopolymer auf einmal, und wobei die Bindungsstelle dazu ausgelegt ist, ein Ende des Biopolymers an einer Oberfläche (117) der Fluidkammer zu fixieren und es dem magnetischen Teilchen zu ermöglichen, sich zu bewegen;
zumindest einen Prozessor (130); und
einen Magnetsensor (105) mit einer Abfühlregion (206) innerhalb der Fluidkammer, wobei die Abfühlregion die Bindungsstelle umfasst, aber keine andere Bindungsstelle, und wobei der Magnetsensor dazu ausgelegt ist, ein Signal (207) zu erzeugen, das eine magnetische Umgebung innerhalb der Abfühlregion kennzeichnet, und das Signal für den zumindest einen Prozessor bereitzustellen,
**dadurch gekennzeichnet, dass** der zumindest eine Prozessor für Folgendes konfiguriert ist:
Erhalten eines ersten Abschnitts des Signals, wobei der erste Abschnitt des Signals die magnetische Umgebung innerhalb der Abfühlregion während eines ersten Detektionszeitraum darstellt,
Bestimmen einer ersten Leistungsspektrumsdichte, PSD, des ersten Abschnitts des Signals,
Erhalten eines zweiten Abschnitts des Signals, wobei der zweite Abschnitt des Signals die magnetische Umgebung innerhalb der Abfühlregion während eines zweiten Detektionszeitraums darstellt, wobei der zweite Detektionszeitraum nach dem ersten Detektionszeitraum liegt,
Bestimmen einer zweiten PSD des zweiten Abschnitts des Signals;
Analysieren des ersten Abschnitts des Signals und des zweiten Abschnitts des Signals, um eine Bewegung des magnetischen Teilchens zu detektieren, durch Folgendes:
Anpassen einer ersten Lorentz-Funktion an die erste PSD,
Anpassen einer zweiten Lorentz-Funktion an die zweite PSD und
Vergleichen einer ersten Grenzfrequenz der ersten Lorentz-Funktion mit einer zweiten Grenzfrequenz der zweiten Lorentz-Funktion.

12. System nach Anspruch 11, wobei das Signal ein Frequenzrauschen, ein Phasenrauschen oder eine Schwingungsfrequenz des Magnetsensors übermittelt.

13. System nach Anspruch 11 oder 12, wobei der zumindest eine Prozessor ferner für Folgendes konfiguriert ist:
Bestimmen einer ersten Autokorrelationsfunktion des ersten Abschnitts des Signals; und
Bestimmen einer zweiten Autokorrelationsfunktion des zweiten Abschnitts des Signals;
und wobei das Analysieren des ersten Abschnitts des Signals und des zweiten Abschnitts des Signals zum Detektieren einer Bewegung des magnetischen Partikels das Vergleichen der ersten Autokorrelationsfunktion mit der zweiten Autokorrelationsfunktion umfasst.

14. System nach einem der Ansprüche 11 bis 13, ferner umfassend eine Detektionsschaltungsanordnung, die mit dem Magnetsensor und dem zumindest einen Prozessor gekoppelt ist.

15. System nach einem der Ansprüche 11 bis 14, wobei das magnetische Partikel ein erstes magnetisches Partikel ist, das Biopolymer ein erstes Biopolymer ist, der Magnetsensor ein erster Magnetsensor ist, die Abfühlregion eine erste Abfühlregion ist und das Signal ein erstes Signal ist und wobei die Fluidkammer ferner eine zweite Bindungsstelle zum Enthalten von nicht mehr als einem einzigen Bipolymer auf einmal umfasst und wobei die zweite Bindungsstelle dazu ausgelegt ist, ein Ende eines zweiten Biopolymers an einer Oberfläche der Fluidkammer zu fixieren und es einem an das zweite Biopolymer gekoppelten zweiten magnetischen Partikel zu ermöglichen, sich zu bewegen, und ferner Folgendes umfassend:
einen zweiten Magnetsensor mit einer zweiten Abfühlregion innerhalb der Fluidkammer, wobei die zweite Abfühlregion die zweite Bindungsstelle umfasst, aber keine andere Bindungsstelle, und wobei der zweite Magnetsensor dazu ausgelegt ist, ein zweites Signal zu erzeugen, das eine magnetische Umgebung innerhalb der zweiten Abfühlregion kennzeichnet, und das zweite Signal für den zumindest einen Prozessor bereitzustellen,
und wobei der zumindest eine Prozessor ferner für Folgendes konfiguriert ist:
Erhalten eines ersten Abschnitts des zweiten Signals, wobei der erste Abschnitt des zweiten Signals die magnetische Umgebung innerhalb der zweiten Abfühlregion während eines dritten Detektionszeitraums darstellt,
Erhalten eines zweiten Abschnitts des zweiten Signals, wobei der zweite Abschnitt des zweiten Signals die magnetische Umgebung innerhalb der zweiten Abfühlregion während eines vierten Detektionszeitraums darstellt, und
Analysieren des ersten Abschnitts des zweiten Signals und des zweiten Abschnitts des zweiten Signals, um eine Bewegung des zweiten magnetischen Partikels zu detektieren.

16. System nach Anspruch 15, wobei der erste und der dritte Detektionszeitraum identisch sind und der zweite und der vierte Detektionszeitraum identisch sind.

17. System nach einem der Ansprüche 11 bis 16, wobei der Magnetsensor einer aus einer Vielzahl von Magnetsensoren, die in einer Sensoranordnung (110) angeordnet sind, ist.

18. System nach Anspruch 17, ferner umfassend zumindest eine Leitung, welche die Sensoranordnung mit dem zumindest einen Prozessor koppelt, wobei die Bindungsstelle sich in einer Vertiefung in einer ersten Leitung der zumindest einen Leitung befindet.

19. System nach einem der Ansprüche 11 bis 18, wobei der zumindest eine Prozessor zumindest zwei Prozessoren umfasst, wobei ein erster Prozessor aus den zumindest zwei Prozessoren konfiguriert ist, um den ersten und den zweiten Abschnitt des Signals zu erhalten, und ein zweiter Prozessor aus den zumindest zwei Prozessoren konfiguriert ist, um den ersten und den zweiten Abschnitt des Signals zu analysieren, um die Bewegung des magnetischen Teilchens zu detektieren.

20. System nach einem der Ansprüche 11 bis 19, wobei der zumindest eine Prozessor ferner konfiguriert ist, um basierend auf einem Vergleich der ersten Grenzfrequenz mit der zweiten Grenzfrequenz zu bestimmen, dass ein bestimmter Biomarker an das Biopolymer gekoppelt hat.

## Revendications

1. Procédé (300) de surveillance de processus biologiques à molécule unique en utilisant un capteur magnétique (105) présentant une région de détection, le procédé comprenant les étapes consistant à :
coupler (304) un biopolymère à un site de liaison détecté par le capteur magnétique ;
coupler (306) une particule magnétique au biopolymère ; et
obtenir (308) un signal à partir du capteur magnétique pendant une première période de détection et pendant une deuxième période de détection, dans lequel le signal caractérise un environnement magnétique dans une région de détection du capteur magnétique,
**caractérisé en ce qu'**il comprend en outre les étapes consistant à :
déterminer une première densité spectrale de puissance, PSD, du signal obtenu pendant la première période de détection ;
ajuster une première fonction lorentzienne **caractérisée par** une première fréquence de coude à la première PSD ;
déterminer une seconde PSD du signal obtenu pendant la deuxième période de détection ;
adapter une seconde fonction lorentzienne **caractérisée par** une seconde fréquence de coude à la seconde PSD ; et
détecter (310) un mouvement de la particule magnétique sur la base d'un changement du signal entre la première période de détection et la deuxième période de détection, la détection du mouvement de la particule magnétique sur la base du changement du signal entre la première période de détection et la deuxième période de détection comprenant une identification d'une différence entre la première fréquence de coude et la seconde fréquence de coude.

2. Procédé selon la revendication 1, dans lequel la détection du mouvement de la particule magnétique sur la base du changement du signal entre la première période de détection et la deuxième période de détection comprend les étapes consistant à :
obtenir une première auto-corrélation d'une partie du signal correspondant à la première période de détection ;
obtenir une seconde auto-corrélation d'une partie du signal correspondant à la deuxième période de détection ; et
identifier au moins une différence entre la première auto-corrélation et la seconde auto-corrélation.

3. Procédé selon la revendication 1 ou la revendication 2, dans lequel le site de liaison est situé dans la chambre à fluide d'un système de détection, et comprenant en outre un ajout d'une solution à la chambre à fluide entre la première période de détection et la deuxième période de détection.

4. Procédé selon l'une quelconque des revendications 1 à 3, comprenant en outre une application d'un champ magnétique à la particule magnétique.

5. Procédé selon l'une quelconque des revendications 1 à 4, comprenant en outre l'étape consistant à obtenir le signal à partir du capteur magnétique pendant une troisième période de détection, dans lequel la troisième période de détection a lieu tandis que la particule magnétique est à l'extérieur de la région de détection.

6. Procédé selon la revendication 5, comprenant en outre l'étape consistant à déterminer une densité spectrale de puissance de bruit de base, PSD, du capteur magnétique en utilisant le signal détecté pendant la troisième période de détection.

7. Procédé selon la revendication 6, dans lequel une somme de la première fonction lorentzienne et de la PSD de bruit de base du capteur magnétique est approximativement égale à une PSD du signal provenant du capteur magnétique pendant la première période de détection, et dans lequel une somme de la seconde fonction lorentzienne et de la PSD de bruit de base du capteur magnétique est approximativement égale à une PSD du signal provenant du capteur magnétique pendant la deuxième période de détection.

8. Procédé selon la revendication 7, comprenant en outre l'étape consistant à :
conclure qu'un processus biologique est survenu sur la base de la première fréquence de coude étant différente de la seconde fréquence de coude.

9. Procédé selon la revendication 8, dans lequel le processus biologique comprend un couplage d'un biomarqueur au biopolymère, et la deuxième période de détection suit l'ajout d'une solution biologique complexe comprenant une pluralité de biomarqueurs, et dans lequel la première fréquence de coude est supérieure à la seconde fréquence de coude.

10. Procédé selon l'une quelconque des revendications 1 à 9, dans lequel la deuxième période de détection suit l'ajout d'une solution biologique complexe comprenant une pluralité de biomarqueurs, et dans lequel la première fréquence de coude est supérieure à la seconde fréquence de coude.

11. Système (100) pour surveiller le mouvement d'une particule magnétique (102) couplée à un biopolymère (101), le système comprenant :
une chambre à fluide (115) comprenant un site de liaison (116) destiné à contenir pas plus d'un seul biopolymère à la fois, et dans lequel le site de liaison est configuré pour fixer une extrémité du biopolymère à une surface (117) de la chambre à fluide et pour permettre à la particule magnétique de se déplacer ;
au moins un processeur (130) ; et
un capteur magnétique (105) présentant une région de détection (206) à l'intérieur de la chambre à fluide, dans lequel la région de détection inclut le site de liaison mais aucun autre site de liaison, et dans lequel le capteur magnétique est configuré pour générer un signal (207) caractérisant un environnement magnétique à l'intérieur de la région de détection et pour fournir le signal au au moins un processeur,
**caractérisé en ce que** le au moins un processeur est configuré pour :
obtenir une première partie du signal, la première partie du signal représentant l'environnement magnétique à l'intérieur de la région de détection pendant une première période de détection,
déterminer une première densité spectrale de puissance, PSD, de la première partie du signal,
obtenir une seconde partie du signal, la seconde partie du signal représentant l'environnement magnétique au sein de la région de détection pendant une deuxième période de détection, la deuxième période de détection étant après la première période de détection,
déterminer une seconde PSD de la seconde partie du signal ;
analyser la première partie du signal et la seconde partie du signal pour détecter un mouvement de la particule magnétique par l'intermédiaire des étapes consistant à :
ajuster une première fonction lorentzienne à la première PSD,
ajuster une seconde fonction lorentzienne à la seconde PSD, et
comparer une première fréquence de coude de la première fonction lorentzienne à une seconde fréquence de coude de la seconde fonction lorentzienne.

12. Système selon la revendication 11, dans lequel le signal transporte un bruit de fréquence, un bruit de phase ou une fréquence d'oscillation du capteur magnétique.

13. Système selon la revendication 11 ou la revendication 12, dans lequel le au moins un processeur est en outre configuré pour :
déterminer une première fonction d'auto-corrélation de la première partie du signal ; et
déterminer une seconde fonction d'autocorrélation de la seconde partie du signal ;
et dans lequel l'analyse de la première partie du signal et de la seconde partie du signal pour détecter un mouvement de la particule magnétique comprend une comparaison de la première fonction d'autocorrélation à la seconde fonction d'autocorrélation.

14. Système selon l'une quelconque des revendications 11 à 13, comprenant en outre un circuit de détection couplé au capteur magnétique et au au moins un processeur.

15. Système selon l'une quelconque des revendications 11 à 14, dans lequel la particule magnétique est une première particule magnétique, le biopolymère est un premier biopolymère, le capteur magnétique est un premier capteur magnétique, la région de détection est une première région de détection, et le signal est un premier signal, et dans lequel la chambre à fluide comprend en outre un second site de liaison destiné à contenir pas plus d'un seul biopolymère à la fois, et dans lequel le second site de liaison est configuré pour fixer une extrémité d'un second biopolymère à la surface de la chambre à fluide et pour permettre à une seconde particule magnétique couplée au second biopolymère de se déplacer, et comprenant en outre :
un second capteur magnétique présentant une seconde région de détection à l'intérieur de la chambre à fluide, dans lequel la seconde région de détection inclut le second site de liaison mais aucun autre site de liaison, et dans lequel le second capteur magnétique est configuré pour générer un second signal caractérisant un environnement magnétique à l'intérieur de la seconde région de détection et pour fournir le second signal au au moins un processeur,
et dans lequel le au moins un processeur est en outre configuré pour :
obtenir une première partie du deuxième signal, la première partie du second signal représentant l'environnement magnétique au sein de la seconde région de détection pendant une troisième période de détection,
obtenir une seconde partie du second signal, la seconde partie du second signal représentant l'environnement magnétique à l'intérieur de la seconde région de détection pendant une quatrième période de détection, et
analyser la première partie du second signal et la seconde partie du second signal pour détecter un mouvement de la seconde particule magnétique.

16. Système selon la revendication 15, dans lequel les première et troisième périodes de détection sont identiques, et les deuxième et quatrième périodes de détection sont identiques.

17. Système selon l'une quelconque des revendications 11 à 16, dans lequel le capteur magnétique est l'un d'une pluralité de capteurs magnétiques disposés dans un réseau de capteurs (110).

18. Système selon la revendication 17, comprenant en outre au moins une ligne couplant le réseau de capteurs au au moins un processeur, dans lequel le site de liaison est situé dans une tranchée dans une première ligne de la au moins une ligne.

19. Système selon l'une quelconque des revendications 11 à 18, dans lequel le au moins un processeur comprend au moins deux processeurs, dans lequel un premier processeur des au moins deux processeurs est configuré pour obtenir les première et seconde parties du signal, et un second processeur des au moins deux processeurs est configuré pour analyser les première et seconde parties du signal afin de détecter le mouvement de la particule magnétique.

20. Système selon l'une quelconque des revendications 11 à 19, dans lequel le au moins un processeur est en outre configuré pour déterminer, sur la base d'une comparaison de la première fréquence de coude à la seconde fréquence de coude, qu'un biomarqueur particulier a été couplé au biopolymère.
